# EUROPEAN PATENT APPLICATION

(11) **EP 2 824 190 A1**
(43) Date of publication of application: **14.01.2015**
(21) Application number: 14166974.7
(22) Date of filing: 09.09.2004
(51) Int. Cl.: C12Q 1/68, C12P 19/34, C07H 21/04, C12N 15/13, C07K 16/00, C12N 9/00, C12N 9/50

(54) **Methods and compositions for generation of germline human antibody genes**

(30) Priority: 09.09.2003 US 501073 P
(62) Divisional of application: 04788690.8
(71) Applicant: Integrigen, Inc., Novato, CA 94949 (US)
(72) Inventor: Sharma, Vikram, San Francisco, CA 94131 (US); Leonard, Lindsay, Santa Barbara, CA 93190 (US); Smider, Vaughn, San Diego, CA 92129 (US)
(74) Representative: Bühler, Dirk

(57) **Abstract**

The present invention relates to a method for in vitro producing polynucleotides encoding human germline antibody V-regions. Also disclosed is a library of human germline antibody V-region genes.

## Description

### CROSS-REFERENCES TO RELATED APPLICATIONS

This application claims priority to U.S. Provisional Application No. 60/501,073, filed September 9, 2003, the contents of which are incorporated herein by reference in the entirety.

### BACKGROUND OF THE INVENTION

The immune system of a mammal is one of the most versatile biological systems as probably greater than 10⁷ antibody specificities can be produced. Indeed, much of contemporary biological and medical research is directed toward tapping this repertoire. Recently there has been a dramatic increase in the ability to harness the output of the vast immunological repertoire. The development of the hybridoma methodology by Kohler and Milstein has made it possible to produce monoclonal antibodies, i.e., a composition of antibody molecules of a single specificity, from the repertoire of antibodies induced during an immune response.

Unfortunately, current methods for generating monoclonal antibodies are not capable of efficiently surveying the entire antibody response induced by a particular immunogen. In an individual animal there are at least 5-10,000 different B-cell clones capable of generating unique antibodies to a small relatively rigid immunogens, such as, for example dinitrophenol. Further, because of the process of somatic mutation during the generation of antibody diversity, essentially an unlimited number of unique antibody molecules may be generated. In contrast to this vast potential for different antibodies, current hybridoma methodologies typically yield only a few hundred different monoclonal antibodies per fusion.

Approaches to mimicking the first stage randomisation process which have been described in the literature include those based on the construction of 'naïve' or 'germline' combinatorial antibody libraries prepared by isolating panels of immunoglobulin heavy chain variable (VH) domains and recombining these with panels of light variable chains (VL) domains (see, for example, Gram et al, Proc. Natl. Acad. Sa, USA, 89, 3576-3580, 1992). Naive libraries of antibody fragments have been constructed, for example, by cloning the rearranged V-genes from the IgM RNA ofB cells of un-immunised donors isolated from peripheral blood lymphocytes, bone marrow or spleen cells (see, for example, Griffiths et al, EMBO Journal, 12(2), 725-734, 1993, Marks et al, J. Mol. Biol., 222, 581-597, 1991). Such libraries can be screened for antibodies against a range of different antigens.

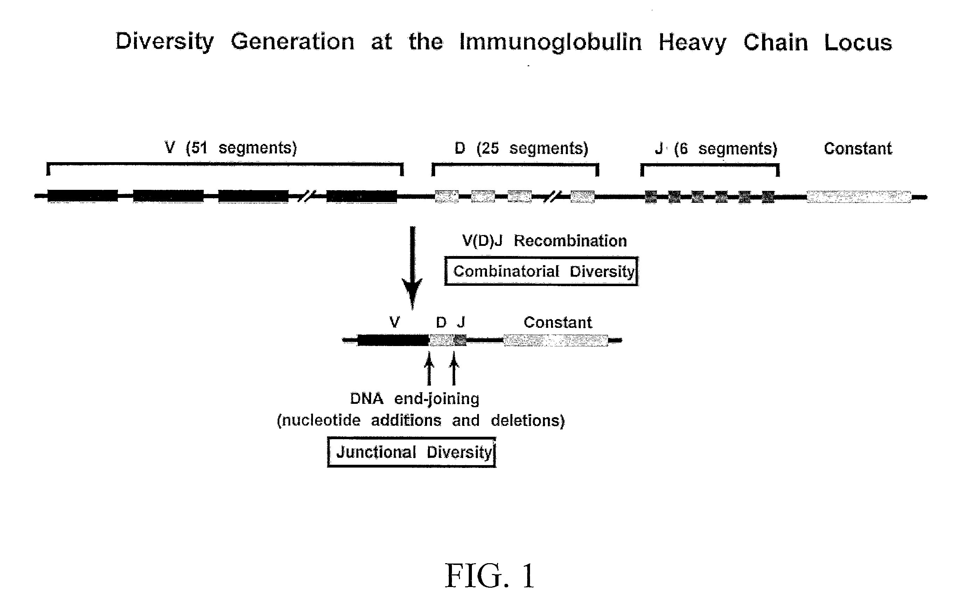

Germline antibody genes form precursors to the high affinity antibodies characterized by the secondary immune response. Germline antibody diversity can reach nearly 2x10⁷ different antibodies that derive from the combinatorial use of different V, D, or J minigenes (see FIG. 1). Further diversity is generated by insertional or deletional events occurring at the V-D, D-J, or V-J junctions. Antibody proteins encoded by germline genes are important for several reasons: (i) they form the basis from which higher affinity and more specific antibodies can be derived by further mutation, (ii) all germline heavy or light chain proteins can efficiently pair with other one another, (iii) germline antibody proteins have a flexible structure, allowing polyspecificity and antigen induced complimentarity, and (iv) germline encoded proteins can confer unique activities such as protease function to antibodies.

Thus, germline antibody genes have a commercial use as precursors to more high affinity antibodies, can be useful in the generation of efficiently pairing libraries of heavy and light chains, and could uniquely confer properties like protease activity to antibodies that contain them. Prior to the present invention, no known methods existed to recombine human antibody germline minigenes *in vitro* in order to produce functional antibody genes. The aforementioned techniques to produce antibodies suffer from limitations which are overcome by the present invention. For example, all of the above techniques rely on the *in vivo* recombination of antibody genes. In an animal, negative and positive selection events act upon antibody producing B-cells to limit the antibody repertoire. Thus, antibodies or antibody libraries from an animal can be "skewed" towards those antibody sequences compatible with a particular organism or biological environment. In the pharmaceutical industry, drug targets for antibody therapeutics are often "self" antigens. Antibodies to "self" antigens (or antigens endogenously produced by the animal) would be negatively selected, and removed from the animal, in order to avoid autoimmune disease. Although fully synthetic methods to produce antibody genes in vitro have been described, such methods produce significant changes in the antibody genes which could render them immunogenic as a therapeutic. The present invention allows a completely *in vitro* approach to produce germline antibody genes, which mimicks the natural process of V(D)J recombination that occurs in vivo. Such antibody genes are completely human and native in their sequence, and libraries of such antibody genes can be constructed which represent an unselected population representing the entire antibody repertoire. This invention addresses these and other related needs.

### BRIEF SUMMARY OF THE INVENTION

The present invention provides a method to generate full length antibody germline V-region genes, and the proteins which they encode. The method utilizes gene amplification to produce a V minigene, and a hybrid primer capable of hybridizing to a V minigene and either a D or J minigene. Such a hybrid primer facilitates recombination of a V minigene to a D or J minigene to produce a full length V-region gene. The method described herein allows production of V-regions comprising: a) degenerate codons in germline antibody CDRs, b) germline V-regions of different lengths, c) germline V-region genes encoding unique functional activities such as protease function, d) protein molecules encoded by said germline V-region genes, e) cells transfected with said germline V-region genes, including hybridoma cells or hybridoma fusion partners, f) transgenic mice comprising the rearranged germline V-region genes, g) germline V-region genes used in display technologies like phage display, ribosome display, RNA display, or plasmid display, h) germline V-region genes as part of an addressable array.

The present invention additionally provides for libraries of exogenously rearranged germline antibody genes. Such libraries can comprise antibody genes of human origin, and may include light chain V-regions, heavy chain V-regions, or light chain V-regions operably linked to heavy chain V-regions. Protein molecules produced from such libraries can be monomers, heterodimers, or homodimers. The library format can be phage display, ribosome display, RNA display, plasmid display, or any other display technology compatible with antibody expression. Additionally, the libraries could form part of an addressable array.

### BRIEF DESCRIPTION OF THE DRAWINGS

**FIG. 1** shows a schematic diagram of the antibody heavy chain germline locus. V, D, and J minigenes are arranged on the chromosome and are recombined to produce a functional antibody V-region. Combinatorial and junctional diversity generate a diverse array of antibody germline genes.

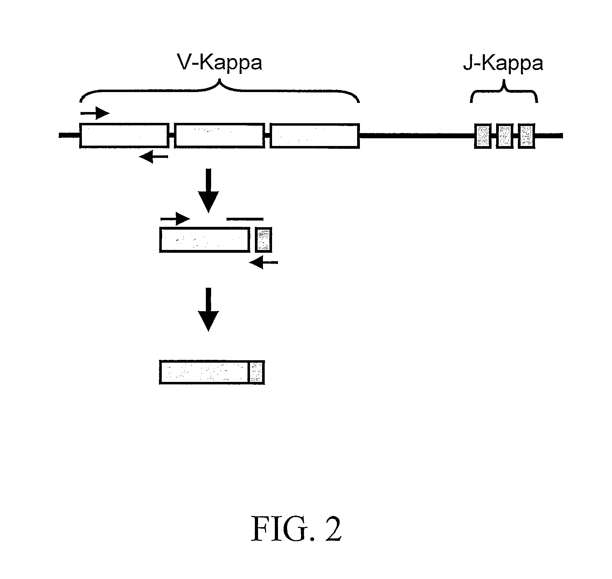

**FIG. 2** shows a schematic diagram of the V(D)J recombination method for generating germline V-region antibody genes described herein.

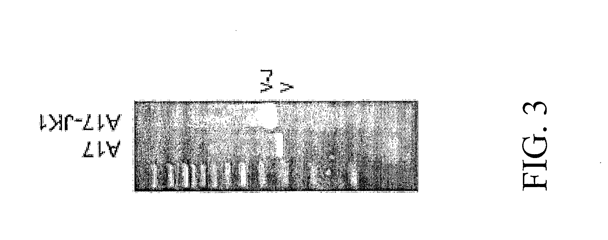

**FIG. 3** shows an agarose gel of the A17 V minigene as amplified by polymerase chain reaction, and the result of recombination of the A17 V minigene and the JK1 minigene.

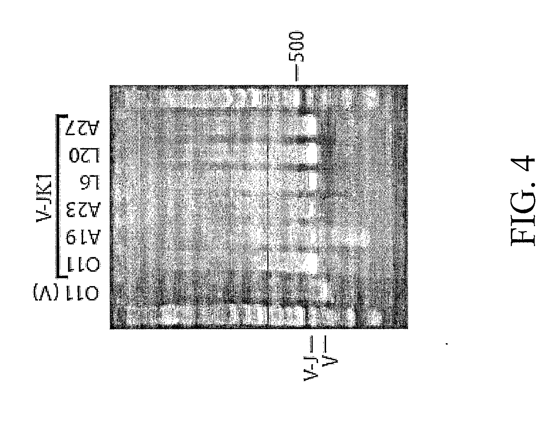

**FIG. 4** shows an agarose gel of several V minigenes recombined with the JK1 minigene.

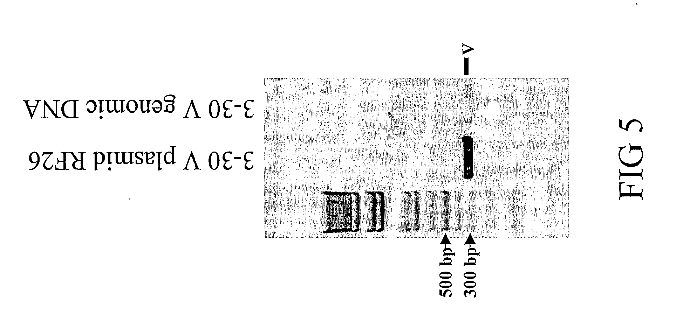

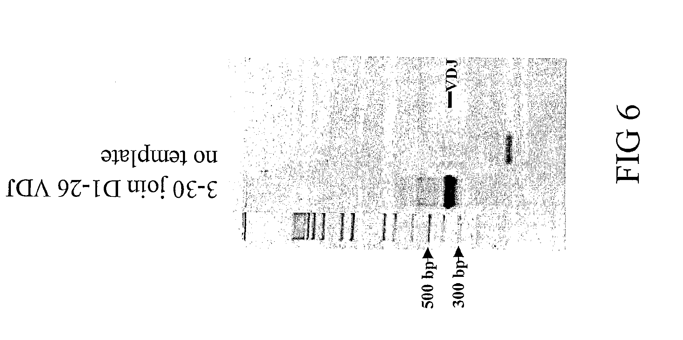

**FIG** 5 shows the 3-30 VH minigene PCR amplified with primers annealing at 63° C to human genomic DNA and plasmid RF26 that contains the 3-30 VH gene. The derived PCR products served as template in VDJ reactions for Figures 6, 7, and 9.
**FIG 6** shows the VDJ recombined product for a VDJ reaction involving a single 3-30 VH sequence as derived from pRF26 and a single non-degenerate joining oligo D1-26. Sequencing results in Table 2 show that 2 of 2 clones analyzed are identical to the computer assembled (electronic) copy of 3-30 VDJ: All clones have the same invariant 3-30 VH sequence as defined by plasmid RF26, and single invariant IGHD1-26 and IGHJ4 sequences.

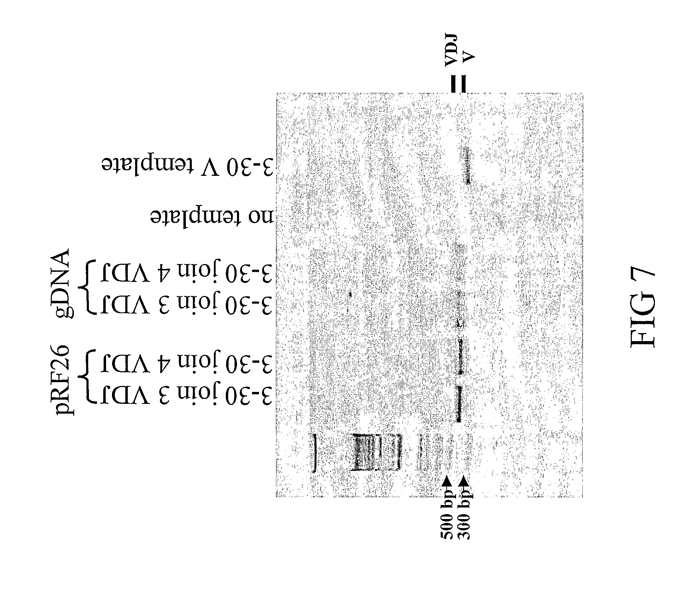

**FIG 7** shows 3-oligo VDJ recombined products using degenerate joining oligos 3 or 4 and the 3-30 VH minigene derived from either plasmid or gDNA in independent recombination reactions. Successful recombination is visually shown by the conversion of input template at ∼300 bp, to VDJ recombined template of ∼400 bp. Sequence analysis in Table 2, show clones which used 3-30VH from plasmid RF26 have an invariant heavy chain, while clones using 3-30VH from genomic DNA have diverse heavy chains since the 3-30V F & R PCR primers will amplify other heavy chain variable regions in gDNA depending on the stringency of the PCR. Clones from both sets of constructs have diverse D-regions demonstrating successful VDJ recombination with degenerate joining oligos.

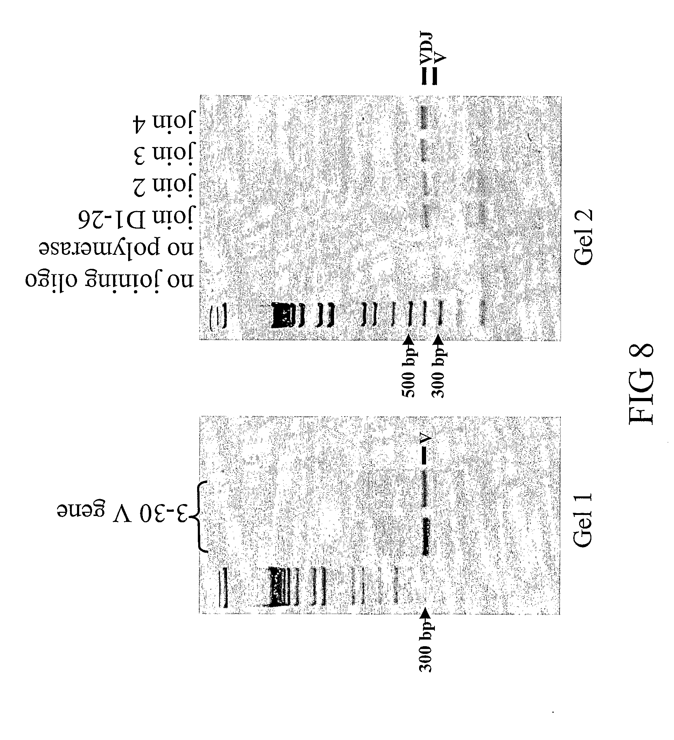

**FIG 8** shows another set VDJ recombined products for each joining oligo (Gel 2), using the 3-30VH gene amplified at low stringency (56 °C annealing) from genomic DNA (Gel 1). Conversion of 3-30VH template (∼300 bp) to recombined VDJ product can be seen. Recombined VDJ products were cloned into appropriate vectors. Sequence analysis in Table 2 shows that reduced stringency PCR increased the diversity of heavy chains in the sample compared to 3-30VH templates derived by PCR of gDNA at 63 C.

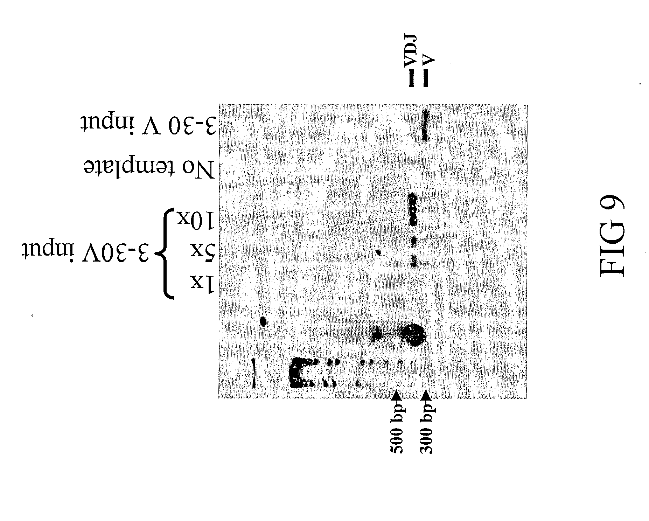

**FIG 9** shows the results of a VDJ recombination experiment using 2 oligos (join4 & JH4 Nhe/Not), instead of 3 oligos (join 4, JH4 Nhe/Not & 3-30R). The original PCR of 3-30VH template from plasmid RF26 was used in varying amounts with fixed concentrations of joining oligo 4 and JH4 Nhe/Not. Effective conversion of the 3-30VH template (∼300 bp) to recombined 3-30H VDJ product (∼400 bp) improves with increasing amounts of 3-30VH template. The 10x 3-30 VDJ product shown on the gel was cloned directly with out further amplification and sequenced. Results in Table 2 show that 3-30VH is invariant as expected while diverse D-regions were incorporated in the resulting 3-30 VDJ clones.

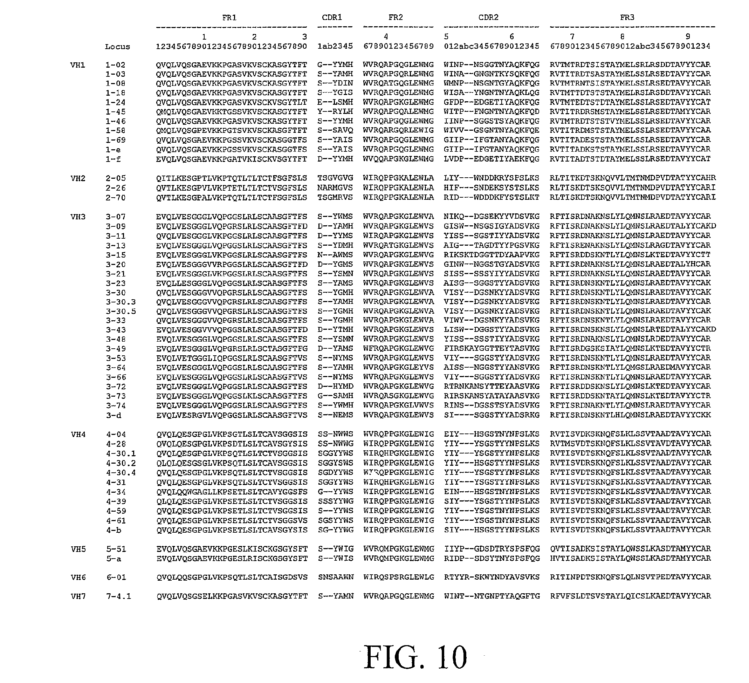

**FIG. 10** shows an amino acid alignment of the polypeptides encoded by the human germline heavy chain V minigenes. The complementarity determining regions are labeled CDR1, CDR2, and CDR3 and the framework regions are labeled FR1, FR2, and FR3. There are seven families of sequences, which are labeled VH1 through VH7 on the left, followed by the designation of each minigene. The amino acid position is indicated at the top.

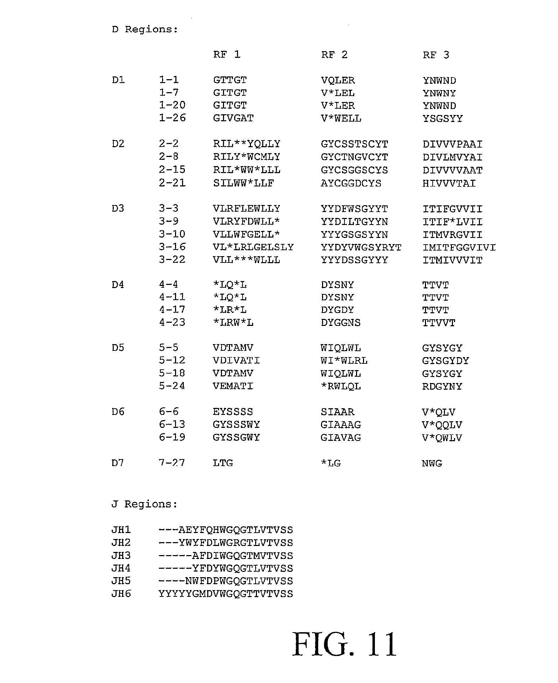

**FIG. 11** shows an amino acid alignment of the human germline heavy chain D (top) and J segments (bottom). The D regions can be read in multiple reading frames, which are designated RF1, RF2, and RF3. There are seven families of D regions, labeled D1 through D7 on the left, followed by the designation of each gene name. There are six J segments, labeled accordingly.

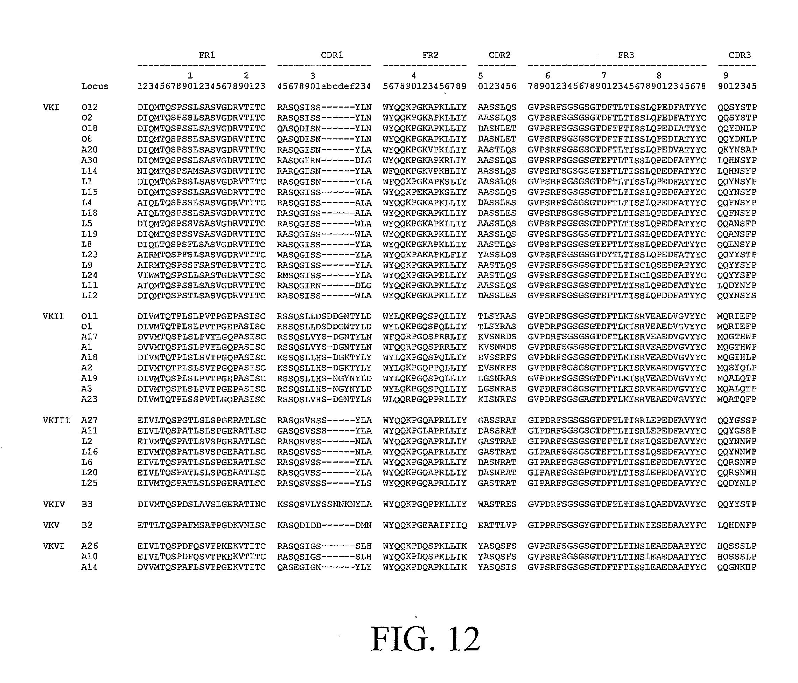

**FIG. 12** shows an amino acid alignment of the polypeptides encoded by the human germline light chain kappa V minigenes. The complementarity determining regions are labeled CDR1, CDR2, and CDR3 and the framework regions are labeled FR1, FR2, and FR3. There are six families of sequences, which are labeled VKI through VKVI on the left, followed by the designation of each minigene. The amino acid position is indicated at the top.

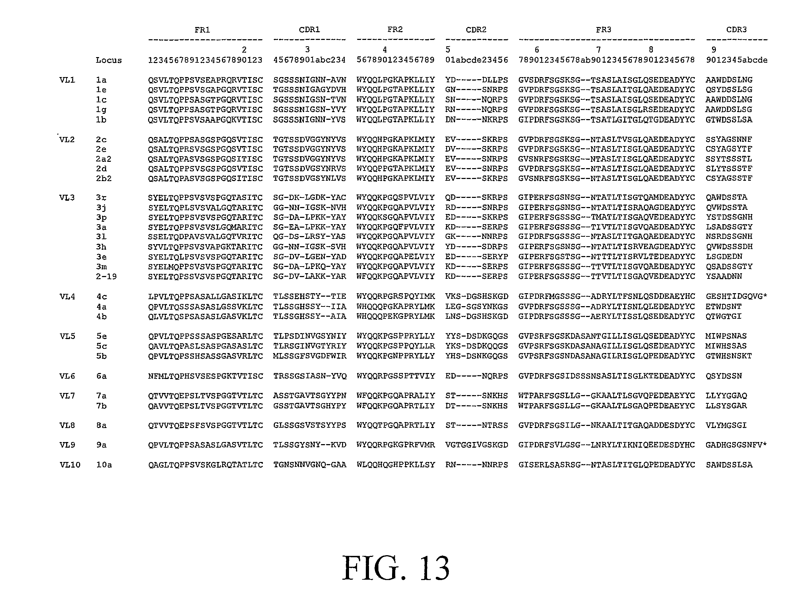

**FIG. 13** shows an amino acid alignment of the polypeptides encoded by the human germline light chain lambda V minigene. The complementarity determining regions are labeled CDR1, CDR2, and CDR3 and the framework regions are labeled FR1, FR2, and FR3. There are ten families of sequences, which are labeled VL1 through VL10 on the left, followed by the designation of each minigene. The amino acid position is indicated at the top.

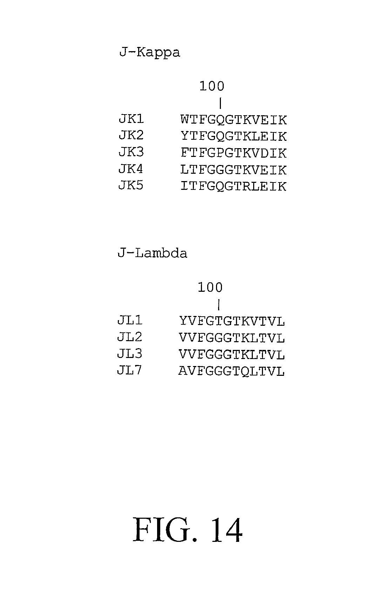

**FIG. 14** shows the polypeptides encoded by the J minigenes for the Kappa locus (top) and Lambda locus (bottom).

### DEFINITIONS

The term **"nucleic acid"** or **"polynucleotide"** refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (*e*.*g*., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)). The term nucleic acid is used interchangeably with gene, cDNA, and mRNA encoded by a gene.

The term **"gene"** means the segment of DNA involved in producing a polypeptide chain; it includes regions preceding and following the coding region (leader and trailer) as well as intervening sequences (introns) between individual coding segments (exons). The term **"minigene"** as applied to antibody genes refers to a polynucleotide sequence corresponding to the V, D, or J genetic element. Each of these genetic elements is incapable of encoding an antibody protein individually unless they are recombined with one another. For instance a functional antibody heavy chain gene comprises a V minigene fused to a D minigene which is fused to a J minigene. For functional light chain genes, a V minigene (either kappa or lambda) is fused to a J minigene. Human V, D, and J minigenes are well known to those in the art. Their sequences can be found online at the National Center for Biotechnology Information (NCBI) or in the literature [Ruiz, et al. Exp.Clin.Immunogenet. 16: 173-184(1999); Pallares, et al. Exp. Clin.Immunogenet. 16: 36-60(1999)]. The term "**germline**" refers to the sequences of the V, D, and J minigenes, prior to the exposure of an antibody to an antigen.

Rearranged **"V-regions"** describe the genetic element which results from the rearrangement event between V, D, and J (for heavy chains) or V and J minigenes (for light chains). An **"antibody V-region"** refers to the polypeptide region encoded by the V, D, and J element. An antibody V-region is encoded by rearranged V,D, and J minigenes. The term **"V(D)J Recombination"** refers to any process wherein a V, D, or J minigene is recombined to another V, D, or J minigene. A V-region may be part of a full length antibody, an FAb, a scFv, or any other derivative of an antibody (see definition of antibody below). A **"germline V-region"** refers to the sequence of rearranged V, D, and J minigenes prior to significant mutagenic events. A germline V-region may have random insertions or deletions at the junctions of the V-D, D-J, or V-J minigenes. A non-germline V-region (or a "**mature**" V-region) will differ from the germline sequences of the minigenes by usually more than 5 residues (not including the junctional deletions or insertions).

**"Polypeptide"** and **"peptide"** are used interchangeably herein to refer to a polymer of amino acid residues; whereas **"protein"** may contain one or multiple polypeptide chains. All three terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins, wherein the amino acid residues are linked by covalent peptide bonds.

An "**exogenously rearranged**" V-region or antibody gene refers to the location where the V, D, or J minigenes were rearranged to form a functional V-region gene capable of encoding an antibody V-region polypeptide. In an animal, rearrangement typically occurs in cells of the B-lymphoid lineage. Thus, an exogenously rearranged V-region is one wherein rearrangement occurs outside of a B lymphocyte. An exogenously rearranged V-region could have been rearranged in vitro, or in a cell line that does not typically undergo V(D)J rearrangement. Such cell lines can be induced to perform V(D)J rearrangement by introduction of the recombination activating genes RAG-1 and RAG-2 and the proper signal sequences adjacent to the V, D, or J minigenes. An **"endogenously rearranged"** V-region or antibody gene means that the gene was rearranged in a B-lymphocyte precursor, in the natural context of an animal.

The term **"amino acid"** refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an α carbon that is bound to a hydrogen, a carboxyl group, an amino group, and an R group, e.g., homoserine, norleucine, methionine sulfoxide, methionine methyl sulfonium. Such analogs have modified R groups (*e*.*g*., norleucine) or modified peptide backbones, but retain the same basic chemical structure as a naturally occurring amino acid. **"Amino acid mimetics"** refers to chemical compounds that have a structure that is different from the general chemical structure of an amino acid, but that functions in a manner similar to a naturally occurring amino acid.

Amino acids may be referred to herein by either the commonly known three letter symbols or by the one-letter symbols recommended by the IUPAC-IUB Biochemical Nomenclature Commission. Nucleotides, likewise, may be referred to by their commonly accepted single-letter codes.

**"Conservatively modified variants"** applies to both amino acid and nucleic acid sequences. With respect to particular nucleic acid sequences, **"conservatively modified variants"** refers to those nucleic acids that encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein that encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid that encodes a polypeptide is implicit in each described sequence.

The term **"degenerate",** when applied to nucleotide sequences, describes a nucleotide sequence wherein more than one residue could be located at a given location. Degenerate nucleotides are given the following notation: R=A/G, Y=C/T, M=A/C, K=G/T, S=C/G, W=A/T, B=C/G/T, D=A/G/T, H=A/C/T, V=A/C/G, and N=A/C/G/T.

The term **"homologous"** or **homology"** means that one single-stranded nucleic acid sequence may hybridize to a second single-stranded nucleic acid sequence under certain conditions. The degree of hybridization may depend on a number of factors including the amount of identity between the sequences and the hybridization conditions such as temperature and salt concentration as discussed later. Preferably the region of identity is greater than about 5 bp, more preferably the region of identity is greater than 10 bp. If two nucleic acids have **"homology,"** they can hybridize to one another under appropriate conditions.

As used herein, the term **"operably linked"** refers to a linkage of polynucleotide elements in a functional relationship. A nucleic acid is "operably linked" when it is placed into a functional relationship with another nucleic acid sequence. For instance, a promoter or enhancer is operably linked to a coding sequence if it affects the transcription of the coding sequence. Nucleic acids encoding proteins that produce multimers, such as heterodimers, heterotrimers, etc., are operably linked when they are expressed together at the same time under conditions where they may interact.

An **"antibody"** refers to a protein of the immunoglobulin family or a polypeptide comprising fragments of an immunoglobulin that is capable of noncovalently, reversibly, and in a specific manner binding a corresponding antigen. An exemplary antibody structural unit comprises a tetramer. Each tetramer is composed of two identical pairs of polypeptide chains, each pair having one "light" (about 25 kD) and one "heavy" chain (about 50-70 kD), connected through a disulfide bond. The recognized immunoglobulin genes include the κ, λ, *α*, *γ*, *δ*, *ε*, and *µ* constant region genes, as well as the myriad immunoglobulin variable region genes. Light chains are classified as either *κ* or λ. Heavy chains are classified as *γ*, *µ*, *α*, *δ*, or *ε*, which in turn define the immunoglobulin classes, IgG, IgM, IgA, IgD, and IgE, respectively. The N-terminus of each chain defines a variable region of about 100 to 110 or more amino acids primarily responsible for antigen recognition. The terms variable light chain (V_{L}) and variable heavy chain (V_{H}) refer to these regions of light and heavy chains respectively.

**"Complementarity-determining domains"** or **"CDRs"** refers to the hypervariable regions of V_{L} and V_{H}. The CDRs are the target protein-binding site of the antibody chains that harbors specificity for such target protein. There are three CDRs (CDR1-3, numbered sequentially from the N-terminus) in each human V_{L} or V_{H}, constituting about 15-20% of the variable domains. The CDRs are structurally complementary to the epitope of the target protein and are thus directly responsible for the binding specificity. The remaining stretches of the V_{L} or V_{H}, the so-called framework regions, exhibit less variation in amino acid sequence (Kuby, Immunology, 4th ed., Chapter 4. W.H. Freeman & Co., New York, 2000).

The positions of the CDRs and framework regions are determined using various well known definitions in the art, *e.g*., Kabat, Chothia, international ImMunoGeneTics database (IMGT), and AbM (*see, e.g.,* Johnson et al., Nucleic Acids Res., 29:205-206 (2001); Chothia and Lesk, J. Mol. Biol., 196:901-917 (1987); Chothia et al., Nature, 342:877-883 (1989); Chothia et al., J. Mol. Biol., 227:799-817 (1992); A1-Lazikani et al., J.Mol.Biol., 273:927-748 (1997)). Definitions of antigen combining sites are also described in the following: Ruiz et al., Nucleic Acids Res., 28:219-221 (2000); and Lefranc, M.P., Nucleic Acids Res., 29:207-209 (2001); MacCallum et al., J. Mol. Biol., 262:732-745 (1996); and Martin et al, Proc. Natl. Acad. Sci. USA, 86:9268-9272 (1989); Martin et al., Methods Enzymol., 203:121-153 (1991); and Rees et al., In Sternberg M.J.E. (ed.), Protein Structure Prediction, Oxford University Press, Oxford, 141-172 (1996).

An **"antibody light chain"** or an **"antibody heavy chain"** as used herein refers to a polypeptide comprising the V_{L} or V_{H}, respectively. The V_{L} is encoded by the minigenes V (variable) and J (junctional), and the V_{H} by minigenes V, D (diversity), and J. Each of V_{L} or V_{H} includes the CDRs as well as the framework regions. In this application, antibody light chains and/or antibody heavy chains may, from time to time, be collectively referred to as **"antibody chains."** These terms encompass antibody chains containing mutations that do not disrupt the basic structure of V_{L} or V_{H}, as one skilled in the art will readily recognize.

Antibodies exist as intact immunoglobulins or as a number of well-characterized fragments produced by digestion with various peptidases. Thus, for example, pepsin digests an antibody below the disulfide linkages in the hinge region to produce F(_{ab})'₂, a dimer of F_{ab}' which itself is a light chain joined to V_{H}-C_{H}1 by a disulfide bond. The F(_{ab})'₂ may be reduced under mild conditions to break the disulfide linkage in the hinge region, thereby converting the F(_{ab})'₂ dimer into an F_{ab}' monomer. The F_{ab}' monomer is essentially F_{ab} with part of the hinge region. Paul, Fundamental Immunology 3d ed. (1993). While various antibody fragments are defined in terms of the digestion of an intact antibody, one of skill will appreciate that such fragments may be synthesized *de novo* either chemically or by using recombinant DNA methodology. Thus, the term antibody, as used herein, also includes antibody fragments either produced by the modification of whole antibodies, or those synthesized de novo using recombinant DNA methodologies (*e.g*., single chain Fᵥ) or those identified using phage display libraries (*see, e.g.,* McCafferty et al., Nature, 348:552-554 (1990)).

For preparation of monoclonal or polyclonal antibodies, any technique known in the art can be used (*see, e.g*., Kohler & Milstein, Nature, 256:495-497 (1975); Kozbor et al., Immunology Today, 4:72 (1983); Cole et al., Monoclonal Antibodies and Cancer Therapy, pp. 77-96. Alan R. Liss, Inc. 1985). Techniques for the production of single chain antibodies (U.S. Patent No. 4,946,778) can be adapted to produce antibodies to polypeptides of this invention. Also, transgenic mice, or other organisms such as other mammals, may be used to express humanized antibodies. Alternatively, phage display technology can be used to identify antibodies, and heteromeric F_{ab} fragments, or scFv fragments that specifically bind to selected antigens (*see, e.g*., McCafferty et al., *supra*; Marks et al., Biotechnology, 10:779-783, (1992)).

The term **"endopeptidase activity"** as used herein refers to the ability of an enzyme to catalyze the hydrolysis of at least one non-terminal peptide bond between two amino acid residues within a polypeptide of any length.

Despite the diversity in primary amino acid sequence among individual members of the family, serine protease activity is supported by a highly conserved tertiary structure, which comprises a serine-histidine-aspartate triad. Studies have shown that the aspartate residue is not always essential for catalytic activity. The **"serine protease dyad"** as used herein is the minimal structure of the catalytic site for a protease to maintain at least a portion of its proteolytic activity. This structure comprises a histidine residue and a serine residue located within CDR3 and CDR2, respectively, of an antibody light chain, where the residues are in a spatial relation to each other similar to their spatial alignment in a serine protease triad, such that the histidine can abstract the proton from the serine hydroxyl group, allowing the serine to act as a nucleophile and attack the carbonyl group of the amide bond within the protein substrate.

**"Mutating"** or **"mutation**" refers to the deletion, insertion, or substitution of any nucleotide, by chemical, enzymatic, or any other means, in a nucleic acid encoding an antibody germline gene such that the amino acid sequence of the resulting polypeptide is altered at one or more amino acid residues.

A **"library"** of germline antibody members refers to a repertoire of recombinant polypeptides comprising at least two different germline V-region genes or proteins.

As used herein, the term **"array"** refers to an ordered spatial arrangement, particularly an arrangement of immobilized biomolecules or polymeric anchoring structures.

As used herein, the term **"addressable array"** refers to an array wherein the individual elements have precisely defined x and y coordinates, so that a given element can be pinpointed.

**"Primer extension"** refers to the process whereby: a homologous polynucleotide hybridizes to a second homologous polynucleotide, wherein at least one of the ends of the hybridized molecule contains a single-stranded region and under conditions wherein a polymerase converts at least a portion of the single stranded region to a double-stranded polynucleotide.

### DETAILED DESCRIPTION OF THE INVENTION

The present invention provides a method to generate full length antibody germline V-region genes, and the proteins which they encode. For example, the method first produces a V minigene, by a method such as gene amplification or chemical synthesis, and then uses a hybrid primer capable of hybridizing to a V minigene and either a D or J minigene. Such a hybrid primer facilitates recombination of a V minigene to a D or J minigene to produce a full length V-region gene. Likewise, a full length V-region gene may be produced from a similar process comprising first obtaining the sequence of a D or J minigene and subsequent recombination using a hybrid primer capable of hybridizing to a V minigene and a D or J minigene. The method described herein allows production of V-regions that include but are not limited to: a) degenerate codons in germline antibody CDRs, b) germline V-regions of different lengths, c) germline V-region genes encoding unique functional activities such as protease function, d) protein molecules encoded by said germline V-region genes, e) cells transfected with said germline V-region genes, including hybridoma cells or hybridoma fusion partners, f) transgenic mice comprising the said rearranged germline V-region genes, g) germline V-region genes used in display technologies like phage display, ribosome display, RNA display, or plasmid display, and h) germline V-region genes as part of an addressable array.

This invention relies on routine techniques in the field of recombinant genetics. Basic texts disclosing the general methods of use in this invention include Sambrook and Russell, Molecular Cloning: A Laboratory Manual 3d ed. (2001); Kriegler, Gene Transfer and Expression: A Laboratory Manual (1990); and Ausubel et al., Current Protocols in Molecular Biology (1994).

For nucleic acids, sizes are given in either kilobases (Kb) or base pairs (bp). These are estimates derived from agarose or polyacrylamide gel electrophoresis, from sequenced nucleic acids, or from published DNA sequences. For proteins, sizes are given in kilo-Daltons (kD) or amino acid residue numbers. Proteins sizes are estimated from gel electrophoresis, from sequenced proteins, from derived amino acid sequences, or from published protein sequences.

Oligonucleotides that are not commercially available can be chemically synthesized according to the solid phase phosphoramidite triester method first described by Beaucage and Caruthers, Tetrahedron Letters, 22:1859-1862 (1981), using an automated synthesizer, as described in Van Devanter et al., Nucleic Acids Res., 12:6159-6168 (1984). Purification of oligonucleotides is by either native polyacrylamide gel electrophoresis or by anion-exchange chromatography as described in Pearson & Reanier, J. Chrom., 255:137-149 (1983). The sequence of the cloned genes and synthetic oligonucleotides can be verified after cloning using, e.g., the chain termination method for sequencing double-stranded templates of Wallace et al., Gene, 16:21-26 (1981).

### Production of germline V regions

The production of a full length antibody V-region requires the V, D, and J minigenes. V minigenes are typically between 250 and 350 nucleotides in length, and could be produced by standard gene synthesis, or by amplification from a template nucleic acid that comprises unrearranged V minigenes. The D or J minigenes can be produced in a similar manner. In one embodiment, a V minigene is amplified directly from germline DNA. An example of germline DNA is genomic DNA prepared from a cell line or tissue. Such a cell line or tissue is preferably derived from a mammal. Germline DNA is preferably not from a mature B-cell that produces an antibody molecule. One example of germline DNA is genomic DNA from a fibroblast.

Production of a polynucleotide sequence encoding a germline V region begins with obtaining the sequence of a V minigene or a J minigene, which may then be joined with at least a J minigene (optionally with a D minigene in between) or a V minigene (optionally with a D minigene in between), respectively. Both chemical methods and enzymatic methods are useful for obtaining the V or J minigene. For instance, the initial V or J minigene may be directly synthesized or may be obtained from a genomic DNA library using amplification methods such as polymerase chain reaction (PCR). Amplification of the V minigene requires primers for an amplification process. Such an amplification process includes the polymerase chain reaction or other isothermal amplification processes (see *e.g*. Kurn, U.S. Pat. 6,251,639). Primers for amplification may comprise nucleic acids or a derivative thereof, and may be at least 10 nucleotides in length. Preferably the primers are between 15 and 100 nucleotides long. The primers can be designed to amplify the full V minigene, and may or may not include the 5' sequence encoding the leader and intron or the 3' recombination signal sequence. Restriction sites may be included in the primer to facilitate cloning of the V minigene. If polymerase chain reaction is used to amplify the V minigene, the primers should be designed as forward and reverse primers such that the minigene is amplified after several rounds of thermocycling. Requirements for design of primers for PCR are well known to those of skill in the art, and are described in several references both generally and specifically for antibody genes.

Table 3 shows a set of oligonucleotide primers for amplifying the repertoire of germline heavy chain V minigenes from human genomic DNA

**Table 3: Multiple family V-region heavy chain primers**

| **Forward Primers:** | | | |
|---|---|---|---|
| **VH Family:** | **Primer Name:** | **V-Regions Amplified:** | **Sequence:** |
| VH1 | VH1FA | VH1-45 | CAGATGCAGCTGGTGCAGTCTGGG |
| | VH1FB | VH1-58 | CAAATGCAGCTGGTGCAGTCTGGG |
| | VH1FC | VH1-2, VH1-46, VH1-69, VH1-8 | CAGGTGCAGCTGGTGCAGTCTG |
| | VHIFD | VH1-3, VH1-18 | CAGGTTCAGCTGGTCCAGTCTGG |
| | VH1FE | VH1-24 | CAGGTCCAGCTGGTACAGCTGG |
| | | | |
| VH2 | VH2FA | VH2-26 | CAGGTCACCTTGAAGGAGTCTGG |
| | VH2FB | VH2-70 | CAGGTCACCTTGAGGGAGTCTGG |
| | VH2FC | VH2-5 | CAGATCACCTTGAAGGAGTCTGG |
| | | | |
| VH3 | VH3FA* | VH3-53, VH3-13, VH3-35, VH3-38, VH3-48, VH3-49 VH3-64, VH3-72, VH3-7, VH3-66, VH3-21, VH3-20, VH3-16, VH3-15 | GAGGTGCAGCTGGTGGAGTCTGG |
| | VH3FC* | VH3-30, VH3-33, VH3-11 | CAGGTGCAGCTGGTGGAGTCTGG |
| | VH3FD* | VH3-74, VH3-73 | GAGGTGCAGCTGGTGGAGTCCG |
| | VH3FE | VH3-43, VH3-9 | GAAGTGCAGCTGGTGGAGTCTGGG |
| | VH3FG | VH3-23 | GAGGTGCAGCTGTTGGAGTCTGG |
| | | | |
| VH4 | VH4FA | VH4-39 | CAGCTGCAGCTGCAGGAGTCGGG |
| | VH4FB | VH4-4, VH4-59, VH4-61, VH4-31, VH4-28 | CAGGTGCAGCTGCAGGAGTCGG |
| | VH4FC | VH4-34 | CAGGTGCAGCTACAGCAGTGG |
| VH5 | VH5F | VH5-51 | GAGGTGCAGCTGGTGCAGTCTG |
| | | | |
| VH6 | VH6F | VH6-1 | CAGGTACAGCTGCAGCAGTCAG |
| | | | |
| VH7 | VH7F | VH7-81 | CAGGTGCAGCTGGTGCAGTCTGG |
| | | | |

| Reverse Primers | | | |
|---|---|---|---|
| Family: | Name: | V regions: | Sequence: |
| VH1 | VH1RA | VH1-45 | TATCTTGCACAGTAATACATGG |
| | VH1RB | VH1-58 | TCTGCCGCACAGTAATACACGGC |
| | VH1C*,3A,4B | VH1-46, VH1-2, VH1-69, VH1-18, VH3-53, VH3-11, VH4-4, VH4-59, VH4-61, VH4-31 | TCTCTCGCACAGTAATACACGG |
| | VHR1D,3C | VH1-3, VH3-30, VH3-33, VH3-7, VH3-66, VH3-21, VH3-64, VH3-48 | TCTCTCGCACAGTAATACACAGC |
| | VH1RE | VH1-24 | TCTGTTGCACAGTAATACACGGC |
| | VH1RF* | VH1-8 | CCTCTCGCACAGTAATACACGGC |
| | | | |
| VH2 | VH2RA | VH2-26 | GTATCCGTGCACAGTAATATGTGG |
| | VH2RB | VH2-70 | GTATCCGTGCACAATAATACG |
| | VH2RC | VH2-5 | GTCTGTGTGCACAGTAATATGTGG |
| | | | |
| VH3 | VH3RB | VH3-16, VH3-35 | TTTCTCACACAGTAATACACAGC |
| | VH3RD | VH3-74, VH3-13 | TCTCTTGCACAGTAATACACAGCC |
| | VH3RE | VH3-43, VH3-9 | TATCTTTTGCACAGTAATACAAGG |
| | VH3RG | VH3-23 | TCTTTCGCACAGTAATATACGGC |
| | VH3RH | VH3-15 | TCTGTGGTACAGTAATACACGG |
| | VH3RI | VH3-49 | TCTCTAGTACAGTAATACACGG |
| | VH3RJ | VH3-73 | TGTCTAGTACAGTAATACACGG |
| | VH3RK | VH3-72 | TCTCTAGCACAGTAATACACGG |
| | VH3RL | VH3-38 | TATCTGGCACAGTAATACACGGC |
| | | | |
| VH4 | VH4RA | VH4-39 | TGTCTCGCACAGTAATACACAGCC |
| | VH4RD | VH4-34 | CCTCTCGCACAGTAATACACAGC |
| | VH4RC | VH4-28 | TTTCTCGCACAGTAATACACGG |
| | | | |
| VH5 | VH5R | VH5-51 | TGTCTCGCACAGTAATACATGG |
| | | | |
| VH6 | VH6R | VH6-1 | TCTCTTGCACAGTAATACACAG |
| | | | |
| VH7 | VH7R | VH7-81 | TATCTCGCACAGTAATACATGG |

The rearrangement of a V minigene to either a D minigene or a J minigene requires polynucleotides encoding the D and/or J minigenes. D and J minigenes are typically less than 100 nucleotides long. Thus, these minigenes can be synthesized chemically, by standard oligonucleotide synthesis. The D and J minigenes may be single-stranded or double stranded. Sequences of human D and J minigenes are publicly available at the National Center for Biotechnology Information (NCBI), or in the literature [Ruiz, et al. Exp. Clin. Immunogenet. 16: 173-184(1999); Pallares, et al. Exp .Clin. Immunogenet. 16: 36-60(1999)]. Amino acid sequences ancoded by the minigenes are shown in FIGS XX-XX, thus any nucleotide sequence encoding the amino acid sequences of FIGS XX-XX can be considered V, D, or J minigenes, respectively. Efficient rearrangement of a V minigene to a D minigene can utilize a primer that can hybridize with both a V minigene and either a D or J minigene. The primer may hybridize at its 5' end with a V minigene and at its 3' end with a D or J minigene, or the primer may hybridize at its 3' end with a V minigene and at its 5' end with a D or J minigene. The primer may also be capable of hybridizing with both a D and J minigene. In fact, since several D minigenes are less than 30 nucleotides in length, a primer may include an entire D minigene between the sequences that can hybridize to a V and J minigene. In one embodiment, such a hybrid primer is utilized in an amplification reaction along with a back primer that can hybridize with the 5' end of the V minigene, and a forward primer capable of hybridizing to the 3' end of the J minigene as well as the J minigene sequence present in the hybrid primer. An amplification reaction can then occur, where the V minigene is ultimately fused to the D and/or J minigene in the final product. The success of the recombination can be determined using agarose gel electrophoresis as in FIG. 3 or FIG. 4, and comparing the size of the final product to the size of the original V minigene. Furthermore, the rearranged V(D)J V-region can be directly sequenced using standard techniques, or cloned into a plasmid vector for DNA sequencing or restriction enzyme analysis.

Given the description of the general principle and methodology used in producing polynucleotide sequences encoding germline V-regions, one of skill in the art would recognize that various modifications can be made to the methods specifically described herein and achieve essentially the same results.

### Light Chain

The method generally described above to generate rearranged antibody genes can be specifically applied to generate germline light chain antibody genes. Antibody light chains utilize only V minigenes and J minigenes. Thus, the hybrid primer described above should hybridize to both a germline V minigene and a germline J minigene. The V and J minigenes may be from either the Kappa or Lambda families, and should preferably be derived from a mammal. In order to produce a full length light chain V-region, a V minigene and a J minigene should be produced. One method to produce a light chain V minigene is to amplify the V minigene by a method such as PCR using genomic DNA as a template. A J minigene is typically less than 100 nucleotides and can be produced by standard oligonucleotide synthesis. The V or J minigenes can be single or double-stranded at this stage. A hybrid nucleic acid primer can be used which hybridizes to the V minigene as well as the J minigene. Preferably this primer is greater than 10 nucleotides. A reverse primer hybridizing to the 5' end of the V minigene and a forward primer hybridizing to the 3' end of the J minigene can also be included in the recombination reaction. The reaction can use PCR and standard amplification conditions. The success of the recombination can be determined using agarose gel electrophoresis as in FIG. 3 or FIG. 4, and comparing the size of the final product to the size of the original V minigene. Furthermore, the rearranged VJ light chain V-region can be directly sequenced using standard techniques, or cloned into a plasmid vector for DNA sequencing or restriction enzyme analysis.

### Heavy chain

The method generally described above to generate rearranged antibody germline genes can be specifically applied to generate germline heavy chain antibody genes. Antibody heavy chains typically utilize V, D, and J minigenes, however they could utilize only V and J minigenes. Additionally, more than one D-region may be used between V and J regions. Thus, the hybrid primer described above should hybridize to both a germline V minigene and a germline J minigene, but may additionally contain a region capable of hybridizing to a D minigene. The V, D, and J minigenes may be from should preferably be derived from a mammal. In order to produce a full length heavy chain V-region, a V, D, and J minigene should be produced. One method to produce a heavy chain V minigene is to amplify the V minigene by a method such as PCR using genomic DNA as a template. Both D and J minigenes are typically less than 100 nucleotides and can be produced by standard oligonucleotide synthesis. The D and J minigenes need not be produced as separate molecules. They could be produced as a single hybrid primer comprised of regions of homology to V, D, and J minigenes. The V, D, and J minigenes can be single or double-stranded at this stage. A hybrid nucleic acid primer can be used which hybridizes to the V minigene as well as the J minigene, and optionally including a region capable of hybridizing to a D minigene located between the V and J regions. Preferably this primer is greater than 10 nucleotides. A recombination reaction can then be performed containing at least two primers, but optionally containing three. A forward primer hybridizing to the 5' end of the V minigene and a reverse primer hybridizing to the 3' end of the J minigene can be included in the recombination reaction. The reaction can use PCR and standard amplification conditions. The success of the recombination can be determined using agarose gel electrophoresis as in FIG. 3 or FIG. 4, and comparing the size of the final product to the size of the original V minigene. Furthermore, the rearranged VJ light chain V-region can be directly sequenced using standard techniques, or cloned into a plasmid vector for DNA sequencing or restriction enzyme analysis.

### Degeneracy in CDRs

In performing the rearrangement reaction to produce a rearranged germline V-region, degeneracy may be present in the primer componenets such that diversity is generated in the final rearranged V-region. Codon based degeneracy is well known to those in the art and can be accomplished by standard techniques. Based on sequence homology, degenerate oligonucleotides can be designed as primer sets and PCR can be performed under suitable conditions (*see, e.g.,* White et al., PCR Protocols: Current Methods and Applications, 1993; Griffin and Griffin, PCR Technology, CRC Press Inc. 1994) to amplify a segment of nucleotide sequence from a human cDNA or genomic library.

### Mutagenesis

Following the generation of the germline V-region, further mutagenesis could be accomplished in order to enhance its binding affinity or another useful activity such as a catalytic function. Furthermore, a library can be created consisting of mutagenized versions of the parental germline V-region. Current methods in widespread use for creating mutant proteins in a library format are error-prone polymerase chain reaction [Caldwell and Joyce (1992); Gram, et al. Proc Natl Acad Sci 89: 3576-80(1992)] and cassette mutagenesis [Stemmer and Morris Biotechniques 13: 214-20(1992); Arkin and Youvan Proc Natl Acad Sci 89: 7811-5(1992); Oliphant, et al. Gene 44: 177-83(1986); Hermes, et al. Proc Natl Acad Sci 87: 696-700(1990)], in which the specific region to be optimized is replaced with a synthetically mutagenized oligonucleotide. Alternatively, mutator strains of host cells have been employed to add mutational frequency [Greener, et al. Mol Biotechnol 7: 189-95(1997)]. In each case, a 'mutant cloud' [Kauffman New York (1993)] is generated around certain sites in the original sequence.

Error-prone PCR uses low-fidelity polymerization conditions to introduce a low level of point mutations randomly over a long sequence. Error prone PCR can also be used to mutagenize a mixture of fragments of unknown sequence. Error-prone PCR can randomly mutate genes by altering the concentrations of respective dNTP's in the presence of dITP [Caldwell and Joyce (1992);Leung and Miyamoto Nucleic Acids Res 17: 1177-95(1989); Spee, et al. Nucleic Acids Res 21: 777-8(1993)]. Methods of saturation mutagenesis utilizing random or partially degenerate primers that incorporate restriction sites have also been described [Oliphant, et al. Gene 44: 177-83(1986); Hill, et al. Methods Enzymol 155: 558-68(1987); Reidhaar-Olson, et al. Methods Enzymol 208: 564-86(1991)].

"Cassette" mutagenesis is another method for creating libraries of mutant proteins [Hill, et al. Methods Enzymol 155: 558-68(1987); Shiraishi and Shimura Gene 64: 313-9(1988); Bock, et al. U S 5,830,720 (1995); Stemmer and Crameri U S 5,830,721 (1998); Miller, et al. U S 5,830,740 (1998); Christou and McCabe 5,830,728 (1998)]. Cassette mutagenesis typically replaces a sequence block length of a template with a partially randomized sequence. The maximum information content that can be obtained is thus limited statistically to the number of random sequences in the randomized portion of the cassette.

A protocol has also been developed by which synthesis of an oligonucleotide is "doped" with non-native phosphoramidites, resulting in randomization of the gene section targeted for random mutagenesis [Wang and Hoover J Bacteriol 179: 5812-9(1997)]. This method allows control of position selection, while retaining a random substitution rate.

Zaccolo and Gherardi (1999) describe a method of random mutagenesis utilizing pyrimidine and purine nucleoside analogs [Zaccolo and Gherardi J Mol Biol 285: 775-83(1999)]. This method was successful in achieving substitution mutations which rendered *β*-lactamase with an increased catalytic rate against the cephalosporin cefotaxime. Crea describes a "walk through" method, wherein a predetermined amino acid is introduced into a targeted sequence at pre-selected positions [Crea U S 5,798,208 (1998)].

The technique most often used to evolve proteins *in vitro* is known as "DNA Shuffling". In this method, a library of gene modifications is created by fragmenting homologous sequences of a gene, allowing the fragments to randomly anneal to one another, and filling in the overhangs with polymerase. A full length gene library is then reconstructed with polymerase chain reaction (PCR). The utility of this method occurs at the step of annealing, whereby homologous sequences may anneal to one another, producing sequences with attributes of both starting sequences. In effect, the method affects recombination between two or more genes that are homologous, but that contain significant differences at several positions. It has been shown that creation of the library using several homologous sequences allows a sampling of more sequence space than using a randomly mutated single starting sequence [Crameri, et al. Nature 391: 288-291(1998)]. This effect is likely due to the fact that years of evolution have already selected for different advantageous or neutral mutations amongst the homologs of the different species. Starting with homologs, then, appreciably limits the number of deleterious mutations in the creation of the library which is to be screened. Combinatorially rearranging the advantageous positions of the homologs can apparently allow for an optimized secondary protein structure for catalyzing a biochemical reaction. The resulting evolved protein appears to contain positive features contributed from each of the starting sequences, which results in drastically improved function following selection.

Alterations to the DNA shuffling technique have been devised. One process is termed the 'staggered extension' process, or StEP. Instead of reassembling the pool of fragments created by the extended primers, full-length genes are assembled directly in the presence of the template(s). The StEP consists of repeated cycles of denaturation followed by extremely abbreviated annealing/extension steps. In each cycle the extended fragments can anneal to different templates based on complementarity and extend a little further to create "recombinant cassettes." Due to this template switching, most of the polynucleotides contain sequences from different parental genes (i.e. are novel recombinants). This process is repeated until full-length genes form. It can be followed by an optional gene amplification step [Arnold, et al. U S 6,177,263 (2001)].

In another technique, fragmentation of the initial DNA can be accomplished by premature termination of the polymerase in an extension reaction by inducing adduct formation in the target gene [Short U S 5, 965, 408 (1999)]. In a different technique, a library is created by inducing incremental truncations in each of two homologs to produce a library of fusion genes, each of which contains domains donated from each homolog [Ostermeier, et al. Nat. Biotechnol. 17: 1205-1209(1999)]. The advantage of this approach is that significant homology amongst the starting sequences is not required since the annealing step of previous methods is omitted. It is unclear, however, whether this modified technique actually will lead to generation of improved gene function after selection techniques are applied to the library.

### Cloning into an expression vector

The nucleic acids encoding recombinant polypeptides of the present invention are typically cloned into an intermediate vector before transformation into prokaryotic or eukaryotic cells for replication and/or expression. The intermediate vector is typically a prokaryote vector such as a plasmid or shuttle vector.

To obtain high level expression of a cloned V-region one typically subclones the DNA into an expression vector that contains a strong promoter to direct transcription, a transcription/translation terminator, and a ribosome binding site for translational initiation. Additionally, the V-region may optionally be fused to a C-region to produce an antibody comprising constant regions. Suitable bacterial promoters are well known in the art and fully described in scientific literature such as Sambrook and Russell, *supra,* and Ausubel *et al, supra.* Bacterial expression systems for expressing antibody chains of the recombinant catalytic polypeptide are available in, *e.g*., *E. coli, Bacillus sp.,* and *Salmonella* (Palva et al., Gene, 22:229-235 (1983); Mosbach et al., Nature, 302:543-545 (1983)). Kits for such expression systems are commercially available. Eukaryotic expression systems for mammalian cells, yeast, and insect cells are well known in the art and are also commercially available.

Selection of the promoter used to direct expression of a heterologous nucleic acid depends on the particular application. The promoter is preferably positioned about the same distance from the heterologous transcription start site as it is from the transcription start site in its natural setting. As is known in the art, however, some variation in this distance can be accommodated without loss of promoter function.

In addition to the promoter, the expression vector typically contains a transcription unit or expression cassette that contains all the additional elements required for the expression of the antibody chain in host cells. A typical expression cassette thus contains a promoter operably linked to the nucleic acid sequence encoding the germline antibody chain and signals required for efficient polyadenylation of the transcript, ribosome binding sites, and translation termination. Additional elements of the cassette may include enhancers and, if genomic DNA is used as the structural gene, introns with functional splice donor and acceptor sites.

In addition to a promoter sequence, the expression cassette should also contain a transcription termination region downstream of the structural gene to provide for efficient termination. The termination region may be obtained from the same gene as the promoter sequence or may be obtained from different genes.

The particular expression vector used to transport the genetic information into the cell is not particularly critical. Any of the conventional vectors used for expression in eukaryotic or prokaryotic cells may be used. Standard bacterial expression vectors include plasmids such as pBR322 based plasmids, pSKF, pET23D, and fusion expression systems such as MBP, GST, and LacZ. Epitope tags can also be added to recombinant proteins to provide convenient methods of isolation, *e.g*., c-myc or histidine tags.

Expression vectors containing regulatory elements from eukaryotic viruses are typically used in eukaryotic expression vectors, *e.g*., SV40 vectors, papilloma virus vectors, and vectors derived from Epstein-Barr virus. Other exemplary eukaryotic vectors include pMSG, pAV009/A⁺, pMTO10/A⁺, pMAMneo-5, baculovirus pDSVE, and any other vector allowing expression of proteins under the direction of the CMV promoter, SV40 early promoter, SV40 later promoter, metallothionein promoter, murine mammary tumor virus promoter, Rous sarcoma virus promoter, polyhedrin promoter, or other promoters shown effective for expression in eukaryotic cells.

Some expression systems have markers that provide gene amplification such as thymidine kinase and dihydrofolate reductase. Alternatively, high yield expression systems not involving gene amplification are also suitable, such as using a baculovirus vector in insect cells, with a nucleic acid sequence encoding a germline antibody chain under the direction of the polyhedrin promoter or other strong baculovirus promoters.

The elements that are typically included in expression vectors also include a replicon that functions in *E. coli,* a gene encoding antibiotic resistance to permit selection of bacteria that harbor recombinant plasmids, and unique restriction sites in nonessential regions of the plasmid to allow insertion of eukaryotic sequences. The particular antibiotic resistance gene chosen is not critical, any of the many resistance genes known in the art are suitable. The prokaryotic sequences are preferably chosen such that they do not interfere with the replication of the DNA in eukaryotic cells, if necessary.

### Transfection of germline V-regions

Standard transfection methods are used to produce bacterial, mammalian, yeast, or insect cell lines that express large quantity of antibody chains, which is then purified using standard techniques *(see, e.g.,* Colley et al., J. Biol. Chem., 264:17619-17622 (1989); Guide to Protein Purification, in Methods in Enzymology, vol. 182 (Deutscher, ed.), 1990). Transformation of eukaryotic and prokaryotic cells are performed according to standard techniques *(see, e.g.,* Morrison, J. Bact., 132:349-351 (1977); Clark-Curtiss and Curtiss, Methods in Enzymology, 101:347-362 (Wu et al., eds), (1983)).

Any of the well-known procedures for introducing foreign nucleotide sequences into host cells may be used. These include the use of calcium phosphate transfection, polybrene, protoplast fusion, electroporation, biolistics, liposomes, microinjection, plasma vectors, viral vectors and any of the other well known methods for introducing cloned genomic DNA, cDNA, synthetic DNA, or other foreign genetic material into a host cell *(see, e.g.,* Sambrook and Russell, *supra*). It is only necessary that the particular genetic engineering procedure used be capable of successfully introducing at least both genes into the host cell capable of expressing germline antibody polypeptide.

After the expression vector is introduced into the cells, the transfected cells are cultured under conditions favoring expression of the germline antibody chain, which is recovered from the culture using standard techniques identified below.

### Library Formats

A current focus of interest in molecular biology and biotechnology is in the display of large libraries of proteins and peptides and in means of searching them by affinity selection. The key to genetic exploitation of a selection method is a physical link between individual molecules of the library (phenotype) and the genetic information encoding them (genotype). The libraries of the present invention can be prepared in a number of formats, including those described below.

A number of cell-based methods are available, such as on the surfaces of phages (Smith, G. P. (1985) Science 228 1315-1317), bacteria (Georgiou, G., et.al. (1993) TIBTECH 11 6-10.) and animal viruses (Kasahara, N et.al. (1994) Science 266, 1373-1376). Of these, the most widely used is phage display, in which proteins or peptides are expressed individually on the surface of phage as fusions to a coat protein, while the same phage particle carries the DNA encoding the protein or peptide. Selection of the phage is achieved through a specific binding reaction involving recognition of the protein or peptide, enabling the particular phage to be isolated and cloned and the DNA for the protein or peptide to be recovered and propagated or expressed.

A particularly desirable application of display technology is the selection of antibody combining sites from combinatorial libraries. Screening for high affinity antibodies to specific antigens has been widely carried out by phage display of antibody fragments (Winter, G.et.al.(1994) Annu. Rev. Immunol. 12, 433-455). Combinations of the variable (V) regions of heavy (H) and light (L) chains are displayed on the phage surface and recombinant phage are selected by binding to immobilized antigen. Single-chain (sc) Fv fragments, in which the V_{H} and V_{L} domains are linked by a flexible linker peptide, have been widely used to construct such libraries. Another type of single chain antibody fragment is termed V_{H} /K, in which the V_{H} domain is linked to the complete light chain, i.e. V_{H} -linker-V_{L} -C_{L} (He, M. et.al. (1995) Immunology 84, 662-668.). This has a number of advantages, including stability of expression in E. coli and the use of the C_{L} domain as a spacer and as a tag in detection systems such as ELISA and Western blotting. Antibody V_{H} and V_{L} region genes are readily obtained by the methods of the current invention. Single chain antibody libraries are potentially of a size of >10¹⁰ members. Libraries can also be generated by mutagenesis of cloned DNA fragments encoding specific V_{H} /V_{L} combinations and screened for mutants having improved properties of affinity or specificity. Mutagenesis is carried out preferably on the CDR regions, and particularly on the highly variable H-CDR3, where the potential number of variants which could be constructed from a region of 10 amino acids is 20¹⁰ or 10¹³.

One such method is the display of proteins or peptides in nascent form on the surface of ribosomes, such that a stable complex with the encoding mRNA is also formed; the complexes are selected with a ligand for the protein or peptide and the genetic information obtained by reverse transcription of the isolated mRNA. This is known as ribosome or polysome display. A description of such a method is to be found in two U.S. patents, granted to G. Kawasaki/Optein Inc. (Kawasaki, G. U.S. Pat. Nos. 5,643,768 Cell free synthesis and isolation of novel genes and polypeptides (Jul. 1, 1997) and 5,658,754 (Aug. 19, 1997)).

A further recent display method was described by Roberts and Szostak (Roberts R. W. and Szostak J. W. (1997) Proc. Nat. Acad. Sci USA 94, 12297-12302), in which the nascent protein is caused to bind covalently to its mRNA through a puromycin link (termed RNA display). In this system, selection is carried out on these protein-mRNA fusions after dissociation of the ribosome.

### Detection of cells expressing germline antibody genes

Following the transfection procedure, cells are screened for the expression of antibody chains of the recombinant germline antibody polypeptides.

Several general methods for screening gene expression are well known among those skilled in the art. First, gene expression can be detected at nucleic acid level. A variety of methods of specific DNA and RNA measurement using nucleic acid hybridization techniques are commonly used *(e.g.,* Sambrook and Russell, *supra*). Some methods involve an electrophoretic separation (e.g., Southern blot for detecting DNA and Northern blot for detecting RNA), but detection of DNA or RNA can be carried out without electrophoresis as well (such as by dot blot). The presence of nucleic acid encoding recombinant germline antibodies in transfected cells can also be detected by PCR or RT-PCR using sequence-specific primers.

Second, gene expression can be detected at the polypeptide level. Various immunological assays are routinely used by those skilled in the art to measure the level of a gene product, particularly using polyclonal or monoclonal antibodies that react specifically with a recombinant polypeptide of the present invention, such as an antibody light chain or heavy chain *(e.g.,* Harlow and Lane, Antibodies, A Laboratory Manual, Chapter 14, Cold Spring Harbor, 1988; Kohler and Milstein, Nature, 256:495-497 (1975)). Such techniques require antibody preparation by selecting antibodies with high specificity against the recombinant polypeptide or an antigenic portion thereof. The methods of raising polyclonal and monoclonal antibodies are well established and their descriptions can be found in the literature, *see, e.g*., Harlow and Lane, *supra*; Kohler and Milstein, Eur. J. Immunol., 6:511-519 (1976).

### Producing and purifying protein

Antibody chains of the present invention can be purified for use in functional assays. The recombinant germline antibodies of the invention may be purified to substantial purity by standard techniques, including selective precipitation with such substances as ammonium sulfate; column chromatography, gel filtration, immunopurification methods, and others (*see, e.g.,* U.S. Patent No. 4,673,641; Scopes, Protein Purification: Principles and Practice, 1982*;* Sambrook and Russell, *supra*; and Ausubel et al., *supra*).

A number of procedures can be employed when recombinant germline antibodies are purified. For example, proteins having established molecular adhesion properties can be reversibly fused to polypeptides of the invention. With the appropriate ligand, the polypeptides can be selectively adsorbed to a purification column and then freed from the column in a relatively pure form. The fused protein is then removed by enzymatic cleavage. Finally the polypeptide can be purified using affinity columns.

When recombinant polypeptides are expressed by the transformed bacteria in large amounts, typically after promoter induction, although expression can be constitutive, the polypeptides may form insoluble aggregates. There are several protocols that are suitable for purification of polypeptide inclusion bodies and are described in detail in numerous scientific publications (such as Sambrook and Russell, *supra*, and Ausubel *et al., supra*). Numerous variations will be apparent to those of skill in the art.

The cell suspension is generally centrifuged and the pellet containing the inclusion bodies resuspended in buffer which does not dissolve but washes the inclusion bodies, *e.g*., 20 mM Tris-HCl (pH 7.2), 1 mM EDTA, 150 mM NaCl and 2% Triton-X 100, a non-ionic detergent. It may be necessary to repeat the wash step to remove as much cellular debris as possible. The remaining pellet of inclusion bodies may be resuspended in an appropriate buffer (*e.g*., 20 mM sodium phosphate, pH 6.8,150 mM NaCl). Other appropriate buffers will be apparent to those of skill in the art.

Alternatively, it is possible to purify recombinant germline antibody polypeptides from bacteria periplasm. Where the polypeptide is exported into the periplasm of the bacteria, the periplasmic fraction of the bacteria can be isolated by cold osmotic shock in addition to other methods known to those of skill in the art *(e.g.,* Ausubel et al., *supra*). To isolate recombinant polypeptides from the periplasm, the bacterial cells are centrifuged to form a pellet. The pellet is resuspended in a buffer containing 20% sucrose. To lyse the cells, the bacteria are centrifuged and the pellet is resuspended in ice-cold 5 mM MgSO₄ and kept in an ice bath for approximately 10 minutes. The cell suspension is centrifuged and the supernatant decanted and saved. The recombinant polypeptides present in the supernatant can be separated from the host proteins by standard separation techniques well known to those of skill in the art. These methods include, but are not limited to, the following steps: solubility fractionation, size differential filtration, and column chromatography.

### Operably Joining Antibody Light Chain and Antibody Heavy Chain

There is particular value in joining a germline antibody polypeptide to a second antibody polypeptide, wherein the second polypeptide is either a germline antibody or a non-germline antibody polypeptide. There are several methods to join the antibody light chain and heavy chain of the recombinant germline antibodies. For example, one skilled in the art will recognize that when genes encoding two antibody chains are expressed in transfected cells simultaneously, they will be joined during the process. The two antibody chains may also be joined at nucleic acid level or at polypeptide level, before or after their expression.

### Recombinant Methods

An antibody light chain and an antibody heavy chain can be joined by recombinant DNA technology prior to their expression (*see*, *e.g*., Chaudhary et al, Nature, 339:394-397 (1989); Pantoliano et al., Biochemistry, 30:10117-10125 (1991); Kim et al., Mol. Immunol., 34:891-906 (1997)). As a person of ordinary skill in the art will know, a polynucleotide sequence can be introduced to connect the coding sequences for the antibody light and heavy chains (e.g. to construct a scFv) by employing various tools and techniques such as enzymatic digestion/ligation and /or PCR. The precise length of the insertion is essential in that the open reading frame of the coding sequence down stream from the insertion should not be disrupted. Upon transfection and expression, one single polypeptide is generated, which contains both the antibody light and heavy chains and a peptide linker of appropriate length joining them.

### Chemical Methods

The two antibody chains may also be joined by chemical means following their expression and purification. Chemical modifications include, for example, derivitization for the purpose of linking the antibody chains to each other, either directly or through a linking compound, by methods that are well known in the art of protein chemistry. Both covalent and noncovalent attachment means may be used with the recombinant germline antibodies of the present invention.

The procedure for linking the two antibody chains will vary according to the chemical structure of the moieties where the chains are joined. As a polypeptide one antibody chain typically contain a variety of functional groups such as carboxylic acid (-COOH), free amine (-NH₂), or sulfhydryl (-SH) groups, which are available for reaction with a suitable functional group on the other antibody chain to result in a linkage.

Alternatively, one antibody chain can be derivatized to expose or to attach additional reactive functional groups. The derivatization may involve attachment of any of a number of linker molecules such as those available from Pierce Chemical Company, Rockford Illinois. The linker is capable of forming covalent bonds to both antibody chains. Suitable linkers are well known to those of skill in the art and include, but are not limited to, straight or branched-chain carbon linkers, heterocyclic carbon linkers, or peptide linkers. Since the antibody chains are polypeptides, the linkers may be joined to the constituent amino acids through their side groups (for example, through a disulfide linkage to cysteine). The linkers may also be joined to the alpha carbon amino and carboxyl groups of the terminal amino acids.

### Cellular Methods

Hybridoma cells can be generated by fusing B cells producing a desired antibody with an immortalized cell line, usually a myeloma cell line, so that the resulting fusion cells will be an immortalized cell line that secrets a particular antibody. By the same principle, myeloma cells can be first transfected with a nucleic acid encoding a germline antibody V-region and can be screened for the expression of the germline V-region. Those myeloma cells with highest level of proteolytic light chain expression can be subsequently fused with B cells that produce an antibody with desired target protein specificity. The fusion cells will produce two types of antibodies: one is a heterologous antibody containing an endogenous antibody chain (either heavy or light) operably joined to the recombinant germline V-region (either heavy or light), and the other is the same antibody that the parental B cells would secrete (*e.g*. both endogenous heavy and light chains). The operably joined heterologous heavy and light chains can be isolated by conventional methods such as chromatography and identification can be confirmed by target protein binding assays, assays identifying a unique tag of the germline polypeptide, or endopeptidase activity assays described in other sections of this disclosure. In some cases, where the heterologous antibody is the predominant type in quantity among the two types of antibodies, such isolation may not be needed.

### Protease Activity

Several assays are available to determine whether an antibody polypeptide contains endopeptidase activity. Generally, any assay that can detect hydrolysis of a secondary amide bond may be used to determine endopeptidase activity. Commonly used assays utilize peptide analogs conjugated to reporter molecules that can be detected when released from the peptide. A commonly used assay involves a peptide-methylcoumarinamide (MCA) derivative, such that hydrolysis of the peptide-MCA bond produces the leaving group aminomethylcoumarin whose fluorescence is measured at an excitation of 370 nm and an emmission of 460 nm. Such an assay has been practiced to detect proteolytic activity of murine light chains (Gao, et al, J. Biol. Chem. 269:32389-32393 (1994); Sun et al, J. Mol. Biol. 271:374-385 (1997)). Other similar methods are known in the art to conjugate peptides to molecules that have altered spectral properties when they are cleaved (*e.g*., nitroaniline conjugates).

Any method that allows detection of a cleaved peptide bond in a target protein is suitable for use in the present invention. Since hydrolysis of a peptide bond necessarily produces more that one polypeptide product, several standard size or mass analysis techniques well known in the art can be used to identify peptide bond hydrolysis. These techniques include electrophoretic mobility techniques such as SDS polyacrylamide gel electrophoresis, high performance liquid chromatography (HPLC), and mass spectrometry methods such as MALDI-TOF. Alternatively, a protein labeled with a radioisotope can be precipitated in TCA, wherein hydrolysis of a peptide bond will be indicated by the amount of TCA soluble radioactivity (Gao, et al, J. Biol. Chem. 269: 32389-32393 (1994)). Other methods for detecting target protein hydrolysis include coupling a labeled target protein to a solid support, and measuring release of the labeled protein following exposure to the catalytic polypeptide. Furthermore, Smith and Kohorn (PNAS 88: 5159-5162 (1991)), Lawler and Snyder (Anal. Biochem. 269: 133-138 (1999)), Dasmahaptra, et al (PNAS 89: 4159-4162(1992)), Murray, et al (Gene 134: 123-128 (1993)), and Kim, et al (Biochem. Biophys. Res. Commun. 296: 419 (2002)) describe genetic mechanisms for detecting proteolytic activity using variants of the yeast two-hybrid system. This system could be modified to accommodate recombinant germline antibodies of the present invention.

### Non-Human Transgenic Mammals

A nucleic acid sequence encoding a germline antibody polypeptide of the present invention can be introduced into a non-human mammal to generate a transgenic animal that expresses the germline antibody polypeptide. Unlike the transgenic animal models more commonly seen, the transgene expressed by the transgenic mammals of the present invention need not replace at least one allele of the endogenous coding sequence responsible for the variable regions of antibody chains following somatic recombination. Due to allelic exclusion, the presence of an exogenous, post-somatic rearrangement version of the germline V-region DNA will inhibit the endogenous alleles ofpre-somatic rearrangement V minigenes from undergoing somatic rearrangement and contributing to the makeup of antibody chains this mammal may produce. Thus, when exposed to a particular antigen, the mammal will generate heterologous antibodies comprising one endogenously rearranged antibody chain, and one transgenic gene which was rearranged *a priori.* Such heterologous antibodies are invaluable in research and in treating certain conditions in live subjects. On the other hand, a method that directs the integration of the transgene to the locus of an endogenous allele will fully serve the purpose of practicing the present invention as well.

The general methods of generating transgenic animals have been well established and frequently practiced. For reviews and protocols for generating transgenic animals and related methods for genetic manipulations, *see, e.g.,* Mansour et al., Nature 336:348-352 (1988); Capecchi et al., Trends Genet. 5:70-76 (1989); Capecchi, Science 244:1288-1292 (1989); Capecchi et al., Current Communications in Molecular Biology, pp45-52, Capecchi, M.R. (ed.), Cold Spring Harbor Press, Cold Spring Harbor, N.Y. (1989); Frohman et al., Cell 56: 145-147 (1989); Brinster et al., Proc. Natl. Acad. Sci. USA 82:4438-4442 (1985); Evans et. al., Nature 292:154-156 (1981); Bradley et al., Nature 309:255-258 (1984); Gossler et al., Proc. Natl. Acad. Sci. USA 83:9065-9069 (1986); Robertson et al., Nature 322:445-448 (1986); Jaenisch Science 240:1468-1474 (1988); and Siedel, G. E., Jr., "Critical review of embryo transfer procedures with cattle" in Fertilization and Embryonic Development in Vitro, page 323, L. Mastroianni, Jr. and J. D. Biggers, ed., Plenum Press, New York, N.Y. (1981).

An exemplary transgenic animal of the present invention is mouse, whereas a number of other transgenic animals can also be produced using the same general method. These animals include, but are not limited to: rabbits, sheep, cattle, and pigs (Jaenisch Science 240:1468-1474 (1988); Hammer et al., J. Animal. Sci. 63:269 (1986); Hammer et al. Nature 315:680 (1985); Wagner et al., Theriogenology 21:29 (1984)).

### Addressable Arrays

Of particular note are spatially addressable arrays (i.e., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one germline antibody V-region, or an amino acid sequence encoded thereby. Methods for attaching biopolymers to, or synthesizing biopolymers on, solid support matrices, and conducting binding studies thereon are disclosed in, *inter alia,* U.S. Pat. Nos. 5,700,637, 5,556,752, 5,744,305, 4,631,211, 5,445,934, 5,252,743, 4,713,326, 5,424,186, and 4,689,405 the-disclosures of which are herein incorporated by reference in their entirety.

Addressable arrays comprising germline antibody V-regions can be used to identify and characterize the temporal and tissue specific expression of an antibody as well as analyze its affinity for a given antigen. These addressable arrays incorporate oligonucleotide or peptide sequences of sufficient length to confer the required specificity, yet be within the limitations of the production technology. The length of these probes is within a range of between about 8 to about 2000 nucleotides. Preferably the probes consist of 60 nucleotides and more preferably 25 nucleotides from the germline antibody V-regions.

For example, a series of the described oligonucleotide sequences, or the complements thereof, can be used in chip format to represent all or a portion of the germline antibody repertoire. The oligonucleotides, typically between about 16 to about 40 (or any whole number within the stated range) nucleotides in length can partially overlap each other and/or the sequence may be represented using oligonucleotides that do not overlap. Accordingly, the described polynucleotide sequences shall typically comprise at least about two or three distinct oligonucleotide sequences of at least about 8 nucleotides in length that encode an antibody germline V-region. Such oligonucleotide sequences can begin at any nucleotide present within a germline V-region and proceed in either a sense (5'-to-3') orientation vis-a-vis the described sequence or in an antisense orientation.

Microarray-based analysis allows the discovery of broad patterns of genetic activity, providing new understanding of gene functions and generating novel and unexpected insight into transcriptional processes and biological mechanisms.

### EXAMPLES

The following examples are provided by way of illustration only and not by way of limitation. Those of skill in the art will readily recognize a variety of non-critical parameters that could be changed or modified to yield essentially similar results.

### Rearranging A18b and A2c human light chains

The V minigenes for human A18b or A2c were amplified from genomic DNA using primers hybridizing to the 5' and 3' ends of the minigenes. Primers were annealed to 100 ng of human genomic fibroblast DNA at 56°C for 30 seconds, followed by extension at 70°C for 30 seconds and denaturation at 94°C for 30 seconds. Thirty thermocycles following this pattern were completed. In a subsequent "joining" reaction, a primer comprising the 3' sequence of A18b or A2c and the 5' region of human JK1 was included in a PCR reaction along with a back primer specific for the 5' end of either A18b or A2c, as well as a forward primer specific for the 3' end of JK1. The 3' forward primer included a BsiWI restriction site that allowed fusion to the human CK gene. The 5' back primer included an Sfi I site that allowed cloning into a bacterial periplasmic expression vector containing the CK constant region fused to six histidines at the C-termmus. Amplification conditions were the same as described above. The rearranged antibody genes were cloned into the vector and expressed as described below.

### Expression and purification

The A18b and A2c genes in a pCANTAB derived vector (Amersham) were electroporated into *E.coli* strain TOP10F', single colonies were isolated and grown at 30°C for 12 hrs in Luria-Bertani broth, and expression induced with 100 mM IPTG for 8 hours. Periplasmic extracts were prepared by osmotic lysis, and subjected to two rounds of immobilized metal chelate chromatography to purify the antibody light chains.

### Protease Activity

Protease activity was determined by incubating 200 ng of recombinant germline antibody with PFR-MCA protease substrate for 14 hours. Peptide hydrolysis was determined by measuring fluorescence of the methylcoumarinamide (MCA) leaving group at ex370/em465. Activity was quantitated using known MCA concentrations to produce a standard curve.

### Rearranging 3-30 VH human heavy chains

The V minigenes for human 3-30 V were amplified from genomic DNA or plasmid containing 3-30 V, using primers hybridizing to the 5' and 3' ends of the minigenes. PCR was performed on 100 ng of gDNA or 10 ng of plasmid RF26 at 63 °C primer annealing for 35 cycles. Figure 7 shows a standard 3-oligo joining reaction comprised of the 3-30 V minigenes as template (1/10 volume of original 3-30 V PCR), the 3-30 R primer from the 5' end of 3-30 V, joining oligos 3 or 4 which are partially complementary to the 3' end of the 3-30 V minigenes and to the 5' end of the J-region primer, and the J-region primer JH4 Nhe-I/Not-I with cloning sites and which is the reverse PCR primer for assembled recombined VDJ products. Figure 9 shows a 2-oligo joining reaction which is identical to the above except that the 3-30 R primer for the 5'end of the 3-30 V minigenes was left out. Conversion of the 3-30 V template to recombined 3-30 VDJ product occurred with increasing input amounts of the 3-30 V template. The oligonucleotide primers used in VDJ recombinations described in this example are listed in Table 1.

**Table 1: 3-30 VDJ oligos**

| **Oligo** | **Sequence 5'** | **Comment** |
|---|---|---|
| **3-30F** | GTAGTGATTTGGCCCAGCCGGCCAGGTGCAGCTG GTGGAGTCTGGGG | 3-30 VH gene primer / forward primer of assembled VDJ products / cloning sites |
| **3-30R** | CTTTCGCACAGTAATACACAGCCGTG | 3-30 VH gene primer |
| **3-30joinD1-26** | GTATTACTGTGCGAAAGGGTATAGTGGGAGCTAC TACTACTTTGACTACTGGGG | no degeneracy |
| **3-30join2** | GTATTACTGTGCGAAAGNNTATAGTGGGAGCTAC NNCTACTTTGACTACTGGGG | degeneracy at 2 amino acids |
| **3-30join3** | GTATTACTGTGCGAAAGNNTATAGTGGGAGCTAC NNCNNKTACTTTGACTACTGGGG | degeneracy at 3 amino acids; length increased by 1 amino acid |
| **3**-**30join4** | GTATTACTGTGCGAAANNKNNKNNKNNKNNKNN KTACTTTGACTACTGGGG | degeneracy at 6 amino acids |
| **JH4**-**Nhe**/**Not** | AGCCATCGCGGCCGCGCTAGCTGAGGAGACGATG ACCAGGGTTCCTTGGCCCCAGTAGTCAAAG | IGHJ4 gene primer / reverse primer for assembled VDJ products /cloning sites |

| | | |
|---|---|---|
| Note: "N" = any of 4 nucleotides (A, C, G, T) Note: "K" = any of 2 nucleotides (G, T) | | |

### Adding V-region diversity through PCR from genomic DNA

Many V-regions are highly homologous to each other. Changing the stringency of a primer pair annealing to genomic DNA in PCR will result in different populations of V minigenes for each PCR condition. We performed VDJ recombination on 2 different populations of 3-30 VH minigenes-one derived from PCR at 56°C and the other from PCR at 63 °C (Figure 7 & 8). Sequencing results for the 2 different populations of VDJ rearranged clones are shown within Table 2. Reduced stringency PCR (56°C) resulted in 5 of 5 clones (J4A-J4E) having unique V-regions from 2 different heavy chain families (1 & 3). In contrast, 6 clones (L1A-L1F) from higher stringency PCR (63°C), were limited to 3 V-regions from a single heavy chain family (3) which has 22 V-regions.

**Table 2: Sequence Analysis of 3-30 VDJ clones**

| **Clone** | **heavy chain ID** | **ID name** | **Diversity sequence** | **Diversity sequence translation** | **Joining Oligo** |
|---|---|---|---|---|---|
| **V template: plasmid RF26, join primer: D1-26,** **Figure 6** | | | | | |
| D126electronic | IGHV3-30*18 | IGHD1-26*01 | tgt gcg aaa ggg tat agt ggg agc tac tac tac ttt gac tac tgg | CAKGYSGSYYYFDYW | |
| D126A | IGHV3-30*18 | IGHD1-26*01 | tgt gcg aaa ggg tat agt ggg agc tac tac tac ttt gac tac tgg | CAKGYSGSYYYFDYW | 3-30 joinD1-26 |
| D126B | IGHV3-30*1 | IGHD1-26*01 | tgt gcg aaa ggg tat agt ggg agc tac tac tac ttt gac tac tgg | CAKGYSGSYYYFDYW | 330 joinD1-26 |

| **V template: plasmid RF26, join primer: join 3&4,** **Figure 7** | | | | | |
|---|---|---|---|---|---|
| L2A | IGHV3-30*18 | IGHD1-26*01 | tgt gcg aaa gta tat agt ggg agc tac gtc gag tac ttt gac tac tgg | CAKVYSGSYVEYFDW | 3-30join3 |
| L2B | IGHV3-30*18 | IGHD1-26*01 | tgt gcg aaa gat agt ggg agc tac ggc gat tac ttt gac tac tga | CAKDSGSYGDYFDW* | 3-30join4 |
| L2C | IGHV3-30*18 | IGHD3-16*01 | tgt gcg aaa att acg gcg gag gag gtg tac ttt gac tac tgg | CAKITAEEVYFDYW | 3-30join4 |
| L2D | IGHV3-30*18 | IGHD3-3*01 | tgt gcg aaa cgg cag agg atg ttt gtt gnn tac ttt gac tac tgg | CAKRQRMFVXYFDYW | 3-30join3 |
| L2E | IGHV3-30*18 | IGHD1-26*01 | tgt gcg aaa gcc tat agt ggg agc tac gtc ggt tac ttt gac tac tgg | CAKAYSGSYVGYFDYW | 3-30join3 |
| L2F | IGHV3-30*18 | IGHD1-26*01 | tgt gcg aaa gat tat agt ggg agc tac ncc tag tac ttt gac tac tgg | CAKDYSGSYX*YFDYW | 3-30join3 |

| **V template: human gDNA @ 63°C, join primer: join 3&4,** **Figure 7** | | | | | |
|---|---|---|---|---|---|
| L1A | IGHV3-30*14 | IGHD2-8*01 | tgt gcg aaa atg gtg tcg gcg agg ttg tac ttt gac tac tgg | CAKMVSARLYFDYW | 3-30join4 |
| L1B | IGHV3-33*04 | IGHD2-2*01 | tgt gcg aaa ggg ttg aag tan atg aat tac ttt gac tac tgg | CAKGLKXMNYFDYW | 3-30join4 |
| L1C | IGHV3-11*01 | IGHD3-10*01 | tgt gcg aaa tat ggt gtg ggg cgg gag tac ttt gac tac tgg | CAKYGVGREYFDYW | 3-30join4 |
| L1D | IGHV3-33*01 | IGHD1-26*01 | tgt gcg aaa ggg tat agt ggg agc tac ngc tat tac ttt gac tac tgg | CAKGYSGSYXYYFDYW | 3-30join3 |
| L1E | IGHV3-30*18 | IGHD1-26*01 | tgt gcg aaa gat tat agt ggg agc tac ggc atg tac ttt gac tac tgg | CAKDYSGSYGMYFDYW | 3-30join3 |
| L1F | IGHV3-30*19 | IGHD1-1*01 | tgt gcg aaa gcn aag ggt act acg ggg tac ttt gac tac tgg | CAKXKGTTGYFDYW | 3-30join4 |

| **V template: human gDNA @ 56°C, join primer: join 4,** **Figure 8** | | | | | |
|---|---|---|---|---|---|
| J4A | IGHV3-66*02 | IGHD2-21*01 | tgt gcg aaa att ggt cat cgg tgt tct tac ttt gac tac tgg | CAKIGHRCSYFDYW | 3-30join4 |
| J4B | IGHV1-46*03 | IGHD3-9*01 | tgt gcg aaa tat tgg gat agg ttg gcg tac ttt gac tac tgg | CAKYWDRLAYFDYW | 3-30join4 |
| J4C | IGHV1-2*02 | IGHD2-21*01 | tgt gcg aaa tgg ggt ggt tag cgg cgg tac ttt gac tac tgg | CAKWGG*RRYFDYW | 3-30join4 |
| J4D | IGHV3-35*01 | IGHD2-15*01 | tgt gcg aaa acg gtg ccg gtt gct gct tac ttt gac tac tgg | CAKTVPVAAYFDYW | 3-30join4 |
| J4E | IGHV1-69*01 | IGHD2-8*02 | tgt gcg aaa cag cgg cgt gtg cct gcg tac ttt gac tac tgg | CAKQRRVPAYFDYW | 3-30join4 |

| **V template: plasmid RF26**, **join primer: join 4,** **Figure 9** | | | | | |
|---|---|---|---|---|---|
| L3A | IGHV3-30*18 | IGHD6-19*01 | tgt gcg aaa gtg ctg agg ctg ggg acg tac ttt gac tac tgg | CAKVLRLGTYFDYW | 3-30join4 |
| L3B | IGHV3-30*18 | truncated sequence | truncated sequence | truncated sequence | |
| L3C | IGHV3-30*18 | IGHD1-26*01 | tgt gcg aaa gat agt ggg agc tac tcc cct ggt tac tgg | CAKDSGSYSPGYW | 3-30join4 |
| L3D | IGHV3-30*18 | IGHD2-8*01 | tgt gcg aaa gag ggg agg atg tax act tac ttt gac tac tgg | CAKEGRMXTYFDYW | 3-30join4 |
| L3E | IGHV3-30*18 | IGHD2-8*01 | tgt gcg aaa gxg gax atg ggg txt ggg tac ttt gac tac tgg | CAKXXMG?GYFDYW | 3-30join4 |

All patents, patent applications, and other publications cited in this application, including published amino acid or polynucleotide sequences, are incorporated by reference in the entirety for all purposes.

### Embodiments of the invention

1. A method for producing a polynucleotide encoding a human germline antibody V-region, comprising the steps of:
   (a) obtaining a V minigene or a J minigene; and
   (b) joining the V minigene with at least one J minigene, or joining the J minigenes with a V minigene, wherein the J minigene is located at the 3' end of the V minigene.
2. The method of embodiment 1, wherein a D minigene is further joined to the 3' 2 end of the V minigene and the 5' end of the J minigene.
3. The method of embodiment 1, wherein the V minigene or the J minigene in step (a) is obtained by chemical synthesis.
4. The method of embodiment 1, wherein the V minigene or the J minigene in 2 step (a) is obtained by amplification from a germline DNA library.
5. The method of embodiment 1, wherein step (b) is performed by primer extension using at least two oligonucleotide primers.
6. The method of embodiment 2, wherein step (b) is performed by primer extension using at least three oligonucleotide primers.
7. The method of embodiment 5, wherein one of the primers comprises homology to both the V minigene and the J minigene.
8. The method of embodiment 6, wherein one of the primers comprises homology to both the V minigene and the D minigene.
9. The method of embodiment 6, wherein one of the primers comprises homology to both the D minigene and the J minigene.
10. The method of embodiment 6, wherein at least one of the oligonucleotide primers comprises degeneracy at one nucleotide position.
11. The method of embodiment 1, wherein the V minigene is derived from human immunoglobulin kappa locus.
12. The method of embodiment 1, wherein the V minigene is derived from human immunoglobulin lambda locus.
13. The method of embodiment 1, wherein the V minigene is derived from human immunoglobulin heavy chain locus.
14. The method of embodiment 1, wherein the V-region comprises a serine protease triad.
15. A library comprising member polynucleotides encoding exogenously rearranged human germline antibody V-regions.
16. The library of embodiment 15, wherein the germline V -regions are light chain V-regions.
17. The library of embodiment 16, wherein each of the light chain V-regions is operably linked to an endogenously rearranged heavy chain V -region.
18. The library of embodiment 15, wherein the germline V-regions are heavy chain V-regions.
19. The library of embodiment 18, wherein each of the heavy chain V-regions is operably linked to an endogenously rearranged light chain V -region.
20. The library of embodiment 15, wherein the germline V-regions comprise 2 operably linked heavy chain and light chain V-regions.
21. The library of embodiment 15, which is a phage library.
22. The library of embodiment 15, which resides in a eukaryotic cell.
23. The library of embodiment 15, which is a ribosome display library.
24. The library of embodiment 15, which is an RNA display library.
25. The library of embodiment 15, which is a plasmid display library.

## Claims

1. A spatially addressable library comprising member polynucleotides encoding exogenously rearranged human germline antibody V-regions or corresponding polypeptides.

2. The spatially addressable library of claim 1, wherein the germline V-regions are light chain V-regions, wherein optionally each of the light chain V-regions is operably linked to an endogenously rearranged heavy chain V-region.

3. The spatially addressable library of claim 1, wherein the germline V-regions are heavy chain V-regions, wherein optionally each of the heavy chain V-regions is operably linked to an endogenously rearranged light chain V-region.

4. The spatially addressable library of claim 1, wherein the germline V-regions comprise operably linked heavy chain and light chain V-regions.

5. The spatially addressable library of claim 1, wherein the member polynucleotides encoding exogenously rearranged human germline antibody V-regions are obtainable by a method comprising the steps of:
(a) obtaining a V minigene or a J minigene; and
(b) joining the V minigene with at least one J minigene, or joining the J mini gene with a V minigene, wherein the J mini gene is located at the 3' end of the V minigene.

6. The spatially addressable library of claim 1, wherein
a) a D minigene is further joined to the 3' end of the V mini gene and the 5' end of the J minigene,
b) wherein the V minigene or the J mini gene in step (a) is obtained by chemical synthesis,
c) wherein the V minigene or the J minigene in step (a) is obtained by amplification from a germline DNA library, or
d) wherein step (b) is performed by primer extension using at least two oligonucleotide primers.

7. The spatially addressable library of claim 6 a), wherein step (b) is performed by primer extension using at least three oligonucleotide primers.

8. The spatially addressable library of claim 7 d), wherein one of the primers comprises homology to both the V minigene and the J minigene.

9. A method of producing a polynucleotide encoding a human germline antibody V-region, comprising the steps of:
(a) obtaining a V minigene or a J minigene; and
(b) joining the V minigene with at least one J minigene, or joining the J mini gene with a V minigene, wherein the J mini gene is located at the 3' end of the V minigene.

10. The method of claim 9, wherein
a) a D minigene is further joined to the 3' end of the V mini gene and the 5' end of the J minigene,
b) wherein the V minigene or the J mini gene in step (a) is obtained by chemical synthesis,
c) wherein the V minigene or the J minigene in step (a) is obtained by amplification from a germline DNA library, or
d) wherein step (b) is performed by primer extension using at least two oligonucleotide primers.

11. The method of claim 10 a), wherein step (b) is performed by primer extension using at least three oligonucleotide primers.

12. The method of claim 10 d), wherein one of the primers comprises homology to both the V minigene and the J minigene.

13. The method of claim 11, wherein
a) one of the primers comprises homology to both the V mini gene and the D minigene,
b) wherein one of the primers comprises homology to both the D mini gene and the J minigene, or
c) wherein at least one of the oligonucleotide primers comprises degeneracy at one nucleotide position.

14. The method of claim 9, wherein
a) the V minigene is derived from human immunoglobulin kappa locus,
b) wherein the V minigene is derived from human immunoglobulin lambda locus, or
c) wherein the V mini gene is derived from human immunoglobulin heavy chain locus.

15. The method of claim 9, wherein the V-region comprises a serine protease triad.


SEQUENCE LISTING

<110> Sharma, Vikram
      Leonard, Lindsay
      Smider, Vaughn
      Integrigen, Inc.

<120> Methods and Compositions for Generation
  of Germline Human Antibody Genes

<130> 021216-000700PC

<140> WO PCT/US04/29617
<141> 2004-09-09

<150> US 60/501,073
<151> 2003-09-09

<160> 285

<170> FastSEQ for Windows Version 4.0

<210> 1
<211> 98
<212> PRT
<213> Homo sapiens

<220>
<223> human germline heavy chain variable region VH1
      minigene 1-02

<400> 1
Gln Val Gln Leu Val Gln Ser Gly Ala Glu Val Lys Lys Pro Gly Ala
 1               5                  10                  15
Ser Val Lys Val Ser Cys Lys Ala Ser Gly Tyr Thr Phe Thr Gly Tyr
            20                  25                  30
Tyr Met His Trp Val Arg Gln Ala Pro Gly Gln Gly Leu Glu Trp Met
        35                  40                  45
Gly Trp Ile Asn Pro Asn Ser Gly Gly Thr Asn Tyr Ala Gln Lys Phe
    50                  55                  60
Gln Gly Arg Val Thr Met Thr Arg Asp Thr Ser Ile Ser Thr Ala Tyr
65                  70                  75                  80
Met Glu Leu Ser Arg Leu Arg Ser Asp Asp Thr Ala Val Tyr Tyr Cys
                85                  90                  95
Ala Arg


<210> 2
<211> 98
<212> PRT
<213> Homo sapiens

<220>
<223> human germline heavy chain variable region VH1
      minigene 1-03

<400> 2
Gln Val Gln Leu Val Gln Ser Gly Ala Glu Val Lys Lys Pro Gly Ala
 1               5                  10                  15
Ser Val Lys Val Ser Cys Lys Ala Ser Gly Tyr Thr Phe Thr Ser Tyr
            20                  25                  30
Ala Met His Trp Val Arg Gln Ala Pro Gly Gln Arg Leu Glu Trp Met
        35                  40                  45

Gly Trp Ile Asn Ala Gly Asn Gly Asn Thr Lys Tyr Ser Gln Lys Phe
    50                  55                  60
Gln Gly Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg

<210> 3
<211> 98
<212> PRT
<213> Homo sapiens

<220>
<223> human germline heavy chain variable region VH1
      minigene 1-08

<400> 3
Gln Val Gln Leu Val Gln Ser Gly Ala Glu Val Lys Lys Pro Gly Ala
 1               5                  10                  15
Ser Val Lys Val Ser Cys Lys Ala Ser Gly Tyr Thr Phe Thr Ser Tyr
            20                  25                  30
Asp Ile Asn Trp Val Arg Gln Ala Thr Gly Gln Gly Leu Glu Trp Met
        35                  40                  45
Gly Trp Met Asn Pro Asn Ser Gly Asn Thr Gly Tyr Ala Gln Lys Phe
    50                  55                  60
Gln Gly Arg Val Thr Met Thr Arg Asn Thr Ser Ile Ser Thr Ala Tyr
65                  70                  75                  80
Met Glu Leu Ser Ser Leu Arg Ser Glu Asp Thr Ala Val Tyr Tyr Cys
                85                  90                  95
Ala Arg

<210> 4
<211> 98
<212> PRT
<213> Homo sapiens

<220>
<223> human germline heavy chain variable region VH1
      minigene 1-18

<400> 4
Gln Val Gln Leu Val Gln Ser Gly Ala Glu Val Lys Lys Pro Gly Ala
 1               5                  10                  15
Ser Val Lys Val Ser Cys Lys Ala Ser Gly Tyr Thr Phe Thr Ser Tyr
            20                  25                  30
Gly Ile Ser Trp Val Arg Gln Ala Pro Gly Gln Gly Leu Glu Trp Met
        35                  40                  45
Gly Trp Ile Ser Ala Tyr Asn Gly Asn Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Arg Ser Leu Arg Ser Asp Asp Thr Ala Val Tyr Tyr Cys
                85                  90                  95
Ala Arg

<210> 5
<211> 98
<212> PRT
<213> Homo sapiens

<220>
<223> human germline heavy chain variable region VH1
      minigene 1-24

<400> 5
Gln Val Gln Leu Val Gln Ser Gly Ala Glu Val Lys Lys Pro Gly Ala
 1               5                  10                  15
Ser Val Lys Val Ser Cys Lys Val Ser Gly Tyr Thr Leu Thr Glu Leu
            20                  25                  30
Ser Met His Trp Val Arg Gln Ala Pro Gly Lys Gly Leu Glu Trp Met
        35                  40                  45
Gly Gly Phe Asp Pro Glu Asp Gly Glu Thr Ile Tyr Ala Gln Lys Phe
    50                  55                  60
Gln Gly Arg Val Thr Met Thr Glu Asp Thr Ser Thr Asp Thr Ala Tyr
65                  70                  75                  80
Met Glu Leu Ser Ser Leu Arg Ser Glu Asp Thr Ala Val Tyr Tyr Cys
                85                  90                  95
Ala Thr


<210> 6
<211> 98
<212> PRT
<213> Homo sapiens

<220>
<223> human germline heavy chain variable region VH1
      minigene 1-45

<400> 6
Gln Met Gln Leu Val Gln Ser Gly Ala Glu Val Lys Lys Thr Gly Ser
 1               5                  10                  15
Ser Val Lys Val Ser Cys Lys Ala Ser Gly Tyr Thr Phe Thr Tyr Arg
            20                  25                  30
Tyr Leu His Trp Val Arg Gln Ala Pro Gly Gln Ala Leu Glu Trp Met
        35                  40                  45
Gly Trp Ile Thr Pro Phe Asn Gly Asn Thr Asn Tyr Ala Gln Lys Phe
    50                  55                  60
Gln Asp Arg Val Thr Ile Thr Arg Asp Arg Ser Met Ser Thr Ala Tyr
65                  70                  75                  80
Met Glu Leu Ser Ser Leu Arg Ser Glu Asp Thr Ala Met Tyr Tyr Cys
                85                  90                  95
Ala Arg


<210> 7
<211> 98
<212> PRT
<213> Homo sapiens

<220>
<223> human germline heavy chain variable region VH1
      minigene 1-46

<400> 7
Gln Val Gln Leu Val Gln Ser Gly Ala Glu Val Lys Lys Pro Gly Ala
 1               5                  10                  15
Ser Val Lys Val Ser Cys Lys Ala Ser Gly Tyr Thr Phe Thr Ser Tyr
            20                  25                  30
Tyr Met His Trp Val Arg Gln Ala Pro Gly Gln Gly Leu Glu Trp Met
        35                  40                  45
Gly Ile Ile Asn Pro Ser Gly Gly Ser Thr Ser Tyr Ala Gln Lys Phe

50                  55                  60
Gln Gly Arg Val Thr Met Thr Arg Asp Thr Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Arg

<210> 8
<211> 98
<212> PRT
<213> Homo sapiens

<220>
<223> human germline heavy chain variable region VH1
      minigene 1-58

<400> 8
Gln Met Gln Leu Val Gln Ser Gly Pro Glu Val Lys Lys Pro Gly Thr
 1               5                  10                  15
Ser Val Lys Val Ser Cys Lys Ala Ser Gly Phe Thr Phe Thr Ser Ser
            20                  25                  30
Ala Val Gln Trp Val Arg Gln Ala Arg Gly Gln Arg Leu Glu Trp Ile
        35                  40                  45
Gly Trp Ile Val Val Gly Ser Gly Asn Thr Asn Tyr Ala Gln Lys Phe
    50                  55                  60
Gln Glu Arg Val Thr Ile Thr Arg Asp Met Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Ala

<210> 9
<211> 98
<212> PRT
<213> Homo sapiens

<220>
<223> human germline heavy chain variable region VH1
      minigene 1-69

<400> 9
Gln Val Gln Leu Val Gln Ser Gly Ala Glu Val Lys Lys Pro Gly Ser
 1               5                  10                  15
Ser Val Lys Val Ser Cys Lys Ala Ser Gly Gly Thr Phe Ser Ser Tyr
            20                  25                  30
Ala Ile Ser Trp Val Arg Gln Ala Pro Gly Gln Gly Leu Glu Trp Met
        35                  40                  45
Gly Gly Ile Ile Pro Ile Phe Gly Thr Ala Asn Tyr Ala Gln Lys Phe
    50                  55                  60
Gln Gly Arg Val Thr Ile Thr Ala Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg

<210> 10
<211> 98
<212> PRT
<213> Homo sapiens

<220>
<223> human germline heavy chain variable region VH1
      minigene 1-e

<400> 10
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Ser Tyr
            20                  25                  30
Ala Ile Ser Trp Val Arg Gln Ala Pro Gly Gln Gly Leu Glu Trp Met
        35                  40                  45
Gly Gly Ile Ile Pro Ile Phe Gly Thr Ala Asn Tyr Ala Gln Lys Phe
    50                  55                  60
Gln Gly Arg Val Thr Ile Thr Ala Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg


<210> 11
<211> 98
<212> PRT
<213> Homo sapiens

<220>
<223> human germline heavy chain variable region VH1
      minigene 1-f

<400> 11
Glu Val Gln Leu Val Gln Ser Gly Ala Glu Val Lys Lys Pro Gly Ala
1               5                   10                  15
Thr Val Lys Ile Ser Cys Lys Val Ser Gly Tyr Thr Phe Thr Asp Tyr
            20                  25                  30
Tyr Met His Trp Val Gln Gln Ala Pro Gly Lys Gly Leu Glu Trp Met
        35                  40                  45
Gly Leu Val Asp Pro Glu Asp Gly Glu Thr Ile Tyr Ala Glu Lys Phe
    50                  55                  60
Gln Gly Arg Val Thr Ile Thr Ala Asp Thr Ser Thr Asp Thr Ala Tyr
65                  70                  75                  80
Met Glu Leu Ser Ser Leu Arg Ser Glu Asp Thr Ala Val Tyr Tyr Cys
                85                  90                  95
Ala Thr


<210> 12
<211> 100
<212> PRT
<213> Homo sapiens

<220>
<223> human germline heavy chain variable region VH2
      minigene 2-05

<400> 12
Gln Ile Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Val Gly Val Gly Trp Ile Arg Gln Pro Pro Gly Lys Ala Leu Glu
        35                  40                  45
Trp Leu Ala Leu Ile Tyr Trp Asn Asp Asp Lys Arg Tyr Ser Pro Ser
    50                  55                  60

Leu Lys Ser Arg Leu Thr Ile Thr Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala His Arg
            100

<210> 13
<211> 100
<212> PRT
<213> Homo sapiens

<220>
<223> human germline heavy chain variable region VH2
      minigene 2-26

<400> 13
Gln Val Thr Leu Lys Glu Ser Gly Pro Val Leu Val Lys Pro Thr Glu
 1               5                  10                  15
Thr Leu Thr Leu Thr Cys Thr Val Ser Gly Phe Ser Leu Ser Asn Ala
            20                  25                  30
Arg Met Gly Val Ser Trp Ile Arg Gln Pro Pro Gly Lys Ala Leu Glu
        35                  40                  45
Trp Leu Ala His Ile Phe Ser Asn Asp Glu Lys Ser Tyr Ser Thr Ser
    50                  55                  60
Leu Lys Ser Arg Leu Thr Ile Ser Lys Asp Thr Ser Lys Ser Gln Val
65                  70                  75                  80
Val Leu Thr Met Thr Asn Met Asp Pro Val Asp Thr Ala Thr Tyr Tyr
                85                  90                  95
Cys Ala Arg Ile
            100

<210> 14
<211> 100
<212> PRT
<213> Homo sapiens

<220>
<223> human germline heavy chain variable region VH2
      minigene 2-70

<400> 14
Gln Val Thr Leu Lys Glu Ser Gly Pro Ala Leu Val Lys Pro Thr Gln
 1               5                  10                  15
Thr Leu Thr Leu Thr Cys Thr Phe Ser Gly Phe Ser Leu Ser Thr Ser
            20                  25                  30
Gly Met Arg Val Ser Trp Ile Arg Gln Pro Pro Gly Lys Ala Leu Glu
        35                  40                  45
Trp Leu Ala Arg Ile Asp Trp Asp Asp Asp Lys Phe Tyr Ser Thr Ser
    50                  55                  60
Leu Lys Thr Arg Leu Thr Ile Ser Lys Asp Thr Ser Lys Asn Gln Val
65                  70                  75                  80
Val Leu Thr Met Thr Asn Met Asp Pro Val Asp Thr Ala Thr Tyr Tyr
                85                  90                  95
Cys Ala Arg Ile
            100

<210> 15
<211> 98
<212> PRT

<213> Homo sapiens

<220>
<223> human germline heavy chain variable region VH3
      minigene 3-07

<400> 15
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Trp Met Ser Trp Val Arg Gln Ala Pro Gly Lys Gly Leu Glu Trp Val
        35                  40                  45
Ala Asn Ile Lys Gln Asp Gly Ser Glu Lys Tyr Tyr Val Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg


<210> 16
<211> 99
<212> PRT
<213> Homo sapiens

<220>
<223> human germline heavy chain variable region VH3
      minigene 3-09

<400> 16
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Gly Ile Ser Trp Asn Ser Gly Ser Ile Gly Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Leu Tyr Tyr Cys
                85                  90                  95
Ala Lys Asp


<210> 17
<211> 98
<212> PRT
<213> Homo sapiens

<220>
<223> human germline heavy chain variable region VH3
      minigene 3-11

<400> 17
Gln Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Asp Tyr
            20                  25                  30
Tyr Met Ser Trp Ile Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg

<210> 18
<211> 97
<212> PRT
<213> Homo sapiens

<220>
<223> human germline heavy chain variable region VH3
      minigene 3-13

<400> 18
Glu Val Gln Leu Val Glu Ser Gly Gly Gly Leu Val Gln Pro Gly Gly
 1               5                  10                  15
Ser Leu Arg Leu Ser Cys Ala Ala Ser Gly Phe Thr Phe Ser Ser Tyr
            20                  25                  30
Asp Met His Trp Val Arg Gln Ala Thr Gly Lys Gly Leu Glu Trp Val
        35                  40                  45
Ser Ala Ile Gly Thr Ala Gly Asp Thr Tyr Tyr Pro Gly Ser Val Lys
    50                  55                  60
Gly Arg Phe Thr Ile Ser Arg Glu Asn Ala Lys Asn Ser Leu Tyr Leu
65                  70                  75                  80
Gln Met Asn Ser Leu Arg Ala Gly Asp Thr Ala Val Tyr Tyr Cys Ala
                85                  90                  95
Arg

<210> 19
<211> 100
<212> PRT
<213> Homo sapiens

<220>
<223> human germline heavy chain variable region VH3
      minigene 3-15

<400> 19
Glu Val Gln Leu Val Glu Ser Gly Gly Gly Leu Val Lys Pro Gly Gly
 1               5                  10                  15
Ser Leu Arg Leu Ser Cys Ala Ala Ser Gly Phe Thr Phe Ser Asn Ala
            20                  25                  30
Trp Met Ser Trp Val Arg Gln Ala Pro Gly Lys Gly Leu Glu Trp Val
        35                  40                  45
Gly Arg Ile Lys Ser Lys Thr Asp Gly Gly Thr Thr Asp Tyr Ala Ala
    50                  55                  60
Pro Val Lys Gly Arg Phe Thr Ile Ser Arg Asp Asp Ser Lys Asn Thr
65                  70                  75                  80
Leu Tyr Leu Gln Met Asn Ser Leu Lys Thr Glu Asp Thr Ala Val Tyr
                85                  90                  95
Tyr Cys Thr Thr
            100

<210> 20
<211> 98
<212> PRT

<213> Homo sapiens

<220>
<223> human germline heavy chain variable region VH3
      minigene 3-20

<400> 20
Glu Val Gln Leu Val Glu Ser Gly Gly Gly Val Val Arg Pro Gly Gly
1               5                   10                  15
Ser Leu Arg Leu Ser Cys Ala Ala Ser Gly Phe Thr Phe Asp Asp Tyr
            20                  25                  30
Gly Met Ser Trp Val Arg Gln Ala Pro Gly Lys Gly Leu Glu Trp Val
        35                  40                  45
Ser Gly Ile Asn Trp Asn Gly Gly Ser Thr Gly Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Leu Tyr His Cys
                85                  90                  95
Ala Arg


<210> 21
<211> 98
<212> PRT
<213> Homo sapiens

<220>
<223> human germline heavy chain variable region VH3
      minigene 3-21

<400> 21
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Thr Phe Ser Ser Tyr
            20                  25                  30
Ser Met Asn Trp Val Arg Gln Ala Pro Gly Lys Gly Leu Glu Trp Val
        35                  40                  45
Ser Ser Ile Ser Ser Ser Ser Ser Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg


<210> 22
<211> 98
<212> PRT
<213> Homo sapiens

<220>
<223> human germline heavy chain variable region VH3
      minigene 3-23

<400> 22
Glu Val Gln Leu Leu Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys



<210> 23
<211> 98
<212> PRT
<213> Homo sapiens

<220>
<223> human germline heavy chain variable region VH3
      minigene 3-30

<400> 23
Gln Val Gln Leu Val Glu Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Ser Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys



<210> 24
<211> 98
<212> PRT
<213> Homo sapiens

<220>
<223> human germline heavy chain variable region VH3
      minigene 3-30.3

<400> 24
Gln Val Gln Leu Val Glu Ser Gly Gly Gly Val Val Gln Pro Gly Arg
 1               5                  10                  15
Ser Leu Arg Leu Ser Cys Ala Ala Ser Gly Phe Thr Phe Ser Ser Tyr
            20                  25                  30
Ala Met His Trp Val Arg Gln Ala Pro Gly Lys Gly Leu Glu Trp Val
        35                  40                  45
Ala Val Ile Ser Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg



<210> 25
<211> 98
<212> PRT
<213> Homo sapiens

<220>
<223> human germline heavy chain variable region VH3
      minigene 3-30.5

<400> 25
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Ser Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Lys


<210> 26
<211> 98
<212> PRT
<213> Homo sapiens

<220>
<223> human germline heavy chain variable region VH3
      minigene 3-33

<400> 26
Gln Val Gln Leu Val Glu Ser Gly Gly Gly Val Val Gln Pro Gly Arg
1               5                   10                  15
Ser Leu Arg Leu Ser Cys Ala Ala Ser Gly Phe Thr Phe Ser Ser Tyr
            20                  25                  30
Gly Met His Trp Val Arg Gln Ala Pro Gly Lys Gly Leu Glu Trp Val
        35                  40                  45
Ala Val Ile Trp Tyr Asp Gly Ser Asn Lys Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg


<210> 27
<211> 99
<212> PRT
<213> Homo sapiens

<220>
<223> human germline heavy chain variable region VH3
      minigene 3-43

<400> 27
Glu Val Gln Leu Val Glu Ser Gly Gly Val Val Val Gln Pro Gly Gly
1               5                   10                  15
Ser Leu Arg Leu Ser Cys Ala Ala Ser Gly Phe Thr Phe Asp Asp Tyr
            20                  25                  30
Thr Met His Trp Val Arg Gln Ala Pro Gly Lys Gly Leu Glu Trp Val
        35                  40                  45
Ser Leu Ile Ser Trp Asp Gly Gly Ser Thr Tyr Tyr Ala Asp Ser Val

50                  55                  60
Lys Gly Arg Phe Thr Ile Ser Arg Asp Asn Ser Lys Asn Ser Leu Tyr
65                  70                  75                  80
Leu Gln Met Asn Ser Leu Arg Thr Glu Asp Thr Ala Leu Tyr Tyr Cys
                85                  90                  95
Ala Lys Asp

<210> 28
<211> 98
<212> PRT
<213> Homo sapiens

<220>
<223> human germline heavy chain variable region VH3
      minigene 3-48

<400> 28
Glu Val Gln Leu Val Glu Ser Gly Gly Gly Leu Val Gln Pro Gly Gly
 1               5                  10                  15
Ser Leu Arg Leu Ser Cys Ala Ala Ser Gly Phe Thr Phe Ser Ser Tyr
            20                  25                  30
Ser Met Asn Trp Val Arg Gln Ala Pro Gly Lys Gly Leu Glu Trp Val
        35                  40                  45
Ser Tyr Ile Ser Ser Ser Ser Ser Thr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Asp Glu Asp Thr Ala Val Tyr Tyr Cys
                85                  90                  95
Ala Arg

<210> 29
<211> 100
<212> PRT
<213> Homo sapiens

<220>
<223> human germline heavy chain variable region VH3
      minigene 3-49

<400> 29
Glu Val Gln Leu Val Glu Ser Gly Gly Gly Leu Val Gln Pro Gly Arg
 1               5                  10                  15
Ser Leu Arg Leu Ser Cys Thr Ala Ser Gly Phe Thr Phe Gly Asp Tyr
            20                  25                  30
Ala Met Ser Trp Phe Arg Gln Ala Pro Gly Lys Gly Leu Glu Trp Val
        35                  40                  45
Gly Phe Ile Arg Ser Lys Ala Tyr Gly Gly Thr Thr Glu Tyr Thr Ala
    50                  55                  60
Ser Val Lys Gly Arg Phe Thr Ile Ser Arg Asp Gly Ser Lys Ser Ile
65                  70                  75                  80
Ala Tyr Leu Gln Met Asn Ser Leu Lys Thr Glu Asp Thr Ala Val Tyr
                85                  90                  95
Tyr Cys Thr Arg
            100

<210> 30
<211> 97
<212> PRT
<213> Homo sapiens

<220>
<223> human germline heavy chain variable region VH3
      minigene 3-53

<400> 30
Glu Val Gln Leu Val Glu Thr Gly Gly Gly Leu Ile Gln Pro Gly Gly
1               5                   10                  15
Ser Leu Arg Leu Ser Cys Ala Ala Ser Gly Phe Thr Val Ser Ser Asn
            20                  25                  30
Tyr Met Ser Trp Val Arg Gln Ala Pro Gly Lys Gly Leu Glu Trp Val
        35                  40                  45
Ser Val Ile Tyr Ser Gly Gly Ser Thr Tyr Tyr Ala Asp Ser Val Lys
    50                  55                  60
Gly Arg Phe Thr Ile Ser Arg Asp Asn Ser Lys Asn Thr Leu Tyr Leu
65                  70                  75                  80
Gln Met Asn Ser Leu Arg Ala Glu Asp Thr Ala Val Tyr Tyr Cys Ala
                85                  90                  95
Arg


<210> 31
<211> 98
<212> PRT
<213> Homo sapiens

<220>
<223> human germline heavy chain variable region VH3
      minigene 3-64

<400> 31
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Ala Met His Trp Val Arg Gln Ala Pro Gly Lys Gly Leu Glu Tyr Val
        35                  40                  45
Ser Ala Ile Ser Ser Asn Gly Gly Ser Thr Tyr Tyr Ala Asn Ser Val
    50                  55                  60
Lys Gly Arg Phe Thr Ile Ser Arg Asp Asn Ser Lys Asn Thr Leu Tyr
65                  70                  75                  80
Leu Gln Met Gly Ser Leu Arg Ala Glu Asp Met Ala Val Tyr Tyr Cys
                85                  90                  95
Ala Arg


<210> 32
<211> 97
<212> PRT
<213> Homo sapiens

<220>
<223> human germline heavy chain variable region VH3
      minigene 3-66

<400> 32
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Val Ser Ser Asn
            20                  25                  30
Tyr Met Ser Trp Val Arg Gln Ala Pro Gly Lys Gly Leu Glu Trp Val
        35                  40                  45
Ser Val Ile Tyr Ser Gly Gly Ser Thr Tyr Tyr Ala Asp Ser Val Lys

50                  55                  60
Gly Arg Phe Thr Ile Ser Arg Asp Asn Ser Lys Asn Thr Leu Tyr Leu
65                  70                  75                  80
Gln Met Asn Ser Leu Arg Ala Glu Asp Thr Ala Val Tyr Tyr Cys Ala
                85                  90                  95
Arg

<210> 33
<211> 100
<212> PRT
<213> Homo sapiens

<220>
<223> human germline heavy chain variable region VH3
      minigene 3-72

<400> 33
Glu Val Gln Leu Val Glu Ser Gly Gly Gly Leu Val Gln Pro Gly Gly
 1               5                  10                  15
Ser Leu Arg Leu Ser Cys Ala Ala Ser Gly Phe Thr Phe Ser Asp His
            20                  25                  30
Tyr Met Asp Trp Val Arg Gln Ala Pro Gly Lys Gly Leu Glu Trp Val
        35                  40                  45
Gly Arg Thr Arg Asn Lys Ala Asn Ser Tyr Thr Thr Glu Tyr Ala Ala
    50                  55                  60
Ser Val Lys Gly Arg Phe Thr Ile Ser Arg Asp Asp Ser Lys Asn Ser
65                  70                  75                  80
Leu Tyr Leu Gln Met Asn Ser Leu Lys Thr Glu Asp Thr Ala Val Tyr
                85                  90                  95
Tyr Cys Ala Arg
            100

<210> 34
<211> 100
<212> PRT
<213> Homo sapiens

<220>
<223> human germline heavy chain variable region VH3
      minigene 3-73

<400> 34
Glu Val Gln Leu Val Glu Ser Gly Gly Gly Leu Val Gln Pro Gly Gly
 1               5                  10                  15
Ser Leu Lys Leu Ser Cys Ala Ala Ser Gly Phe Thr Phe Ser Gly Ser
            20                  25                  30
Ala Met His Trp Val Arg Gln Ala Ser Gly Lys Gly Leu Glu Trp Val
        35                  40                  45
Gly Arg Ile Arg Ser Lys Ala Asn Ser Tyr Ala Thr Ala Tyr Ala Ala
    50                  55                  60
Ser Val Lys Gly Arg Phe Thr Ile Ser Arg Asp Asp Ser Lys Asn Thr
65                  70                  75                  80
Ala Tyr Leu Gln Met Asn Ser Leu Lys Thr Glu Asp Thr Ala Val Tyr
                85                  90                  95
Tyr Cys Thr Arg
            100

<210> 35
<211> 98
<212> PRT

<213> Homo sapiens

<220>
<223> human germline heavy chain variable region VH3
      minigene 3-74

<400> 35
Glu Val Gln Leu Val Glu Ser Gly Gly Gly Leu Val Gln Pro Gly Gly
 1               5                   10                  15
Ser Leu Arg Leu Ser Cys Ala Ala Ser Gly Phe Thr Phe Ser Ser Tyr
            20                  25                  30
Trp Met His Trp Val Arg Gln Ala Pro Gly Lys Gly Leu Val Trp Val
        35                  40                  45
Ser Arg Ile Asn Ser Asp Gly Ser Ser Thr Ser Tyr Ala Asp Ser Val
    50                  55                  60
Lys Gly Arg Phe Thr Ile Ser Arg Asp Asn Ala Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg


<210> 36
<211> 96
<212> PRT
<213> Homo sapiens

<220>
<223> human germline heavy chain variable region VH3
      minigene 3-d

<400> 36
Glu Val Gln Leu Val Glu Ser Arg Gly Val Leu Val Gln Pro Gly Gly
 1               5                   10                  15
Ser Leu Arg Leu Ser Cys Ala Ala Ser Gly Phe Thr Val Ser Ser Asn
            20                  25                  30
Glu Met Ser Trp Val Arg Gln Ala Pro Gly Lys Gly Leu Glu Trp Val
        35                  40                  45
Ser Ser Ile Ser Gly Gly Ser Thr Tyr Tyr Ala Asp Ser Arg Lys Gly
    50                  55                  60
Arg Phe Thr Ile Ser Arg Asp Asn Ser Lys Asn Thr Leu His Leu Gln
65                  70                  75                  80
Met Asn Ser Leu Arg Ala Glu Asp Thr Ala Val Tyr Tyr Cys Lys Lys
                85                  90                  95


<210> 37
<211> 98
<212> PRT
<213> Homo sapiens

<220>
<223> human germline heavy chain variable region VH4
      minigene 4-04

<400> 37
Gln Val Gln Leu Gln Glu Ser Gly Pro Gly Leu Val Lys Pro Ser Gly
 1               5                   10                  15
Thr Leu Ser Leu Thr Cys Ala Val Ser Gly Gly Ser Ile Ser Ser Ser
            20                  25                  30
Asn Trp Trp Ser Trp Val Arg Gln Pro Pro Gly Lys Gly Leu Glu Trp
        35                  40                  45
Ile Gly Glu Ile Tyr His Ser Gly Ser Thr Asn Tyr Asn Pro Ser Leu

50                  55                  60
Lys Ser Arg Val Thr Ile Ser Val Asp Lys Ser Lys Asn Gln Phe Ser
65                  70                  75                  80
Leu Lys Leu Ser Ser Val Thr Ala Ala Asp Thr Ala Val Tyr Tyr Cys
                85                  90                  95
Ala Arg

<210> 38
<211> 98
<212> PRT
<213> Homo sapiens

<220>
<223> human germline heavy chain variable region VH4
      minigene 4-28

<400> 38
Gln Val Gln Leu Gln Glu Ser Gly Pro Gly Leu Val Lys Pro Ser Asp
 1               5                  10                  15
Thr Leu Ser Leu Thr Cys Ala Val Ser Gly Tyr Ser Ile Ser Ser Ser
            20                  25                  30
Asn Trp Trp Gly Trp Ile Arg Gln Pro Pro Gly Lys Gly Leu Glu Trp
        35                  40                  45
Ile Gly Tyr Ile Tyr Tyr Ser Gly Ser Thr Tyr Tyr Asn Pro Ser Leu
    50                  55                  60
Lys Ser Arg Val Thr Met Ser Val Asp Thr Ser Lys Asn Gln Phe Ser
65                  70                  75                  80
Leu Lys Leu Ser Ser Val Thr Ala Val Asp Thr Ala Val Tyr Tyr Cys
                85                  90                  95
Ala Arg

<210> 39
<211> 99
<212> PRT
<213> Homo sapiens

<220>
<223> human germline heavy chain variable region VH4
      minigene 4-30.1

<400> 39
Gln Val Gln Leu Gln Glu Ser Gly Pro Gly Leu Val Lys Pro Ser Gln
 1               5                  10                  15
Thr Leu Ser Leu Thr Cys Thr Val Ser Gly Gly Ser Ile Ser Ser Gly
            20                  25                  30
Gly Tyr Tyr Trp Ser Trp Ile Arg Gln His Pro Gly Lys Gly Leu Glu
        35                  40                  45
Trp Ile Gly Tyr Ile Tyr Tyr Ser Gly Ser Thr Tyr Tyr Asn Pro Ser
    50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg

<210> 40
<211> 99
<212> PRT
<213> Homo sapiens

<220>
<223> human germline heavy chain variable region VH4
      minigene 4-30.2

<400> 40
Gln Leu Gln Leu Gln Glu Ser Gly Ser Gly Leu Val Lys Pro Ser Gln
1               5                   10                  15
Thr Leu Ser Leu Thr Cys Ala Val Ser Gly Gly Ser Ile Ser Ser Gly
            20                  25                  30
Gly Tyr Ser Trp Ser Trp Ile Arg Gln Pro Pro Gly Lys Gly Leu Glu
        35                  40                  45
Trp Ile Gly Tyr Ile Tyr His Ser Gly Ser Thr Tyr Tyr Asn Pro Ser
    50                  55                  60
Leu Lys Ser Arg Val Thr Ile Ser Val Asp Arg Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg


<210> 41
<211> 99
<212> PRT
<213> Homo sapiens

<220>
<223> human germline heavy chain variable region VH4
      minigene 4-30.4

<400> 41
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Ser Gly
            20                  25                  30
Asp Tyr Tyr Trp Ser Trp Ile Arg Gln Pro Pro Gly Lys Gly Leu Glu
        35                  40                  45
Trp Ile Gly Tyr Ile Tyr Tyr Ser Gly Ser Thr Tyr Tyr Asn Pro Ser
    50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg


<210> 42
<211> 99
<212> PRT
<213> Homo sapiens

<220>
<223> human germline heavy chain variable region VH4
      minigene 4-31

<400> 42
Gln Val Gln Leu Gln Glu Ser Gly Pro Gly Leu Val Lys Pro Ser Gln
1               5                   10                  15
Thr Leu Ser Leu Thr Cys Thr Val Ser Gly Gly Ser Ile Ser Ser Gly
            20                  25                  30
Gly Tyr Tyr Trp Ser Trp Ile Arg Gln His Pro Gly Lys Gly Leu Glu
        35                  40                  45
Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg


<210> 43
<211> 97
<212> PRT
<213> Homo sapiens

<220>
<223> human germline heavy chain variable region VH4
      minigene 4-34

<400> 43
Gln Val Gln Leu Gln Gln Trp Gly Ala Gly Leu Leu Lys Pro Ser Glu
 1               5                  10                  15
Thr Leu Ser Leu Thr Cys Ala Val Tyr Gly Gly Ser Phe Ser Gly Tyr
            20                  25                  30
Tyr Trp Ser Trp Ile Arg Gln Pro Pro Gly Lys Gly Leu Glu Trp Ile
        35                  40                  45
Gly Glu Ile Asn His Ser Gly Ser Thr Asn Tyr Asn Pro Ser Leu Lys
    50                  55                  60
Ser Arg Val Thr Ile Ser Val Asp Thr Ser Lys Asn Gln Phe Ser Leu
65                  70                  75                  80
Lys Leu Ser Ser Val Thr Ala Ala Asp Thr Ala Val Tyr Tyr Cys Ala
                85                  90                  95
Arg


<210> 44
<211> 99
<212> PRT
<213> Homo sapiens

<220>
<223> human germline heavy chain variable region VH4
      minigene 4-39

<400> 44
Gln Leu Gln Leu Gln Glu Ser Gly Pro Gly Leu Val Lys Pro Ser Glu
 1               5                  10                  15
Thr Leu Ser Leu Thr Cys Thr Val Ser Gly Gly Ser Ile Ser Ser Ser
            20                  25                  30
Ser Tyr Tyr Trp Gly Trp Ile Arg Gln Pro Pro Gly Lys Gly Leu Glu
        35                  40                  45
Trp Ile Gly Ser Ile Tyr Tyr Ser Gly Ser Thr Tyr Tyr Asn Pro Ser
    50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg


<210> 45
<211> 97
<212> PRT
<213> Homo sapiens

<220>

<223> human germline heavy chain variable region VH4
      minigene 4-59

<400> 45
Gln Val Gln Leu Gln Glu Ser Gly Pro Gly Leu Val Lys Pro Ser Glu
1               5                   10                  15
Thr Leu Ser Leu Thr Cys Thr Val Ser Gly Gly Ser Ile Ser Ser Tyr
            20                  25                  30
Tyr Trp Ser Trp Ile Arg Gln Pro Pro Gly Lys Gly Leu Glu Trp Ile
        35                  40                  45
Gly Tyr Ile Tyr Tyr Ser Gly Ser Thr Asn Tyr Asn Pro Ser Leu Lys
    50                  55                  60
Ser Arg Val Thr Ile Ser Val Asp Thr Ser Lys Asn Gln Phe Ser Leu
65                  70                  75                  80
Lys Leu Ser Ser Val Thr Ala Ala Asp Thr Ala Val Tyr Tyr Cys Ala
                85                  90                  95
Arg


<210> 46
<211> 99
<212> PRT
<213> Homo sapiens

<220>
<223> human germline heavy chain variable region VH4
      minigene 4-61

<400> 46
Gln Val Gln Leu Gln Glu Ser Gly Pro Gly Leu Val Lys Pro Ser Glu
1               5                   10                  15
Thr Leu Ser Leu Thr Cys Thr Val Ser Gly Gly Ser Val Ser Ser Gly
            20                  25                  30
Ser Tyr Tyr Trp Ser Trp Ile Arg Gln Pro Pro Gly Lys Gly Leu Glu
        35                  40                  45
Trp Ile Gly Tyr Ile Tyr Tyr Ser Gly Ser Thr Asn Tyr Asn Pro Ser
    50                  55                  60
Leu Lys Ser Arg Val Thr Ile Ser Val Asp Thr Ser Lys Asn Gln Phe
65                  70                  75                  80
Ser Leu Lys Leu Ser Ser Val Thr Ala Ala Asp Thr Ala Val Tyr Tyr
                85                  90                  95
Cys Ala Arg


<210> 47
<211> 98
<212> PRT
<213> Homo sapiens

<220>
<223> human germline heavy chain variable region VH4
      minigene 4-b

<400> 47
Gln Val Gln Leu Gln Glu Ser Gly Pro Gly Leu Val Lys Pro Ser Glu
1               5                   10                  15
Thr Leu Ser Leu Thr Cys Ala Val Ser Gly Tyr Ser Ile Ser Ser Gly
            20                  25                  30
Tyr Tyr Trp Gly Trp Ile Arg Gln Pro Pro Gly Lys Gly Leu Glu Trp
        35                  40                  45
Ile Gly Ser Ile Tyr His Ser Gly Ser Thr Tyr Tyr Asn Pro Ser Leu
    50                  55                  60
Lys Ser Arg Val Thr Ile Ser Val Asp Thr Ser Lys Asn Gln Phe Ser

65                  70                  75                  80
Leu Lys Leu Ser Ser Val Thr Ala Ala Asp Thr Ala Val Tyr Tyr Cys
                85                  90                  95
Ala Arg



<210> 48
<211> 98
<212> PRT
<213> Homo sapiens

<220>
<223> human germline heavy chain variable region VH5
      minigene 5-51

<400> 48
Glu Val Gln Leu Val Gln Ser Gly Ala Glu Val Lys Lys Pro Gly Glu
 1               5                  10                  15
Ser Leu Lys Ile Ser Cys Lys Gly Ser Gly Tyr Ser Phe Thr Ser Tyr
            20                  25                  30
Trp Ile Gly Trp Val Arg Gln Met Pro Gly Lys Gly Leu Glu Trp Met
        35                  40                  45
Gly Ile Ile Tyr Pro Gly Asp Ser Asp Thr Arg Tyr Ser Pro Ser Phe
    50                  55                  60
Gln Gly Gln Val Thr Ile Ser Ala Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Arg



<210> 49
<211> 98
<212> PRT
<213> Homo sapiens

<220>
<223> human germline heavy chain variable region VH5
      minigene 5-a

<400> 49
Glu Val Gln Leu Val Gln Ser Gly Ala Glu Val Lys Lys Pro Gly Glu
 1               5                  10                  15
Ser Leu Arg Ile Ser Cys Lys Gly Ser Gly Tyr Ser Phe Thr Ser Tyr
            20                  25                  30
Trp Ile Ser Trp Val Arg Gln Met Pro Gly Lys Gly Leu Glu Trp Met
        35                  40                  45
Gly Arg Ile Asp Pro Ser Asp Ser Tyr Thr Asn Tyr Ser Pro Ser Phe
    50                  55                  60
Gln Gly His Val Thr Ile Ser Ala Asp Lys Ser Ile Ser Thr Ala Tyr
65                  70                  75                  80
Leu Gln Trp Ser Ser Leu Lys Ala Ser Asp Thr Ala Met Tyr Tyr Cys
                85                  90                  95
Ala Arg



<210> 50
<211> 101
<212> PRT
<213> Homo sapiens

<220>
<223> human germline heavy chain variable region VH6

minigene 6-01

<400> 50
Gln Val Gln Leu Gln Gln Ser Gly Pro Gly Leu Val Lys Pro Ser Gln
1               5                   10                  15
Thr Leu Ser Leu Thr Cys Ala Ile Ser Gly Asp Ser Val Ser Ser Asn
            20                  25                  30
Ser Ala Ala Trp Asn Trp Ile Arg Gln Ser Pro Ser Arg Gly Leu Glu
        35                  40                  45
Trp Leu Gly Arg Thr Tyr Tyr Arg Ser Lys Trp Tyr Asn Asp Tyr Ala
    50                  55                  60
Val Ser Val Lys Ser Arg Ile Thr Ile Asn Pro Asp Thr Ser Lys Asn
65                  70                  75                  80
Gln Phe Ser Leu Gln Leu Asn Ser Val Thr Pro Glu Asp Thr Ala Val
                85                  90                  95
Tyr Tyr Cys Ala Arg
            100

<210> 51
<211> 98
<212> PRT
<213> Homo sapiens

<220>
<223> human germline heavy chain variable region VH7
      minigene 7-4.1

<400> 51
Gln Val Gln Leu Val Gln Ser Gly Ser Glu Leu Lys Lys Pro Gly Ala
1               5                   10                  15
Ser Val Lys Val Ser Cys Lys Ala Ser Gly Tyr Thr Phe Thr Ser Tyr
            20                  25                  30
Ala Met Asn Trp Val Arg Gln Ala Pro Gly Gln Gly Leu Glu Trp Met
        35                  40                  45
Gly Trp Ile Asn Thr Asn Thr Gly Asn Pro Thr Tyr Ala Gln Gly Phe
    50                  55                  60
Thr Gly Arg Phe Val Phe Ser Leu Asp Thr Ser Val Ser Thr Ala Tyr
65                  70                  75                  80
Leu Gln Ile Cys Ser Leu Lys Ala Glu Asp Thr Ala Val Tyr Tyr Cys
                85                  90                  95
Ala Arg

<210> 52
<211> 5
<212> PRT
<213> Homo sapiens

<220>
<223> human germline heavy chain D region D1 1-1 RF1

<400> 52
Gly Thr Thr Gly Thr
1               5

<210> 53
<211> 5
<212> PRT
<213> Homo sapiens

<220>

<223> human germline heavy chain D region D1 1-1 RF2

<400> 53
Val Gln Leu Glu Arg
1               5



<210> 54
<211> 5
<212> PRT
<213> Homo sapiens

<220>
<223> human germline heavy chain D region D1 1-1 RF3 and
      1-20 RF3

<400> 54
Tyr Asn Trp Asn Asp
1               5



<210> 55
<211> 5
<212> PRT
<213> Homo sapiens

<220>
<223> human germline heavy chain D region D1 1-7 RF1 and
      1-20 RF1

<400> 55
Gly Ile Thr Gly Thr
1               5



<210> 56
<211> 5
<212> PRT
<213> Homo sapiens

<220>
<223> human germline heavy chain D region D1 1-7 RF3

<400> 56
Tyr Asn Trp Asn Tyr
1               5



<210> 57
<211> 6
<212> PRT
<213> Homo sapiens

<220>
<223> human germline heavy chain D region D1 1-26 RF1

<400> 57
Gly Ile Val Gly Ala Thr
1               5



<210> 58
<211> 4

<212> PRT
<213> Homo sapiens

<220>
<223> portion of human germline heavy chain D region D1
      1-26 RF2

<400> 58
Trp Glu Leu Leu
1


<210> 59
<211> 6
<212> PRT
<213> Homo sapiens

<220>
<223> human germline heavy chain D region D1 1-26 RF3

<400> 59
Tyr Ser Gly Ser Tyr Tyr
 1               5


<210> 60
<211> 5
<212> PRT
<213> Homo sapiens

<220>
<223> portion of human germline heavy chain D region D2
      2-2 RF1

<400> 60
Tyr Gln Leu Leu Tyr
 1               5


<210> 61
<211> 10
<212> PRT
<213> Homo sapiens

<220>
<223> human germline heavy chain D region D2 2-2 RF2

<400> 61
Gly Tyr Cys Ser Ser Thr Ser Cys Tyr Thr
 1               5                   10


<210> 62
<211> 9
<212> PRT
<213> Homo sapiens

<220>
<223> human germline heavy chain D region D2 2-2 RF3

<400> 62
Asp Ile Val Val Val Pro Ala Ala Ile
 1               5

<210> 63
<211> 4
<212> PRT
<213> Homo sapiens

<220>
<223> portion of human germline heavy chain D region D2
      2-8 RF1

<400> 63
Arg Ile Leu Tyr
1


<210> 64
<211> 5
<212> PRT
<213> Homo sapiens

<220>
<223> portion of human germline heavy chain D region D2
      2-8 RF1

<400> 64
Trp Cys Met Leu Tyr
 1               5


<210> 65
<211> 10
<212> PRT
<213> Homo sapiens

<220>
<223> human germline heavy chain D region D2 2-8 RF2

<400> 65
Gly Tyr Cys Thr Asn Gly Val Cys Tyr Thr
 1               5                   10


<210> 66
<211> 9
<212> PRT
<213> Homo sapiens

<220>
<223> human germline heavy chain D region D2 2-8 RF3

<400> 66
Asp Ile Val Leu Met Val Tyr Ala Ile
 1               5


<210> 67
<211> 10
<212> PRT
<213> Homo sapiens

<220>
<223> human germline heavy chain D region D2 2-15 RF2

<400> 67
Gly Tyr Cys Ser Gly Gly Ser Cys Tyr Ser
1               5                   10

<210> 68
<211> 9
<212> PRT
<213> Homo sapiens

<220>
<223> human germline heavy chain D region D2 2-15 RF3

<400> 68
Asp Ile Val Val Val Val Ala Ala Thr
1               5

<210> 69
<211> 5
<212> PRT
<213> Homo sapiens

<220>
<223> portion of human germline heavy chain D region D2
      2-21 RF1

<400> 69
Ser Ile Leu Trp Trp
1               5

<210> 70
<211> 9
<212> PRT
<213> Homo sapiens

<220>
<223> human germline heavy chain D region D2 2-21 RF2

<400> 70
Ala Tyr Cys Gly Gly Asp Cys Tyr Ser
1               5

<210> 71
<211> 8
<212> PRT
<213> Homo sapiens

<220>
<223> human germline heavy chain D region D2 2-21 RF3

<400> 71
His Ile Val Val Val Thr Ala Ile
1               5

<210> 72
<211> 10
<212> PRT
<213> Homo sapiens

<220>
<223> human germline heavy chain D region D3 3-3 RF1

<400> 72
Val Leu Arg Phe Leu Glu Trp Leu Leu Tyr
1               5                   10



<210> 73
<211> 10
<212> PRT
<213> Homo sapiens

<220>
<223> human germline heavy chain D region D3 3-3 RF2

<400> 73
Tyr Tyr Asp Phe Trp Ser Gly Tyr Tyr Thr
1               5                   10



<210> 74
<211> 9
<212> PRT
<213> Homo sapiens

<220>
<223> human germline heavy chain D region D3 3-3 RF3

<400> 74
Ile Thr Ile Phe Gly Val Val Ile Ile
1               5



<210> 75
<211> 9
<212> PRT
<213> Homo sapiens

<220>
<223> human germline heavy chain D region D3 3-9 RF1

<400> 75
Val Leu Arg Tyr Phe Asp Trp Leu Leu
1               5



<210> 76
<211> 10
<212> PRT
<213> Homo sapiens

<220>
<223> human germline heavy chain D region D3 3-9 RF2

<400> 76
Tyr Tyr Asp Ile Leu Thr Gly Tyr Tyr Asn
1               5                   10



<210> 77
<211> 4

<212> PRT
<213> Homo sapiens

<220>
<223> portion of human germline heavy chain D region D3
      3-9 RF3

<400> 77
Ile Thr Ile Phe
1


<210> 78
<211> 4
<212> PRT
<213> Homo sapiens

<220>
<223> portion of human germline heavy chain D region D3
      3-9 RF3

<400> 78
Leu Val Ile Ile
1


<210> 79
<211> 9
<212> PRT
<213> Homo sapiens

<220>
<223> human germline heavy chain D region D3 3-10 RF1

<400> 79
Val Leu Leu Trp Phe Gly Glu Leu Leu
 1               5


<210> 80
<211> 10
<212> PRT
<213> Homo sapiens

<220>
<223> human germline heavy chain D region D3 3-10 RF2

<400> 80
Tyr Tyr Tyr Gly Ser Gly Ser Tyr Tyr Asn
 1               5                   10


<210> 81
<211> 9
<212> PRT
<213> Homo sapiens

<220>
<223> human germline heavy chain D region D3 3-10 RF3

<400> 81
Ile Thr Met Val Arg Gly Val Ile Ile
 1               5

<210> 82
<211> 9
<212> PRT
<213> Homo sapiens

<220>
<223> portion of human germline heavy chain D region D3
      3-16 RF1

<400> 82
Leu Arg Leu Gly Glu Leu Ser Leu Tyr
 1               5


<210> 83
<211> 12
<212> PRT
<213> Homo sapiens

<220>
<223> human germline heavy chain D region D3 3-16 RF2

<400> 83
Tyr Tyr Asp Tyr Val Trp Gly Ser Tyr Arg Tyr Thr
 1               5                   10


<210> 84
<211> 11
<212> PRT
<213> Homo sapiens

<220>
<223> human germline heavy chain D region D3 3-16 RF3

<400> 84
Ile Met Ile Thr Phe Gly Gly Val Ile Val Ile
 1               5                   10


<210> 85
<211> 4
<212> PRT
<213> Homo sapiens

<220>
<223> portion of human germline heavy chain D region D3
      3-22 RF1

<400> 85
Trp Leu Leu Leu
1


<210> 86
<211> 10
<212> PRT
<213> Homo sapiens

<220>
<223> human germline heavy chain D region D3 3-22 RF2

<400> 86
Tyr Tyr Tyr Asp Ser Ser Gly Tyr Tyr Tyr
1               5                   10

<210> 87
<211> 9
<212> PRT
<213> Homo sapiens

<220>
<223> human germline heavy chain D region D3 3-22 RF3

<400> 87
Ile Thr Met Ile Val Val Val Ile Thr
1               5

<210> 88
<211> 5
<212> PRT
<213> Homo sapiens

<220>
<223> human germline heavy chain D region D4 4-4 RF2 and
      4-11 RF2

<400> 88
Asp Tyr Ser Asn Tyr
1               5

<210> 89
<211> 4
<212> PRT
<213> Homo sapiens

<220>
<223> human germline heavy chain D region D4 4-4 RF3,
      4-11 RF3 and 4-17 RF3

<400> 89
Thr Thr Val Thr
1

<210> 90
<211> 5
<212> PRT
<213> Homo sapiens

<220>
<223> human germline heavy chain D region D4 4-17 RF2

<400> 90
Asp Tyr Gly Asp Tyr
1               5

<210> 91
<211> 6
<212> PRT

<213> Homo sapiens

<220>
<223> human germline heavy chain D region D4 4-23 RF2

<400> 91
Asp Tyr Gly Gly Asn Ser
1               5


<210> 92
<211> 5
<212> PRT
<213> Homo sapiens

<220>
<223> human germline heavy chain D region D4 4-23 RF3

<400> 92
Thr Thr Val Val Thr
1               5


<210> 93
<211> 6
<212> PRT
<213> Homo sapiens

<220>
<223> human germline heavy chain D region D5 5-5 RF1 and
      5-18 RF1

<400> 93
Val Asp Thr Ala Met Val
1               5


<210> 94
<211> 6
<212> PRT
<213> Homo sapiens

<220>
<223> human germline heavy chain D region D5 5-5 RF2 and
      5-18 RF2

<400> 94
Trp Ile Gln Leu Trp Leu
1               5


<210> 95
<211> 6
<212> PRT
<213> Homo sapiens

<220>
<223> human germline heavy chain D region D5 5-5 RF3 and
      5-18 RF3

<400> 95
Gly Tyr Ser Tyr Gly Tyr
1               5

<210> 96
<211> 7
<212> PRT
<213> Homo sapiens

<220>
<223> human germline heavy chain D region D5 5-12 RF1

<400> 96
Val Asp Ile Val Ala Thr Ile
1               5

<210> 97
<211> 4
<212> PRT
<213> Homo sapiens

<220>
<223> portion of human germline heavy chain D region D5
      5-12 RF2

<400> 97
Trp Leu Arg Leu
1

<210> 98
<211> 7
<212> PRT
<213> Homo sapiens

<220>
<223> human germline heavy chain D region D5 5-12 RF3

<400> 98
Gly Tyr Ser Gly Tyr Asp Tyr
1               5

<210> 99
<211> 6
<212> PRT
<213> Homo sapiens

<220>
<223> human germline heavy chain D region D5 5-24 RF1

<400> 99
Val Glu Met Ala Thr Ile
1               5

<210> 100
<211> 5
<212> PRT
<213> Homo sapiens

<220>
<223> portion of human germline heavy chain D region D5
      5-24 RF2

<400> 100
Arg Trp Leu Gln Leu
1               5

<210> 101
<211> 6
<212> PRT
<213> Homo sapiens

<220>
<223> human germline heavy chain D region D5 5-24 RF3

<400> 101
Arg Asp Gly Tyr Asn Tyr
1               5

<210> 102
<211> 6
<212> PRT
<213> Homo sapiens

<220>
<223> human germline heavy chain D region D6 6-6 RF1

<400> 102
Glu Tyr Ser Ser Ser Ser
1               5

<210> 103
<211> 5
<212> PRT
<213> Homo sapiens

<220>
<223> human germline heavy chain D region D6 6-6 RF2

<400> 103
Ser Ile Ala Ala Arg
1               5

<210> 104
<211> 7
<212> PRT
<213> Homo sapiens

<220>
<223> human germline heavy chain D region D6 6-13 RF1
      and 6-19 RF1

<400> 104
Gly Tyr Ser Ser Ser Trp Tyr
1               5

<210> 105
<211> 6
<212> PRT
<213> Homo sapiens

<220>
<223> human germline heavy chain D region D6 6-13 RF2

<400> 105
Gly Ile Ala Ala Ala Gly
1               5

<210> 106
<211> 4
<212> PRT
<213> Homo sapiens

<220>
<223> portion of human germline heavy chain D region D6
      6-13 RF3

<400> 106
Gln Gln Leu Val
1

<210> 107
<211> 6
<212> PRT
<213> Homo sapiens

<220>
<223> human germline heavy chain D region D6 6-19 RF2

<400> 107
Gly Ile Ala Val Ala Gly
1               5

<210> 108
<211> 4
<212> PRT
<213> Homo sapiens

<220>
<223> portion of human germline heavy chain D region D6
      6-19 RF3

<400> 108
Gln Trp Leu Val
1

<210> 109
<211> 17
<212> PRT
<213> Homo sapiens

<220>
<223> human germline heavy chain J region JH1

<400> 109
Ala Glu Tyr Phe Gln His Trp Gly Gln Gly Thr Leu Val Thr Val Ser
1               5                   10                  15
Ser

<210> 110
<211> 17
<212> PRT
<213> Homo sapiens

<220>
<223> human germline heavy chain J region JH2

<400> 110
Tyr Trp Tyr Phe Asp Leu Trp Gly Arg Gly Thr Leu Val Thr Val Ser
1               5                   10                  15
Ser


<210> 111
<211> 15
<212> PRT
<213> Homo sapiens

<220>
<223> human germline heavy chain J region JH3

<400> 111
Ala Phe Asp Ile Trp Gly Gln Gly Thr Met Val Thr Val Ser Ser
1               5                   10                  15


<210> 112
<211> 15
<212> PRT
<213> Homo sapiens

<220>
<223> human germline heavy chain J region JH4

<400> 112
Tyr Phe Asp Tyr Trp Gly Gln Gly Thr Leu Val Thr Val Ser Ser
1               5                   10                  15


<210> 113
<211> 16
<212> PRT
<213> Homo sapiens

<220>
<223> human germline heavy chain J region JH5

<400> 113
Asn Trp Phe Asp Pro Trp Gly Gln Gly Thr Leu Val Thr Val Ser Ser
1               5                   10                  15


<210> 114
<211> 20
<212> PRT
<213> Homo sapiens

<220>
<223> human germline heavy chain J region JH6

<400> 114

Tyr Tyr Tyr Tyr Tyr Gly Met Asp Val Trp Gly Gln Gly Thr Thr Val
1               5                   10                  15
Thr Val Ser Ser
            20

<210> 115
<211> 95
<212> PRT
<213> Homo sapiens

<220>
<223> human germline light chain kappa variable region
      VKI minigene O12 and O2

<400> 115
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Ile Ser Ser Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ser Tyr Ser Thr Pro
                85                  90                  95

<210> 116
<211> 95
<212> PRT
<213> Homo sapiens

<220>
<223> human germline light chain kappa variable region
      VKI minigene O18 and O8

<400> 116
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Ser Asn Tyr
            20                  25                  30
Leu Asn Trp Tyr Gln Gln Lys Pro Gly Lys Ala Pro Lys Leu Leu Ile
        35                  40                  45
Tyr Asp Ala Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys Gln Gln Tyr Asp Asn Leu Pro
                85                  90                  95

<210> 117
<211> 95
<212> PRT
<213> Homo sapiens

<220>
<223> human germline light chain kappa variable region
      VKI minigene A20

<400> 117
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Ser Asn Tyr
            20                  25                  30
Leu Ala Trp Tyr Gln Gln Lys Pro Gly Lys Val Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Lys Tyr Asn Ser Ala Pro
                85                  90                  95

<210> 118
<211> 95
<212> PRT
<213> Homo sapiens

<220>
<223> human germline light chain kappa variable region
      VKI minigene A30

<400> 118
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Arg Asn Asp
            20                  25                  30
Leu Gly Trp Tyr Gln Gln Lys Pro Gly Lys Ala Pro Lys Arg Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Leu Gln His Asn Ser Tyr Pro
                85                  90                  95

<210> 119
<211> 95
<212> PRT
<213> Homo sapiens

<220>
<223> human germline light chain kappa variable region
      VKI minigene L14

<400> 119
Asn Ile Gln Met Thr Gln Ser Pro Ser Ala Met Ser Ala Ser Val Gly
1               5                   10                  15
Asp Arg Val Thr Ile Thr Cys Arg Ala Arg Gln Gly Ile Ser Asn Tyr
            20                  25                  30
Leu Ala Trp Phe Gln Gln Lys Pro Gly Lys Val Pro Lys His Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Leu Gln His Asn Ser Tyr Pro
                85                  90                  95

<210> 120
<211> 95
<212> PRT
<213> Homo sapiens

<220>
<223> human germline light chain kappa variable region
      VKI minigene L1

<400> 120
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Ser Asn Tyr
            20                  25                  30
Leu Ala Trp Phe Gln Gln Lys Pro Gly Lys Ala Pro Lys Ser Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Asn Ser Tyr Pro
                85                  90                  95


<210> 121
<211> 95
<212> PRT
<213> Homo sapiens

<220>
<223> human germline light chain kappa variable region
      VKI minigene L15

<400> 121
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Ser Ser Trp
            20                  25                  30
Leu Ala Trp Tyr Gln Gln Lys Pro Glu Lys Ala Pro Lys Ser Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Asn Ser Tyr Pro
                85                  90                  95


<210> 122
<211> 95
<212> PRT
<213> Homo sapiens

<220>
<223> human germline light chain kappa variable region
      VKI minigene L4 and L18

<400> 122
Ala Ile Gln Leu Thr Gln Ser Pro Ser Ser Leu Ser Ala Ser Val Gly
1               5                   10                  15
Asp Arg Val Thr Ile Thr Cys Arg Ala Ser Gln Gly Ile Ser Ser Ala
            20                  25                  30
Leu Ala Trp Tyr Gln Gln Lys Pro Gly Lys Ala Pro Lys Leu Leu Ile

35                  40                  45
Tyr Asp Ala Ser Ser Leu Glu Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Phe Asn Ser Tyr Pro
                85                  90                  95

<210> 123
<211> 95
<212> PRT
<213> Homo sapiens

<220>
<223> human germline light chain kappa variable region
      VKI minigene L5 and L19

<400> 123
Asp Ile Gln Met Thr Gln Ser Pro Ser Ser Val Ser Ala Ser Val Gly
 1               5                  10                  15
Asp Arg Val Thr Ile Thr Cys Arg Ala Ser Gln Gly Ile Ser Ser Trp
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ala Asn Ser Phe Pro
                85                  90                  95

<210> 124
<211> 95
<212> PRT
<213> Homo sapiens

<220>
<223> human germline light chain kappa variable region
      VKI minigene L8

<400> 124
Asp Ile Gln Leu Thr Gln Ser Pro Ser Phe Leu Ser Ala Ser Val Gly
 1               5                  10                  15
Asp Arg Val Thr Ile Thr Cys Arg Ala Ser Gln Gly Ile Ser Ser Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Gln Gln Leu Asn Ser Tyr Pro
                85                  90                  95

<210> 125
<211> 95
<212> PRT
<213> Homo sapiens

<220>

<223> human germline light chain kappa variable region
      VKI minigene L23

<400> 125
Ala Ile Arg Met Thr Gln Ser Pro Phe Ser Leu Ser Ala Ser Val Gly
1               5                   10                  15
Asp Arg Val Thr Ile Thr Cys Trp Ala Ser Gln Gly Ile Ser Ser Tyr
            20                  25                  30
Leu Ala Trp Tyr Gln Gln Lys Pro Ala Lys Ala Pro Lys Leu Phe Ile
        35                  40                  45
Tyr Tyr Ala Ser Ser Leu Gln Ser Gly Val Pro Ser Arg Phe Ser Gly
    50                  55                  60
Ser Gly Ser Gly Thr Asp Tyr Thr Leu Thr Ile Ser Ser Leu Gln Pro
65                  70                  75                  80
Glu Asp Phe Ala Thr Tyr Tyr Cys Gln Gln Tyr Tyr Ser Thr Pro
                85                  90                  95


<210> 126
<211> 95
<212> PRT
<213> Homo sapiens

<220>
<223> human germline light chain kappa variable region
      VKI minigene L9

<400> 126
Ala Ile Arg Met Thr Gln Ser Pro Ser Ser Phe Ser Ala Ser Thr Gly
1               5                   10                  15
Asp Arg Val Thr Ile Thr Cys Arg Ala Ser Gln Gly Ile Ser Ser Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Cys Leu Gln Ser
65                  70                  75                  80
Glu Asp Phe Ala Thr Tyr Tyr Cys Gln Gln Tyr Tyr Ser Tyr Pro
                85                  90                  95


<210> 127
<211> 95
<212> PRT
<213> Homo sapiens

<220>
<223> human germline light chain kappa variable region
      VKI minigene L24

<400> 127
Val Ile Trp Met Thr Gln Ser Pro Ser Leu Leu Ser Ala Ser Thr Gly
1               5                   10                  15
Asp Arg Val Thr Ile Ser Cys Arg Met Ser Gln Gly Ile Ser Ser Tyr
            20                  25                  30
Leu Ala Trp Tyr Gln Gln Lys Pro Gly Lys Ala Pro Glu Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Cys Leu Gln Ser
65                  70                  75                  80
Glu Asp Phe Ala Thr Tyr Tyr Cys Gln Gln Tyr Tyr Ser Phe Pro

85                  90                  95

<210> 128
<211> 95
<212> PRT
<213> Homo sapiens

<220>
<223> human germline light chain kappa variable region
      VKI minigene L11

<400> 128
Ala Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Arg Asn Asp
            20                  25                  30
Leu Gly Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Leu Gln Asp Tyr Asn Tyr Pro
                85                  90                  95

<210> 129
<211> 95
<212> PRT
<213> Homo sapiens

<220>
<223> human germline light chain kappa variable region
      VKI minigene L12

<400> 129
Asp Ile Gln Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Asp Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Asn Ser Tyr Ser
                85                  90                  95

<210> 130
<211> 101
<212> PRT
<213> Homo sapiens

<220>
<223> human germline light chain kappa variable region
      VKII minigene O11 and O1

<400> 130
Asp Ile Val Met Thr Gln Thr Pro Leu Ser Leu Pro Val Thr Pro Gly
1               5                   10                  15

Glu Pro Ala Ser Ile Ser Cys Arg Ser Ser Gln Ser Leu Leu Asp Ser
                20                  25                  30
Asp Asp Gly Asn Thr Tyr Leu Asp Trp Tyr Leu Gln Lys Pro Gly Gln
            35                  40                  45
Ser Pro Gln Leu Leu Ile Tyr Thr Leu Ser Tyr Arg Ala Ser Gly Val
    50                  55                  60
Pro Asp Arg Phe Ser Gly Ser Gly Ser Gly Thr Asp Phe Thr Leu Lys
65                  70                  75                  80
Ile Ser Arg Val Glu Ala Glu Asp Val Gly Val Tyr Tyr Cys Met Gln
                85                  90                  95
Arg Ile Glu Phe Pro
                100

<210> 131
<211> 100
<212> PRT
<213> Homo sapiens

<220>
<223> human germline light chain kappa variable region
      VKII minigene A17

<400> 131
Asp Val Val Met Thr Gln Ser Pro Leu Ser Leu Pro Val Thr Leu Gly
 1               5                  10                  15
Gln Pro Ala Ser Ile Ser Cys Arg Ser Ser Gln Ser Leu Val Tyr Ser
                20                  25                  30
Asp Gly Asn Thr Tyr Leu Asn Trp Phe Gln Gln Arg Pro Gly Gln Ser
            35                  40                  45
Pro Arg Arg Leu Ile Tyr Lys Val Ser Asn Arg Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro
                100

<210> 132
<211> 100
<212> PRT
<213> Homo sapiens

<220>
<223> human germline light chain kappa variable region
      VKII minigene A1

<400> 132
Asp Val Val Met Thr Gln Ser Pro Leu Ser Leu Pro Val Thr Leu Gly
 1               5                  10                  15
Gln Pro Ala Ser Ile Ser Cys Arg Ser Ser Gln Ser Leu Val Tyr Ser
                20                  25                  30
Asp Gly Asn Thr Tyr Leu Asn Trp Phe Gln Gln Arg Pro Gly Gln Ser
            35                  40                  45
Pro Arg Arg Leu Ile Tyr Lys Val Ser Asn Trp Asp Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Thr His Trp Pro

            100

<210> 133
<211> 100
<212> PRT
<213> Homo sapiens

<220>
<223> human germline light chain kappa variable region
      VKII minigene A18

<400> 133
Asp Ile Val Met Thr Gln Thr Pro Leu Ser Leu Ser Val Thr Pro Gly
1               5                   10                  15
Gln Pro Ala Ser Ile Ser Cys Lys Ser Ser Gln Ser Leu Leu His Ser
            20                  25                  30
Asp Gly Lys Thr Tyr Leu Tyr Trp Tyr Leu Gln Lys Pro Gly Gln Ser
        35                  40                  45
Pro Gln Leu Leu Ile Tyr Glu Val Ser Ser Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Gly
                85                  90                  95
Ile His Leu Pro
            100


<210> 134
<211> 100
<212> PRT
<213> Homo sapiens

<220>
<223> human germline light chain kappa variable region
      VKII minigene A2

<400> 134
Asp Ile Val Met Thr Gln Thr Pro Leu Ser Leu Ser Val Thr Pro Gly
1               5                   10                  15
Gln Pro Ala Ser Ile Ser Cys Lys Ser Ser Gln Ser Leu Leu His Ser
            20                  25                  30
Asp Gly Lys Thr Tyr Leu Tyr Trp Tyr Leu Gln Lys Pro Gly Gln Pro
        35                  40                  45
Pro Gln Leu Leu Ile Tyr Glu Val Ser Asn Arg Phe Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ser
                85                  90                  95
Ile Gln Leu Pro
            100


<210> 135
<211> 100
<212> PRT
<213> Homo sapiens

<220>
<223> human germline light chain kappa variable region
      VKII minigene A19 and A3

<400> 135
Asp Ile Val Met Thr Gln Ser Pro Leu Ser Leu Pro Val Thr Pro Gly
1               5                   10                  15
Glu Pro Ala Ser Ile Ser Cys Arg Ser Ser Gln Ser Leu Leu His Ser
            20                  25                  30
Asn Gly Tyr Asn Tyr Leu Asp Trp Tyr Leu Gln Lys Pro Gly Gln Ser
        35                  40                  45
Pro Gln Leu Leu Ile Tyr Leu Gly Ser Asn Arg Ala Ser Gly Val Pro
    50                  55                  60
Asp Arg Phe Ser Gly Ser Gly Ser Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Leu Gln Thr Pro
            100

<210> 136
<211> 100
<212> PRT
<213> Homo sapiens

<220>
<223> human germline light chain kappa variable region
      VKII minigene A23

<400> 136
Asp Ile Val Met Thr Gln Thr Pro Leu Ser Ser Pro Val Thr Leu Gly
1               5                   10                  15
Gln Pro Ala Ser Ile Ser Cys Arg Ser Ser Gln Ser Leu Val His Ser
            20                  25                  30
Asp Gly Asn Thr Tyr Leu Ser Trp Leu Gln Gln Arg Pro Gly Gln Pro
        35                  40                  45
Pro Arg Leu Leu Ile Tyr Lys Ile Ser Asn Arg Phe Ser Gly Val Pro
    50                  55                  60
Asp Arg Phe Ser Gly Ser Gly Ala Gly Thr Asp Phe Thr Leu Lys Ile
65                  70                  75                  80
Ser Arg Val Glu Ala Glu Asp Val Gly Val Tyr Tyr Cys Met Gln Ala
                85                  90                  95
Thr Gln Phe Pro
            100

<210> 137
<211> 96
<212> PRT
<213> Homo sapiens

<220>
<223> human germline light chain kappa variable region
      VKIII minigene A27

<400> 137
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Ser Ser Ser
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu

65                  70                  75                  80
Pro Glu Asp Phe Ala Val Tyr Tyr Cys Gln Gln Tyr Gly Ser Ser Pro
                85                  90                  95

<210> 138
<211> 96
<212> PRT
<213> Homo sapiens

<220>
<223> human germline light chain kappa variable region
      VKIII minigene A11

<400> 138
Glu Ile Val Leu Thr Gln Ser Pro Ala Thr Leu Ser Leu Ser Pro Gly
 1               5                  10                  15
Glu Arg Ala Thr Leu Ser Cys Gly Ala Ser Gln Ser Val Ser Ser Ser
            20                  25                  30
Tyr Leu Ala Trp Tyr Gln Gln Lys Pro Gly Leu Ala Pro Arg Leu Leu
        35                  40                  45
Ile Tyr Asp Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Pro
                85                  90                  95

<210> 139
<211> 95
<212> PRT
<213> Homo sapiens

<220>
<223> human germline light chain kappa variable region
      VKIII minigene L2 and L16

<400> 139
Glu Ile Val Met Thr Gln Ser Pro Ala Thr Leu Ser Val Ser Pro Gly
 1               5                  10                  15
Glu Arg Ala Thr Leu Ser Cys Arg Ala Ser Gln Ser Val Ser Ser Asn
            20                  25                  30
Leu Ala Trp Tyr Gln Gln Lys Pro Gly Gln Ala Pro Arg Leu Leu Ile
        35                  40                  45
Tyr Gly Ala Ser Thr Arg Ala Thr Gly Ile Pro Ala Arg Phe Ser Gly
    50                  55                  60
Ser Gly Ser Gly Thr Glu Phe Thr Leu Thr Ile Ser Ser Leu Gln Ser
65                  70                  75                  80
Glu Asp Phe Ala Val Tyr Tyr Cys Gln Gln Tyr Asn Asn Trp Pro
                85                  90                  95

<210> 140
<211> 95
<212> PRT
<213> Homo sapiens

<220>
<223> human germline light chain kappa variable region
      VKIII minigene L6

<400> 140

Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Arg Ser Asn Trp Pro
                85                  90                  95

<210> 141
<211> 95
<212> PRT
<213> Homo sapiens

<220>
<223> human germline light chain kappa variable region
      VKIII minigene L20

<400> 141
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Gly Val Ser Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Pro Gly Thr Asp Phe Thr Leu Thr Ile Ser Ser Leu Glu Pro
65                  70                  75                  80
Glu Asp Phe Ala Val Tyr Tyr Cys Gln Gln Arg Ser Asn Trp His
                85                  90                  95

<210> 142
<211> 96
<212> PRT
<213> Homo sapiens

<220>
<223> human germline light chain kappa variable region
      VKIII minigene L25

<400> 142
Glu Ile Val Met Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Ser
            20                  25                  30
Tyr Leu Ser Trp Tyr Gln Gln Lys Pro Gly Gln Ala Pro Arg Leu Leu
        35                  40                  45
Ile Tyr Gly Ala Ser Thr Arg Ala Thr Gly Ile Pro Ala Arg Phe Ser
    50                  55                  60
Gly Ser Gly Ser Gly Thr Asp Phe Thr Leu Thr Ile Ser Ser Leu Gln
65                  70                  75                  80
Pro Glu Asp Phe Ala Val Tyr Tyr Cys Gln Gln Asp Tyr Asn Leu Pro
                85                  90                  95

<210> 143

<211> 101
<212> PRT
<213> Homo sapiens

<220>
<223> human germline light chain kappa variable region
      VKIV minigene B3

<400> 143
Asp Ile Val Met Thr Gln Ser Pro Asp Ser Leu Ala Val Ser Leu Gly
1               5                   10                  15
Glu Arg Ala Thr Ile Asn Cys Lys Ser Ser Gln Ser Val Leu Tyr Ser
            20                  25                  30
Ser Asn Asn Lys Asn Tyr Leu Ala Trp Tyr Gln Gln Lys Pro Gly Gln
        35                  40                  45
Pro Pro Lys Leu Leu Ile Tyr Trp Ala Ser Thr Arg Glu Ser Gly Val
    50                  55                  60
Pro Asp Arg Phe Ser Gly Ser Gly Ser Gly Thr Asp Phe Thr Leu Thr
65                  70                  75                  80
Ile Ser Ser Leu Gln Ala Glu Asp Val Ala Val Tyr Tyr Cys Gln Gln
                85                  90                  95
Tyr Tyr Ser Thr Pro
            100


<210> 144
<211> 95
<212> PRT
<213> Homo sapiens

<220>
<223> human germline light chain kappa variable region
      VKV minigene B2

<400> 144
Glu Thr Thr Leu Thr Gln Ser Pro Ala Phe Met Ser Ala Thr Pro Gly
1               5                   10                  15
Asp Lys Val Asn Ile Ser Cys Lys Ala Ser Gln Asp Ile Asp Asp Asp
            20                  25                  30
Met Asn Trp Tyr Gln Gln Lys Pro Gly Glu Ala Ala Ile Phe Ile Ile
        35                  40                  45
Gln Glu Ala Thr Thr Leu Val Pro Gly Ile Pro Pro Arg Phe Ser Gly
    50                  55                  60
Ser Gly Tyr Gly Thr Asp Phe Thr Leu Thr Ile Asn Asn Ile Glu Ser
65                  70                  75                  80
Glu Asp Ala Ala Tyr Tyr Phe Cys Leu Gln His Asp Asn Phe Pro
                85                  90                  95


<210> 145
<211> 95
<212> PRT
<213> Homo sapiens

<220>
<223> human germline light chain kappa variable region
      VKVI minigene A26 and A10

<400> 145
Glu Ile Val Leu Thr Gln Ser Pro Asp Phe Gln Ser Val Thr Pro Lys
1               5                   10                  15
Glu Lys Val Thr Ile Thr Cys Arg Ala Ser Gln Ser Ile Gly Ser Ser
            20                  25                  30

Leu His Trp Tyr Gln Gln Lys Pro Asp Gln Ser Pro Lys Leu Leu Ile
            35                  40                  45
Lys Tyr Ala Ser Gln Ser Phe Ser Gly Val Pro Ser Arg Phe Ser Gly
    50                  55                  60
Ser Gly Ser Gly Thr Asp Phe Thr Leu Thr Ile Asn Ser Leu Glu Ala
65                  70                  75                  80
Glu Asp Ala Ala Thr Tyr Tyr Cys His Gln Ser Ser Ser Leu Pro
                85                  90                  95

<210> 146
<211> 95
<212> PRT
<213> Homo sapiens

<220>
<223> human germline light chain kappa variable region
      VKVI minigene A14

<400> 146
Asp Val Val Met Thr Gln Ser Pro Ala Phe Leu Ser Val Thr Pro Gly
 1               5                  10                  15
Glu Lys Val Thr Ile Thr Cys Gln Ala Ser Glu Gly Ile Gly Asn Tyr
            20                  25                  30
Leu Tyr Trp Tyr Gln Gln Lys Pro Asp Gln Ala Pro Lys Leu Leu Ile
            35                  40                  45
Lys Tyr Ala Ser Gln Ser Ile Ser Gly Val Pro Ser Arg Phe Ser Gly
    50                  55                  60
Ser Gly Ser Gly Thr Asp Phe Thr Phe Thr Ile Ser Ser Leu Glu Ala
65                  70                  75                  80
Glu Asp Ala Ala Thr Tyr Tyr Cys Gln Gln Gly Asn Lys His Pro
                85                  90                  95

<210> 147
<211> 98
<212> PRT
<213> Homo sapiens

<220>
<223> human germline light chain lambda variable region
      VL1 minigene 1a

<400> 147
Gln Ser Val Leu Thr Gln Pro Pro Ser Val Ser Glu Ala Pro Arg Gln
 1               5                  10                  15
Arg Val Thr Ile Ser Cys Ser Gly Ser Ser Ser Asn Ile Gly Asn Asn
            20                  25                  30
Ala Val Asn Trp Tyr Gln Gln Leu Pro Gly Lys Ala Pro Lys Leu Leu
            35                  40                  45
Ile Tyr Tyr Asp Asp Leu Leu Pro Ser Gly Val Ser Asp Arg Phe Ser
    50                  55                  60
Gly Ser Lys Ser Gly Thr Ser Ala Ser Leu Ala Ile Ser Gly Leu Gln
65                  70                  75                  80
Ser Glu Asp Glu Ala Asp Tyr Tyr Cys Ala Ala Trp Asp Asp Ser Leu
                85                  90                  95
Asn Gly

<210> 148
<211> 99
<212> PRT
<213> Homo sapiens

<220>
<223> human germline light chain lambda variable region
      VL1 minigene 1e

<400> 148
Gln Ser Val Leu Thr Gln Pro Pro Ser Val Ser Gly Ala Pro Gly Gln
 1               5                  10                  15
Arg Val Thr Ile Ser Cys Thr Gly Ser Ser Ser Asn Ile Gly Ala Gly
            20                  25                  30
Tyr Asp Val His Trp Tyr Gln Gln Leu Pro Gly Thr Ala Pro Lys Leu
        35                  40                  45
Leu Ile Tyr Gly Asn Ser Asn Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Ala Glu Asp Glu Ala Asp Tyr Tyr Cys Gln Ser Tyr Asp Ser Ser
                85                  90                  95
Leu Ser Gly


<210> 149
<211> 98
<212> PRT
<213> Homo sapiens

<220>
<223> human germline light chain lambda variable region
      VL1 minigene 1c

<400> 149
Gln Ser Val Leu Thr Gln Pro Pro Ser Ala Ser Gly Thr Pro Gly Gln
 1               5                  10                  15
Arg Val Thr Ile Ser Cys Ser Gly Ser Ser Ser Asn Ile Gly Ser Asn
            20                  25                  30
Thr Val Asn Trp Tyr Gln Gln Leu Pro Gly Thr Ala Pro Lys Leu Leu
        35                  40                  45
Ile Tyr Ser Asn Asn Gln Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
Ser Glu Asp Glu Ala Asp Tyr Tyr Cys Ala Ala Trp Asp Asp Ser Leu
                85                  90                  95
Asn Gly


<210> 150
<211> 98
<212> PRT
<213> Homo sapiens

<220>
<223> human germline light chain lambda variable region
      VL1 minigene 1g

<400> 150
Gln Ser Val Leu Thr Gln Pro Pro Ser Ala Ser Gly Thr Pro Gly Gln
 1               5                  10                  15
Arg Val Thr Ile Ser Cys Ser Gly Ser Ser Ser Asn Ile Gly Ser Asn
            20                  25                  30
Tyr Val Tyr Trp Tyr Gln Gln Leu Pro Gly Thr Ala Pro Lys Leu Leu
        35                  40                  45
Ile Tyr Arg Asn Asn Gln Arg Pro Ser Gly Val Pro Asp Arg Phe Ser

    50                  55                  60
Gly Ser Lys Ser Gly Thr Ser Ala Ser Leu Ala Ile Ser Gly Leu Arg
65                  70                  75                  80
Ser Glu Asp Glu Ala Asp Tyr Tyr Cys Ala Ala Trp Asp Asp Ser Leu
                85                  90                  95
Ser Gly



<210> 151
<211> 98
<212> PRT
<213> Homo sapiens

<220>
<223> human germline light chain lambda variable region
      VL1 minigene 1b

<400> 151
Gln Ser Val Leu Thr Gln Pro Pro Ser Val Ser Ala Ala Pro Gly Gln
 1               5                  10                  15
Lys Val Thr Ile Ser Cys Ser Gly Ser Ser Ser Asn Ile Gly Asn Asn
            20                  25                  30
Tyr Val Ser Trp Tyr Gln Gln Leu Pro Gly Thr Ala Pro Lys Leu Leu
        35                  40                  45
Ile Tyr Asp Asn Asn Lys Arg Pro Ser Gly Ile Pro Asp Arg Phe Ser
    50                  55                  60
Gly Ser Lys Ser Gly Thr Ser Ala Thr Leu Gly Ile Thr Gly Leu Gln
65                  70                  75                  80
Thr Gly Asp Glu Ala Asp Tyr Tyr Cys Gly Thr Trp Asp Ser Ser Leu
                85                  90                  95
Ser Ala



<210> 152
<211> 99
<212> PRT
<213> Homo sapiens

<220>
<223> human germline light chain lambda variable region
      VL2 minigene 2c

<400> 152
Gln Ser Ala Leu Thr Gln Pro Pro Ser Ala Ser Gly Ser Pro Gly Gln
 1               5                  10                  15
Ser Val Thr Ile Ser Cys Thr Gly Thr Ser Ser Asp Val Gly Gly Tyr
            20                  25                  30
Asn Tyr Val Ser Trp Tyr Gln Gln His Pro Gly Lys Ala Pro Lys Leu
        35                  40                  45
Met Ile Tyr Glu Val Ser Lys Arg Pro Ser Gly Val Pro Asp Arg Phe
    50                  55                  60
Ser Gly Ser Lys Ser Gly Asn Thr Ala Ser Leu Thr Val Ser Gly Leu
65                  70                  75                  80
Gln Ala Glu Asp Glu Ala Asp Tyr Tyr Cys Ser Ser Tyr Ala Gly Ser
                85                  90                  95
Asn Asn Phe



<210> 153
<211> 99
<212> PRT
<213> Homo sapiens

<220>
<223> human germline light chain lambda variable region
      VL2 minigene 2e

<400> 153
Gln Ser Ala Leu Thr Gln Pro Arg Ser Val Ser Gly Ser Pro Gly Gln
1               5                   10                  15
Ser Val Thr Ile Ser Cys Thr Gly Thr Ser Ser Asp Val Gly Gly Tyr
            20                  25                  30
Asn Tyr Val Ser Trp Tyr Gln Gln His Pro Gly Lys Ala Pro Lys Leu
        35                  40                  45
Met Ile Tyr Asp Val Ser Lys Arg Pro Ser Gly Val Pro Asp Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Cys Ser Tyr Ala Gly Ser
                85                  90                  95
Tyr Thr Phe


<210> 154
<211> 99
<212> PRT
<213> Homo sapiens

<220>
<223> human germline light chain lambda variable region
      VL2 minigene 2a2

<400> 154
Gln Ser Ala Leu Thr Gln Pro Ala Ser Val Ser Gly Ser Pro Gly Gln
1               5                   10                  15
Ser Ile Thr Ile Ser Cys Thr Gly Thr Ser Ser Asp Val Gly Gly Tyr
            20                  25                  30
Asn Tyr Val Ser Trp Tyr Gln Gln His Pro Gly Lys Ala Pro Lys Leu
        35                  40                  45
Met Ile Tyr Glu Val Ser Asn Arg Pro Ser Gly Val Ser Asn Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Ser Tyr Thr Ser Ser
                85                  90                  95
Ser Thr Leu


<210> 155
<211> 99
<212> PRT
<213> Homo sapiens

<220>
<223> human germline light chain lambda variable region
      VL2 minigene 2d

<400> 155
Gln Ser Ala Leu Thr Gln Pro Pro Ser Val Ser Gly Ser Pro Gly Gln
1               5                   10                  15
Ser Val Thr Ile Ser Cys Thr Gly Thr Ser Ser Asp Val Gly Ser Tyr
            20                  25                  30
Asn Arg Val Ser Trp Tyr Gln Gln Pro Pro Gly Thr Ala Pro Lys Leu
        35                  40                  45
Met Ile Tyr Glu Val Ser Asn Arg Pro Ser Gly Val Pro Asp Arg Phe
    50                  55                  60

Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Leu Tyr Thr Ser Ser
                85                  90                  95
Ser Thr Phe


<210> 156
<211> 99
<212> PRT
<213> Homo sapiens

<220>
<223> human germline light chain lambda variable region
      VL2 minigene 2b2

<400> 156
Gln Ser Ala Leu Thr Gln Pro Ala Ser Val Ser Gly Ser Pro Gly Gln
 1               5                  10                  15
Ser Ile Thr Ile Ser Cys Thr Gly Thr Ser Ser Asp Val Gly Ser Tyr
            20                  25                  30
Asn Leu Val Ser Trp Tyr Gln Gln His Pro Gly Lys Ala Pro Lys Leu
        35                  40                  45
Met Ile Tyr Glu Val Ser Lys Arg Pro Ser Gly Val Ser Asn Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Cys Ser Tyr Ala Gly Ser
                85                  90                  95
Ser Thr Phe


<210> 157
<211> 95
<212> PRT
<213> Homo sapiens

<220>
<223> human germline light chain lambda variable region
      VL3 minigene 3r

<400> 157
Ser Tyr Glu Leu Thr Gln Pro Pro Ser Val Ser Val Ser Pro Gly Gln
 1               5                  10                  15
Thr Ala Ser Ile Thr Cys Ser Gly Asp Lys Leu Gly Asp Lys Tyr Ala
            20                  25                  30
Cys Trp Tyr Gln Gln Lys Pro Gly Gln Ser Pro Val Leu Val Ile Tyr
        35                  40                  45
Gln Asp Ser Lys Arg Pro Ser Gly Ile Pro Glu Arg Phe Ser Gly Ser
    50                  55                  60
Asn Ser Gly Asn Thr Ala Thr Leu Thr Ile Ser Gly Thr Gln Ala Met
65                  70                  75                  80
Asp Glu Ala Asp Tyr Tyr Cys Gln Ala Trp Asp Ser Ser Thr Ala
                85                  90                  95


<210> 158
<211> 95
<212> PRT
<213> Homo sapiens

<220>
<223> human germline light chain lambda variable region

VL3 minigene 3j

<400> 158
Ser Tyr Glu Leu Thr Gln Pro Leu Ser Val Ser Val Ala Leu Gly Gln
1               5                   10                  15
Thr Ala Arg Ile Thr Cys Gly Gly Asn Asn Ile Gly Ser Lys Asn Val
            20                  25                  30
His Trp Tyr Gln Gln Lys Pro Gly Gln Ala Pro Val Leu Val Ile Tyr
        35                  40                  45
Arg Asp Ser Asn Arg Pro Ser Gly Ile Pro Glu Arg Phe Ser Gly Ser
    50                  55                  60
Asn Ser Gly Asn Thr Ala Thr Leu Thr Ile Ser Arg Ala Gln Ala Gly
65                  70                  75                  80
Asp Glu Ala Asp Tyr Tyr Cys Gln Val Trp Asp Ser Ser Thr Ala
                85                  90                  95


<210> 159
<211> 96
<212> PRT
<213> Homo sapiens

<220>
<223> human germline light chain lambda variable region
      VL3 minigene 3p

<400> 159
Ser Tyr Glu Leu Thr Gln Pro Pro Ser Val Ser Val Ser Pro Gly Gln
1               5                   10                  15
Thr Ala Arg Ile Thr Cys Ser Gly Asp Ala Leu Pro Lys Lys Tyr Ala
            20                  25                  30
Tyr Trp Tyr Gln Gln Lys Ser Gly Gln Ala Pro Val Leu Val Ile Tyr
        35                  40                  45
Glu Asp Ser Lys Arg Pro Ser Gly Ile Pro Glu Arg Phe Ser Gly Ser
    50                  55                  60
Ser Ser Gly Thr Met Ala Thr Leu Thr Ile Ser Gly Ala Gln Val Glu
65                  70                  75                  80
Asp Glu Ala Asp Tyr Tyr Cys Tyr Ser Thr Asp Ser Ser Gly Asn His
                85                  90                  95


<210> 160
<211> 96
<212> PRT
<213> Homo sapiens

<220>
<223> human germline light chain lambda variable region
      VL3 minigene 3a

<400> 160
Ser Tyr Glu Leu Thr Gln Pro Pro Ser Val Ser Val Ser Leu Gly Gln
1               5                   10                  15
Met Ala Arg Ile Thr Cys Ser Gly Glu Ala Leu Pro Lys Lys Tyr Ala
            20                  25                  30
Tyr Trp Tyr Gln Gln Lys Pro Gly Gln Phe Pro Val Leu Val Ile Tyr
        35                  40                  45
Lys Asp Ser Glu Arg Pro Ser Gly Ile Pro Glu Arg Phe Ser Gly Ser
    50                  55                  60
Ser Ser Gly Thr Ile Val Thr Leu Thr Ile Ser Gly Val Gln Ala Glu
65                  70                  75                  80
Asp Glu Ala Asp Tyr Tyr Cys Leu Ser Ala Asp Ser Ser Gly Thr Tyr
                85                  90                  95

<210> 161
<211> 96
<212> PRT
<213> Homo sapiens

<220>
<223> human germline light chain lambda variable region
      VL3 minigene 31

<400> 161
Ser Ser Glu Leu Thr Gln Asp Pro Ala Val Ser Val Ala Leu Gly Gln
1               5                   10                  15
Thr Val Arg Ile Thr Cys Gln Gly Asp Ser Leu Arg Ser Tyr Tyr Ala
            20                  25                  30
Ser Trp Tyr Gln Gln Lys Pro Gly Gln Ala Pro Val Leu Val Ile Tyr
        35                  40                  45
Gly Lys Asn Asn Arg Pro Ser Gly Ile Pro Asp Arg Phe Ser Gly Ser
    50                  55                  60
Ser Ser Gly Asn Thr Ala Ser Leu Thr Ile Thr Gly Ala Gln Ala Glu
65                  70                  75                  80
Asp Glu Ala Asp Tyr Tyr Cys Asn Ser Arg Asp Ser Ser Gly Asn His
                85                  90                  95


<210> 162
<211> 96
<212> PRT
<213> Homo sapiens

<220>
<223> human germline light chain lambda variable region
      VL3 minigene 3h

<400> 162
Ser Tyr Val Leu Thr Gln Pro Pro Ser Val Ser Val Ala Pro Gly Lys
1               5                   10                  15
Thr Ala Arg Ile Thr Cys Gly Gly Asn Asn Ile Gly Ser Lys Ser Val
            20                  25                  30
His Trp Tyr Gln Gln Lys Pro Gly Gln Ala Pro Val Leu Val Ile Tyr
        35                  40                  45
Tyr Asp Ser Asp Arg Pro Ser Gly Ile Pro Glu Arg Phe Ser Gly Ser
    50                  55                  60
Asn Ser Gly Asn Thr Ala Thr Leu Thr Ile Ser Arg Val Glu Ala Gly
65                  70                  75                  80
Asp Glu Ala Asp Tyr Tyr Cys Gln Val Trp Asp Ser Ser Ser Asp His
                85                  90                  95


<210> 163
<211> 94
<212> PRT
<213> Homo sapiens

<220>
<223> human germline light chain lambda variable region
      VL3 minigene 3e

<400> 163
Ser Tyr Glu Leu Thr Gln Leu Pro Ser Val Ser Val Ser Pro Gly Gln
1               5                   10                  15
Thr Ala Arg Ile Thr Cys Ser Gly Asp Val Leu Gly Glu Asn Tyr Ala

20                      25                      30
Asp Trp Tyr Gln Gln Lys Pro Gly Gln Ala Pro Glu Leu Val Ile Tyr
        35                  40                  45
Glu Asp Ser Glu Arg Tyr Pro Gly Ile Pro Glu Arg Phe Ser Gly Ser
    50                  55                  60
Thr Ser Gly Asn Thr Thr Thr Leu Thr Ile Ser Arg Val Leu Thr Glu
65                  70                  75                  80
Asp Glu Ala Asp Tyr Tyr Cys Leu Ser Gly Asp Glu Asp Asn
                85                  90

<210> 164
<211> 96
<212> PRT
<213> Homo sapiens

<220>
<223> human germline light chain lambda variable region
      VL3 minigene 3m

<400> 164
Ser Tyr Glu Leu Met Gln Pro Pro Ser Val Ser Val Ser Pro Gly Gln
 1               5                  10                  15
Thr Ala Arg Ile Thr Cys Ser Gly Asp Ala Leu Pro Lys Gln Tyr Ala
            20                  25                  30
Tyr Trp Tyr Gln Gln Lys Pro Gly Gln Ala Pro Val Leu Val Ile Tyr
        35                  40                  45
Lys Asp Ser Glu Arg Pro Ser Gly Ile Pro Glu Arg Phe Ser Gly Ser
    50                  55                  60
Ser Ser Gly Thr Thr Val Thr Leu Thr Ile Ser Gly Val Gln Ala Glu
65                  70                  75                  80
Asp Glu Ala Asp Tyr Tyr Cys Gln Ser Ala Asp Ser Ser Gly Thr Tyr
                85                  90                  95

<210> 165
<211> 94
<212> PRT
<213> Homo sapiens

<220>
<223> human germline light chain lambda variable region
      VL3 minigene 2-19

<400> 165
Ser Tyr Glu Leu Thr Gln Pro Ser Ser Val Ser Val Ser Pro Gly Gln
 1               5                  10                  15
Thr Ala Arg Ile Thr Cys Ser Gly Asp Val Leu Ala Lys Lys Tyr Ala
            20                  25                  30
Arg Trp Phe Gln Gln Lys Pro Gly Gln Ala Pro Val Leu Val Ile Tyr
        35                  40                  45
Lys Asp Ser Glu Arg Pro Ser Gly Ile Pro Glu Arg Phe Ser Gly Ser
    50                  55                  60
Ser Ser Gly Thr Thr Val Thr Leu Thr Ile Ser Gly Ala Gln Val Glu
65                  70                  75                  80
Asp Glu Ala Asp Tyr Tyr Cys Tyr Ser Ala Ala Asp Asn Asn
                85                  90

<210> 166
<211> 103
<212> PRT
<213> Homo sapiens

<220>
<223> human germline light chain lambda variable region
      VL4 minigene 4c

<400> 166
Leu Pro Val Leu Thr Gln Pro Pro Ser Ala Ser Ala Leu Leu Gly Ala
1               5                   10                  15
Ser Ile Lys Leu Thr Cys Thr Leu Ser Ser Glu His Ser Thr Tyr Thr
            20                  25                  30
Ile Glu Trp Tyr Gln Gln Arg Pro Gly Arg Ser Pro Gln Tyr Ile Met
        35                  40                  45
Lys Val Lys Ser Asp Gly Ser His Ser Lys Gly Asp Gly Ile Pro Asp
    50                  55                  60
Arg Phe Met Gly Ser Ser Ser Gly Ala Asp Arg Tyr Leu Thr Phe Ser
65                  70                  75                  80
Asn Leu Gln Ser Asp Asp Glu Ala Glu Tyr His Cys Gly Glu Ser His
                85                  90                  95
Thr Ile Asp Gly Gln Val Gly
            100


<210> 167
<211> 99
<212> PRT
<213> Homo sapiens

<220>
<223> human germline light chain lambda variable region
      VL4 minigene 4a

<400> 167
Gln Pro Val Leu Thr Gln Ser Ser Ser Ala Ser Ala Ser Leu Gly Ser
1               5                   10                  15
Ser Val Lys Leu Thr Cys Thr Leu Ser Ser Gly His Ser Ser Tyr Ile
            20                  25                  30
Ile Ala Trp His Gln Gln Gln Pro Gly Lys Ala Pro Arg Tyr Leu Met
        35                  40                  45
Lys Leu Glu Gly Ser Gly Ser Tyr Asn Lys Gly Ser Gly Val Pro Asp
    50                  55                  60
Arg Phe Ser Gly Ser Ser Ser Gly Ala Asp Arg Tyr Leu Thr Ile Ser
65                  70                  75                  80
Asn Leu Gln Leu Glu Asp Glu Ala Asp Tyr Tyr Cys Glu Thr Trp Asp
                85                  90                  95
Ser Asn Thr


<210> 168
<211> 99
<212> PRT
<213> Homo sapiens

<220>
<223> human germline light chain lambda variable region
      VL4 minigene 4b

<400> 168
Gln Leu Val Leu Thr Gln Ser Pro Ser Ala Ser Ala Ser Leu Gly Ala
1               5                   10                  15
Ser Val Lys Leu Thr Cys Thr Leu Ser Ser Gly His Ser Ser Tyr Ala
            20                  25                  30
Ile Ala Trp His Gln Gln Gln Pro Glu Lys Gly Pro Arg Tyr Leu Met

35                  40                  45
Lys Leu Asn Ser Asp Gly Ser His Ser Lys Gly Asp Gly Ile Pro Asp
    50                  55                  60
Arg Phe Ser Gly Ser Ser Ser Gly Ala Glu Arg Tyr Leu Thr Ile Ser
65                  70                  75                  80
Ser Leu Gln Ser Glu Asp Glu Ala Asp Tyr Tyr Cys Gln Thr Trp Gly
                85                  90                  95
Thr Gly Ile

<210> 169
<211> 104
<212> PRT
<213> Homo sapiens

<220>
<223> human germline light chain lambda variable region
      VL5 minigene 5e

<400> 169
Gln Pro Val Leu Thr Gln Pro Pro Ser Ser Ser Ala Ser Pro Gly Glu
 1               5                  10                  15
Ser Ala Arg Leu Thr Cys Thr Leu Pro Ser Asp Ile Asn Val Gly Ser
            20                  25                  30
Tyr Asn Ile Tyr Trp Tyr Gln Gln Lys Pro Gly Ser Pro Pro Arg Tyr
        35                  40                  45
Leu Leu Tyr Tyr Tyr Ser Asp Ser Asp Lys Gly Gln Gly Ser Gly Val
    50                  55                  60
Pro Ser Arg Phe Ser Gly Ser Lys Asp Ala Ser Ala Asn Thr Gly Ile
65                  70                  75                  80
Leu Leu Ile Ser Gly Leu Gln Ser Glu Asp Glu Ala Asp Tyr Tyr Cys
                85                  90                  95
Met Ile Trp Pro Ser Asn Ala Ser
            100

<210> 170
<211> 104
<212> PRT
<213> Homo sapiens

<220>
<223> human germline light chain lambda variable region
      VL5 minigene 5c

<400> 170
Gln Ala Val Leu Thr Gln Pro Ala Ser Leu Ser Ala Ser Pro Gly Ala
 1               5                  10                  15
Ser Ala Ser Leu Thr Cys Thr Leu Arg Ser Gly Ile Asn Val Gly Thr
            20                  25                  30
Tyr Arg Ile Tyr Trp Tyr Gln Gln Lys Pro Gly Ser Pro Pro Gln Tyr
        35                  40                  45
Leu Leu Arg Tyr Lys Ser Asp Ser Asp Lys Gln Gln Gly Ser Gly Val
    50                  55                  60
Pro Ser Arg Phe Ser Gly Ser Lys Asp Ala Ser Ala Asn Ala Gly Ile
65                  70                  75                  80
Leu Leu Ile Ser Gly Leu Gln Ser Glu Asp Glu Ala Asp Tyr Tyr Cys
                85                  90                  95
Met Ile Trp His Ser Ser Ala Ser
            100

<210> 171

<211> 105
<212> PRT
<213> Homo sapiens

<220>
<223> human germline light chain lambda variable region
      VL5 minigene 5b

<400> 171
Gln Pro Val Leu Thr Gln Pro Ser Ser His Ser Ala Ser Ser Gly Ala
1               5                   10                  15
Ser Val Arg Leu Thr Cys Met Leu Ser Ser Gly Phe Ser Val Gly Asp
            20                  25                  30
Phe Trp Ile Arg Trp Tyr Gln Gln Lys Pro Gly Asn Pro Pro Arg Tyr
        35                  40                  45
Leu Leu Tyr Tyr His Ser Asp Ser Asn Lys Gly Gln Gly Ser Gly Val
    50                  55                  60
Pro Ser Arg Phe Ser Gly Ser Asn Asp Ala Ser Ala Asn Ala Gly Ile
65                  70                  75                  80
Leu Arg Ile Ser Gly Leu Gln Pro Glu Asp Glu Ala Asp Tyr Tyr Cys
                85                  90                  95
Gly Thr Trp His Ser Asn Ser Lys Thr
            100                 105


<210> 172
<211> 98
<212> PRT
<213> Homo sapiens

<220>
<223> human germline light chain lambda variable region
      VL6minigene 6a

<400> 172
Asn Phe Met Leu Thr Gln Pro His Ser Val Ser Glu Ser Pro Gly Lys
1               5                   10                  15
Thr Val Thr Ile Ser Cys Thr Arg Ser Ser Gly Ser Ile Ala Ser Asn
            20                  25                  30
Tyr Val Gln Trp Tyr Gln Gln Arg Pro Gly Ser Ser Pro Thr Thr Val
        35                  40                  45
Ile Tyr Glu Asp Asn Gln Arg Pro Ser Gly Val Pro Asp Arg Phe Ser
    50                  55                  60
Gly Ser Ile Asp Ser Ser Ser Asn Ser Ala Ser Leu Thr Ile Ser Gly
65                  70                  75                  80
Leu Lys Thr Glu Asp Glu Ala Asp Tyr Tyr Cys Gln Ser Tyr Asp Ser
                85                  90                  95
Ser Asn


<210> 173
<211> 98
<212> PRT
<213> Homo sapiens

<220>
<223> human germline light chain lambda variable region
      VL7 minigene 7a

<400> 173
Gln Thr Val Val Thr Gln Glu Pro Ser Leu Thr Val Ser Pro Gly Gly
1               5                   10                  15
Thr Val Thr Leu Thr Cys Ala Ser Ser Thr Gly Ala Val Thr Ser Gly

20                  25                  30
Tyr Tyr Pro Asn Trp Phe Gln Gln Lys Pro Gly Gln Ala Pro Arg Ala
        35                  40                  45
Leu Ile Tyr Ser Thr Ser Asn Lys His Ser Trp Thr Pro Ala Arg Phe
    50                  55                  60
Ser Gly Ser Leu Leu Gly Gly Lys Ala Ala Leu Thr Leu Ser Gly Val
65                  70                  75                  80
Gln Pro Glu Asp Glu Ala Glu Tyr Tyr Cys Leu Leu Tyr Tyr Gly Gly
                85                  90                  95
Ala Gln


<210> 174
<211> 98
<212> PRT
<213> Homo sapiens

<220>
<223> human germline light chain lambda variable region
      VL7 minigene 7b

<400> 174
Gln Ala Val Val Thr Gln Glu Pro Ser Leu Thr Val Ser Pro Gly Gly
 1               5                  10                  15
Thr Val Thr Leu Thr Cys Gly Ser Ser Thr Gly Ala Val Thr Ser Gly
            20                  25                  30
His Tyr Pro Tyr Trp Phe Gln Gln Lys Pro Gly Gln Ala Pro Arg Thr
        35                  40                  45
Leu Ile Tyr Asp Thr Ser Asn Lys His Ser Trp Thr Pro Ala Arg Phe
    50                  55                  60
Ser Gly Ser Leu Leu Gly Gly Lys Ala Ala Leu Thr Leu Ser Gly Ala
65                  70                  75                  80
Gln Pro Glu Asp Glu Ala Glu Tyr Tyr Cys Leu Leu Ser Tyr Ser Gly
                85                  90                  95
Ala Arg


<210> 175
<211> 98
<212> PRT
<213> Homo sapiens

<220>
<223> human germline light chain lambda variable region
      VL8 minigene 8a

<400> 175
Gln Thr Val Val Thr Gln Glu Pro Ser Phe Ser Val Ser Pro Gly Gly
 1               5                  10                  15
Thr Val Thr Leu Thr Cys Gly Leu Ser Ser Gly Ser Val Ser Thr Ser
            20                  25                  30
Tyr Tyr Pro Ser Trp Tyr Gln Gln Thr Pro Gly Gln Ala Pro Arg Thr
        35                  40                  45
Leu Ile Tyr Ser Thr Asn Thr Arg Ser Ser Gly Val Pro Asp Arg Phe
    50                  55                  60
Ser Gly Ser Ile Leu Gly Asn Lys Ala Ala Leu Thr Ile Thr Gly Ala
65                  70                  75                  80
Gln Ala Asp Asp Glu Ser Asp Tyr Tyr Cys Val Leu Tyr Met Gly Ser
                85                  90                  95
Gly Ile


<210> 176

<211> 104
<212> PRT
<213> Homo sapiens

<220>
<223> human germline light chain lambda variable region
      VL9 minigene 9a

<400> 176
Gln Pro Val Leu Thr Gln Pro Pro Ser Ala Ser Ala Ser Leu Gly Ala
1               5                   10                  15
Ser Val Thr Leu Thr Cys Thr Leu Ser Ser Gly Tyr Ser Asn Tyr Lys
            20                  25                  30
Val Asp Trp Tyr Gln Gln Arg Pro Gly Lys Gly Pro Arg Phe Val Met
        35                  40                  45
Arg Val Gly Thr Gly Gly Ile Val Gly Ser Lys Gly Asp Gly Ile Pro
    50                  55                  60
Asp Arg Phe Ser Val Leu Gly Ser Gly Leu Asn Arg Tyr Leu Thr Ile
65                  70                  75                  80
Lys Asn Ile Gln Glu Glu Asp Glu Ser Asp Tyr His Cys Gly Ala Asp
                85                  90                  95
His Gly Ser Gly Ser Asn Phe Val
            100


<210> 177
<211> 98
<212> PRT
<213> Homo sapiens

<220>
<223> human germline light chain lambda variable region
      VL10 minigene 10a

<400> 177
Gln Ala Gly Leu Thr Gln Pro Pro Ser Val Ser Lys Gly Leu Arg Gln
1               5                   10                  15
Thr Ala Thr Leu Thr Cys Thr Gly Asn Ser Asn Asn Val Gly Asn Gln
            20                  25                  30
Gly Ala Ala Trp Leu Gln Gln His Gln Gly His Pro Pro Lys Leu Leu
        35                  40                  45
Ser Tyr Arg Asn Asn Asn Arg Pro Ser Gly Ile Ser Glu Arg Leu Ser
    50                  55                  60
Ala Ser Arg Ser Gly Asn Thr Ala Ser Leu Thr Ile Thr Gly Leu Gln
65                  70                  75                  80
Pro Glu Asp Glu Ala Asp Tyr Tyr Cys Ser Ala Trp Asp Ser Ser Leu
                85                  90                  95
Ser Ala


<210> 178
<211> 12
<212> PRT
<213> Homo sapiens

<220>
<223> human germline light chain kappa variable region J
      kappa minigene JK1

<400> 178
Trp Thr Phe Gly Gln Gly Thr Lys Val Glu Ile Lys
1               5                   10

<210> 179
<211> 12
<212> PRT
<213> Homo sapiens

<220>
<223> human germline light chain kappa variable region J
      kappa minigene JK2

<400> 179
Tyr Thr Phe Gly Gln Gly Thr Lys Leu Glu Ile Lys
 1               5                   10


<210> 180
<211> 12
<212> PRT
<213> Homo sapiens

<220>
<223> human germline light chain kappa variable region J
      kappa minigene JK3

<400> 180
Phe Thr Phe Gly Pro Gly Thr Lys Val Asp Ile Lys
 1               5                   10


<210> 181
<211> 12
<212> PRT
<213> Homo sapiens

<220>
<223> human germline light chain kappa variable region J
      kappa minigene JK4

<400> 181
Leu Thr Phe Gly Gly Gly Thr Lys Val Glu Ile Lys
 1               5                   10


<210> 182
<211> 12
<212> PRT
<213> Homo sapiens

<220>
<223> human germline light chain kappa variable region J
      kappa minigene JK5

<400> 182
Ile Thr Phe Gly Gln Gly Thr Arg Leu Glu Ile Lys
 1               5                   10


<210> 183
<211> 12
<212> PRT
<213> Homo sapiens

<220>

<223> human germline light chain lambda variable region
      J lambda minigene JL1

<400> 183
Tyr Val Phe Gly Thr Gly Thr Lys Val Thr Val Leu
 1               5                   10


<210> 184
<211> 12
<212> PRT
<213> Homo sapiens

<220>
<223> human germline light chain lambda variable region
      J lambda minigene JL2 and JL3

<400> 184
Val Val Phe Gly Gly Gly Thr Lys Leu Thr Val Leu
 1               5                   10


<210> 185
<211> 12
<212> PRT
<213> Homo sapiens

<220>
<223> human germline light chain lambda variable region
      J lambda minigene JL7

<400> 185
Ala Val Phe Gly Gly Gly Thr Gln Leu Thr Val Leu
 1               5                   10


<210> 186
<211> 24
<212> DNA
<213> Artificial Sequence

<220>
<223> V-region heavy chain forward primer VH1FA

<400> 186
cagatgcagc tggtgcagtc tggg                                              24

<210> 187
<211> 24
<212> DNA
<213> Artificial Sequence

<220>
<223> V-region heavy chain forward primer VH1FB

<400> 187
caaatgcagc tggtgcagtc tggg                                              24

<210> 188
<211> 22
<212> DNA
<213> Artificial Sequence

<220>
<223> V-region heavy chain forward primer VH1FC

<400> 188
caggtgcagc tggtgcagtc tg                                                22

<210> 189
<211> 23
<212> DNA
<213> Artificial Sequence

<220>
<223> V-region heavy chain forward primer VH1FD

<400> 189
caggttcagc tggtgcagtc tgg                                               23

<210> 190
<211> 23
<212> DNA
<213> Artificial Sequence

<220>
<223> V-region heavy chain forward primer VH1FE

<400> 190
caggtccagc tggtacagtc tgg                                               23

<210> 191
<211> 23
<212> DNA
<213> Artificial Sequence

<220>
<223> V-region heavy chain forward primer VH2FA

<400> 191
caggtcacct tgaaggagtc tgg                                               23

<210> 192
<211> 23
<212> DNA
<213> Artificial Sequence

<220>
<223> V-region heavy chain forward primer VH2FB

<400> 192
caggtcacct tgagggagtc tgg                                               23

<210> 193
<211> 23
<212> DNA
<213> Artificial Sequence

<220>
<223> V-region heavy chain forward primer VH2FC

<400> 193
cagatcacct tgaaggagtc tgg                                               23

<210> 194
<211> 23

<212> DNA
<213> Artificial Sequence

<220>
<223> V-region heavy chain forward primer VH3FA

<400> 194
gaggtgcagc tggtggagtc tgg                                               23

<210> 195
<211> 23
<212> DNA
<213> Artificial Sequence

<220>
<223> V-region heavy chain forward primer VH3FC

<400> 195
caggtgcagc tggtggagtc tgg                                               23

<210> 196
<211> 22
<212> DNA
<213> Artificial Sequence

<220>
<223> V-region heavy chain forward primer VH3FD

<400> 196
gaggtgcagc tggtggagtc cg                                                22

<210> 197
<211> 24
<212> DNA
<213> Artificial Sequence

<220>
<223> V-region heavy chain forward primer VH3FE

<400> 197
gaagtgcagc tggtggagtc tggg                                              24

<210> 198
<211> 23
<212> DNA
<213> Artificial Sequence

<220>
<223> V-region heavy chain forward primer VH3FG

<400> 198
gaggtgcagc tgttggagtc tgg                                               23

<210> 199
<211> 23
<212> DNA
<213> Artificial Sequence

<220>
<223> V-region heavy chain forward primer VH4FA

<400> 199
cagctgcagc tgcaggagtc ggg                                               23

<210> 200
<211> 22
<212> DNA
<213> Artificial Sequence

<220>
<223> V-region heavy chain forward primer VH4FB

<400> 200
caggtgcagc tgcaggagtc gg                                                22

<210> 201
<211> 21
<212> DNA
<213> Artificial Sequence

<220>
<223> V-region heavy chain forward primer VH4FC

<400> 201
caggtgcagc tacagcagtg g                                                 21

<210> 202
<211> 22
<212> DNA
<213> Artificial Sequence

<220>
<223> V-region heavy chain forward primer VH5F

<400> 202
gaggtgcagc tggtgcagtc tg                                                22

<210> 203
<211> 22
<212> DNA
<213> Artificial Sequence

<220>
<223> V-region heavy chain forward primer VH6F

<400> 203
caggtacagc tgcagcagtc ag                                                22

<210> 204
<211> 23
<212> DNA
<213> Artificial Sequence

<220>
<223> V-region heavy chain forward primer VH7F

<400> 204
caggtgcagc tggtgcagtc tgg                                               23

<210> 205
<211> 22
<212> DNA
<213> Artificial Sequence

<220>
<223> V-region heavy chain reverse primer VH1RA

<400> 205
tatcttgcac agtaatacat gg                                                22

<210> 206
<211> 23
<212> DNA
<213> Artificial Sequence

<220>
<223> V-region heavy chain reverse primer VH1RB

<400> 206
tctgccgcac agtaatacac ggc                                               23

<210> 207
<211> 22
<212> DNA
<213> Artificial Sequence

<220>
<223> V-region heavy chain reverse primer VH1C,3A,4B

<400> 207
tctctcgcac agtaatacac gg                                                22

<210> 208
<211> 23
<212> DNA
<213> Artificial Sequence

<220>
<223> V-region heavy chain reverse primer VHR1D,3C

<400> 208
tctctcgcac agtaatacac agc                                               23

<210> 209
<211> 23
<212> DNA
<213> Artificial Sequence

<220>
<223> V-region heavy chain reverse primer VH1RE

<400> 209
tctgttgcac agtaatacac ggc                                               23

<210> 210
<211> 23
<212> DNA
<213> Artificial Sequence

<220>
<223> V-region heavy chain reverse primer VH1RF

<400> 210
cctctcgcac agtaatacac ggc                                               23

<210> 211
<211> 24
<212> DNA
<213> Artificial Sequence

<220>
<223> V-region heavy chain reverse primer VH2RA

<400> 211
gtatccgtgc acagtaatat gtgg                                              24

<210> 212
<211> 21
<212> DNA
<213> Artificial Sequence

<220>
<223> V-region heavy chain reverse primer VH2RB

<400> 212
gtatccgtgc acaataatac g                                                 21

<210> 213
<211> 24
<212> DNA
<213> Artificial Sequence

<220>
<223> V-region heavy chain reverse primer VH2RC

<400> 213
gtctgtgtgc acagtaatat gtgg                                              24

<210> 214
<211> 23
<212> DNA
<213> Artificial Sequence

<220>
<223> V-region heavy chain reverse primer VH3RB

<400> 214
tttctcacac agtaatacac agc                                               23

<210> 215
<211> 24
<212> DNA
<213> Artificial Sequence

<220>
<223> V-region heavy chain reverse primer VH3RD

<400> 215
tctcttgcac agtaatacac agcc                                              24

<210> 216
<211> 24
<212> DNA
<213> Artificial Sequence

<220>
<223> V-region heavy chain reverse primer VH3RE

<400> 216
tatcttttgc acagtaatac aagg                                              24

<210> 217

<211> 23
<212> DNA
<213> Artificial Sequence

<220>
<223> V-region heavy chain reverse primer VH3RG

<400> 217
tctttcgcac agtaatatac ggc                                              23

<210> 218
<211> 22
<212> DNA
<213> Artificial Sequence

<220>
<223> V-region heavy chain reverse primer VH3RH

<400> 218
tctgtggtac agtaatacac gg                                               22

<210> 219
<211> 22
<212> DNA
<213> Artificial Sequence

<220>
<223> V-region heavy chain reverse primer VH3RI

<400> 219
tctctagtac agtaatacac gg                                               22

<210> 220
<211> 22
<212> DNA
<213> Artificial Sequence

<220>
<223> V-region heavy chain reverse primer VH3RJ

<400> 220
tgtctagtac agtaatacac gg                                               22

<210> 221
<211> 22
<212> DNA
<213> Artificial Sequence

<220>
<223> V-region heavy chain reverse primer VH3RK

<400> 221
tctctagcac agtaatacac gg                                               22

<210> 222
<211> 23
<212> DNA
<213> Artificial Sequence

<220>
<223> V-region heavy chain reverse primer VH3RL

<400> 222

tatctggcac agtaatacac ggc                                              23

<210> 223
<211> 24
<212> DNA
<213> Artificial Sequence

<220>
<223> V-region heavy chain reverse primer VH4RA

<400> 223
tgtctcgcac agtaatacac agcc                                             24

<210> 224
<211> 23
<212> DNA
<213> Artificial Sequence

<220>
<223> V-region heavy chain reverse primer VH4RD

<400> 224
cctctcgcac agtaatacac agc                                              23

<210> 225
<211> 22
<212> DNA
<213> Artificial Sequence

<220>
<223> V-region heavy chain reverse primer VH4RC

<400> 225
tttctcgcac agtaatacac gg                                               22

<210> 226
<211> 22
<212> DNA
<213> Artificial Sequence

<220>
<223> V-region heavy chain reverse primer VH5R

<400> 226
tgtctcgcac agtaatacat gg                                               22

<210> 227
<211> 22
<212> DNA
<213> Artificial Sequence

<220>
<223> V-region heavy chain reverse primer VH6R

<400> 227
tctcttgcac agtaatacac ag                                               22

<210> 228
<211> 22
<212> DNA
<213> Artificial Sequence

<220>

<223> V-region heavy chain reverse primer VH7R

<400> 228
tatctcgcac agtaatacat gg                                                22

<210> 229
<211> 47
<212> DNA
<213> Artificial Sequence

<220>
<223> VDJ oligo primer 3-30F

<400> 229
gtagtgattt ggcccagccg gccaggtgca gctggtggag tctgggg                     47

<210> 230
<211> 26
<212> DNA
<213> Artificial Sequence

<220>
<223> VDJ oligo primer 3-30R

<400> 230
ctttcgcaca gtaatacaca gccgtg                                            26

<210> 231
<211> 54
<212> DNA
<213> Artificial Sequence

<220>
<223> VDJ oligo primer 3-30joinD1-26

<400> 231
gtattactgt gcgaaagggt atagtgggag ctactactac tttgactact gggg             54

<210> 232
<211> 54
<212> DNA
<213> Artificial Sequence

<220>
<223> VDJ oligo primer 3-30join2

<220>
<221> modified_base
<222> (1)...(54)
<223> n = g, a, c or t

<400> 232
gtattactgt gcgaaagnnt atagtgggag ctacnnctac tttgactact gggg             54

<210> 233
<211> 57
<212> DNA
<213> Artificial Sequence

<220>
<223> VDJ oligo primer 3-30join3

<220>

<221> modified_base
<222> (1)...(57)
<223> n = g, a, c or t

<400> 233
gtattactgt gcgaaagnnt atagtgggag ctacnncnnk tactttgact actgggg    57

<210> 234
<211> 51
<212> DNA
<213> Artificial Sequence

<220>
<223> VDJ oligo primer 3-30join4

<220>
<221> modified_base
<222> (1)...(51)
<223> n = g, a, c or t

<400> 234
gtattactgt gcgaaannkn nknnknnknn knnktacttt gactactggg g         51

<210> 235
<211> 64
<212> DNA
<213> Artificial Sequence

<220>
<223> VDJ oligo primer JH4-Nhe/Not

<400> 235
agccatcgcg gccgcgctag ctgaggagac gatgaccagg gttccttggc cccagtagtc 60
aaag                                                              64

<210> 236
<211> 45
<212> DNA
<213> Artificial Sequence

<220>
<223> VDJ clone D126electronic, D126A and D126B
      diversity sequence

<400> 236
tgtgcgaaag ggtatagtgg gagctactac tactttgact actgg                 45

<210> 237
<211> 48
<212> DNA
<213> Artificial Sequence

<220>
<223> VDJ clone L2A diversity sequence

<400> 237
tgtgcgaaag tatatagtgg gagctacgtc gagtactttg actactgg              48

<210> 238
<211> 45
<212> DNA
<213> Artificial Sequence

<220>
<223> VDJ clone L2B diversity sequence

<400> 238
tgtgcgaaag atagtgggag ctacggcgat tactttgact actga                45

<210> 239
<211> 42
<212> DNA
<213> Artificial Sequence

<220>
<223> VDJ clone L2C diversity sequence

<400> 239
tgtgcgaaaa ttacggcgga ggaggtgtac tttgactact gg                   42

<210> 240
<211> 45
<212> DNA
<213> Artificial Sequence

<220>
<223> VDJ clone L2D diversity sequence

<220>
<221> modified_base
<222> (29)...(30)
<223> n = g, a, c or t

<400> 240
tgtgcgaaac ggcagaggat gtttgttgnn tactttgact actgg                45

<210> 241
<211> 48
<212> DNA
<213> Artificial Sequence

<220>
<223> VDJ clone L2E diversity sequence

<400> 241
tgtgcgaaag cctatagtgg gagctacgtc ggttactttg actactgg             48

<210> 242
<211> 48
<212> DNA
<213> Artificial Sequence

<220>
<223> VDJ clone L2F diversity sequence

<220>
<221> modified_base
<222> (28)...(28)
<223> n = g, a, c or t

<400> 242
tgtgcgaaag attatagtgg gagctacncc tagtactttg actactgg             48

<210> 243
<211> 42
<212> DNA

<213> Artificial Sequence

<220>
<223> VDJ clone L1A diversity sequence

<400> 243
tgtgcgaaaa tggtgtcggc gaggttgtac tttgactact gg                42

<210> 244
<211> 42
<212> DNA
<213> Artificial Sequence

<220>
<223> VDJ clone L1B diversity sequence

<220>
<221> modified_base
<222> (21)...(21)
<223> n = g, a, c or t

<400> 244
tgtgcgaaag ggttgaagta natgaattac tttgactact gg                42

<210> 245
<211> 42
<212> DNA
<213> Artificial Sequence

<220>
<223> VDJ clone L1C diversity sequence

<400> 245
tgtgcgaaat atggtgtggg gcgggagtac tttgactact gg                42

<210> 246
<211> 48
<212> DNA
<213> Artificial Sequence

<220>
<223> VDJ clone L1D diversity sequence

<220>
<221> modified_base
<222> (28)...(28)
<223> n = g, a, c or t

<400> 246
tgtgcgaaag ggtatagtgg gagctacngc tattactttg actactgg          48

<210> 247
<211> 48
<212> DNA
<213> Artificial Sequence

<220>
<223> VDJ clone L1E diversity sequence

<400> 247
tgtgcgaaag attatagtgg gagctacggc atgtactttg actactgg          48

<210> 248

<211> 42
<212> DNA
<213> Artificial Sequence

<220>
<223> VDJ clone L1F diversity sequence

<220>
<221> modified_base
<222> (12)...(12)
<223> n = g, a, c or t

<400> 248
tgtgcgaaag cnaagggtac tacggggtac tttgactact gg                42

<210> 249
<211> 42
<212> DNA
<213> Artificial Sequence

<220>
<223> VDJ clone J4A diversity sequence

<400> 249
tgtgcgaaaa ttggtcatcg gtgttcttac tttgactact gg                42

<210> 250
<211> 42
<212> DNA
<213> Artificial Sequence

<220>
<223> VDJ clone J4B diversity sequence

<400> 250
tgtgcgaaat attgggatag gttggcgtac tttgactact gg                42

<210> 251
<211> 42
<212> DNA
<213> Artificial Sequence

<220>
<223> VDJ clone J4C diversity sequence

<400> 251
tgtgcgaaat ggggtggtta gcggcggtac tttgactact gg                42

<210> 252
<211> 42
<212> DNA
<213> Artificial Sequence

<220>
<223> VDJ clone J4D diversity sequence

<400> 252
tgtgcgaaaa cggtgccggt tgctgcttac tttgactact gg                42

<210> 253
<211> 42
<212> DNA
<213> Artificial Sequence

<220>
<223> VDJ clone J4E diversity sequence

<400> 253
tgtgcgaaac agcggcgtgt gcctgcgtac tttgactact gg                42

<210> 254
<211> 42
<212> DNA
<213> Artificial Sequence

<220>
<223> VDJ clone L3A diversity sequence

<400> 254
tgtgcgaaag tgctgaggct ggggacgtac tttgactact gg                42

<210> 255
<211> 39
<212> DNA
<213> Artificial Sequence

<220>
<223> VDJ clone L3C diversity sequence

<400> 255
tgtgcgaaag atagtgggag ctactcccct ggttactgg                39

<210> 256
<211> 42
<212> DNA
<213> Artificial Sequence

<220>
<223> VDJ clone L3D diversity sequence

<220>
<221> modified_base
<222> (24)...(24)
<223> n = g, a, c or t

<400> 256
tgtgcgaaag aggggaggat gtanacttac tttgactact gg                42

<210> 257
<211> 42
<212> DNA
<213> Artificial Sequence

<220>
<223> VDJ clone L3E diversity sequence

<220>
<221> modified_base
<222> (1)...(42)
<223> n = g, a, c or t

<400> 257
tgtgcgaaag ngganatggg gtntgggtac tttgactact gg                42

<210> 258
<211> 15

<212> PRT
<213> Artificial Sequence

<220>
<223> VDJ clone D126electronic, D126A and D126B
      diversity sequence translation

<400> 258
Cys Ala Lys Gly Tyr Ser Gly Ser Tyr Tyr Tyr Phe Asp Tyr Trp
 1               5                   10                  15


<210> 259
<211> 15
<212> PRT
<213> Artificial Sequence

<220>
<223> VDJ clone L2A diversity sequence translation

<400> 259
Cys Ala Lys Val Tyr Ser Gly Ser Tyr Val Glu Tyr Phe Asp Trp
 1               5                   10                  15


<210> 260
<211> 14
<212> PRT
<213> Artificial Sequence

<220>
<223> VDJ clone L2B diversity sequence translation

<400> 260
Cys Ala Lys Asp Ser Gly Ser Tyr Gly Asp Tyr Phe Asp Trp
 1               5                   10


<210> 261
<211> 14
<212> PRT
<213> Artificial Sequence

<220>
<223> VDJ clone L2C diversity sequence translation

<400> 261
Cys Ala Lys Ile Thr Ala Glu Glu Val Tyr Phe Asp Tyr Trp
 1               5                   10


<210> 262
<211> 15
<212> PRT
<213> Artificial Sequence

<220>
<223> VDJ clone L2D diversity sequence translation

<220>
<221> MOD_RES
<222> (10)...(10)
<223> Xaa = Val, Ala, Asp, Glu or Gly

<400> 262
Cys Ala Lys Arg Gln Arg Met Phe Val Xaa Tyr Phe Asp Tyr Trp
1               5                   10                  15

<210> 263
<211> 16
<212> PRT
<213> Artificial Sequence

<220>
<223> VDJ clone L2E diversity sequence translation

<400> 263
Cys Ala Lys Ala Tyr Ser Gly Ser Tyr Val Gly Tyr Phe Asp Tyr Trp
1               5                   10                  15

<210> 264
<211> 10
<212> PRT
<213> Artificial Sequence

<220>
<223> partial VDJ clone L2F diversity sequence
      translation

<220>
<221> MOD_RES
<222> (10)...(10)
<223> Xaa = Ser, Pro, Thr or Ala

<400> 264
Cys Ala Lys Asp Tyr Ser Gly Ser Tyr Xaa
1               5                   10

<210> 265
<211> 5
<212> PRT
<213> Artificial Sequence

<220>
<223> partial VDJ clone L2F diversity sequence
      translation

<400> 265
Tyr Phe Asp Tyr Trp
1               5

<210> 266
<211> 14
<212> PRT
<213> Artificial Sequence

<220>
<223> VDJ clone L1A diversity sequence translation

<400> 266
Cys Ala Lys Met Val Ser Ala Arg Leu Tyr Phe Asp Tyr Trp
1               5                   10

<210> 267
<211> 14
<212> PRT
<213> Artificial Sequence

<220>
<223> VDJ clone L1B diversity sequence translation

<400> 267
Cys Ala Lys Gly Leu Lys Tyr Met Asn Tyr Phe Asp Tyr Trp
1               5                   10


<210> 268
<211> 6
<212> PRT
<213> Artificial Sequence

<220>
<223> partial VDJ clone L1B diversity sequence
      translation

<400> 268
Cys Ala Lys Gly Leu Lys
1               5


<210> 269
<211> 7
<212> PRT
<213> Artificial Sequence

<220>
<223> partial VDJ clone L1B diversity sequence
      translation

<400> 269
Met Asn Tyr Phe Asp Tyr Trp
1               5


<210> 270
<211> 14
<212> PRT
<213> Artificial Sequence

<220>
<223> VDJ clone L1C diversity sequence translation

<400> 270
Cys Ala Lys Tyr Gly Val Gly Arg Glu Tyr Phe Asp Tyr Trp
1               5                   10


<210> 271
<211> 16
<212> PRT
<213> Artificial Sequence

<220>
<223> VDJ clone L1D diversity sequence translation

<220>
<221> MOD_RES
<222> (10)...(10)
<223> Xaa = Cys, Arg, Ser or Gly

<400> 271
Cys Ala Lys Gly Tyr Ser Gly Ser Tyr Xaa Tyr Tyr Phe Asp Tyr Trp
 1               5                   10                  15


<210> 272
<211> 16
<212> PRT
<213> Artificial Sequence

<220>
<223> VDJ clone L1E diversity sequence translation

<400> 272
Cys Ala Lys Asp Tyr Ser Gly Ser Tyr Gly Met Tyr Phe Asp Tyr Trp
 1               5                   10                  15


<210> 273
<211> 14
<212> PRT
<213> Artificial Sequence

<220>
<223> VDJ clone L1F diversity sequence translation

<400> 273
Cys Ala Lys Ala Lys Gly Thr Thr Gly Tyr Phe Asp Tyr Trp
 1               5                   10


<210> 274
<211> 14
<212> PRT
<213> Artificial Sequence

<220>
<223> VDJ clone J4A diversity sequence translation

<400> 274
Cys Ala Lys Ile Gly His Arg Cys Ser Tyr Phe Asp Tyr Trp
 1               5                   10


<210> 275
<211> 14
<212> PRT
<213> Artificial Sequence

<220>
<223> VDJ clone J4B diversity sequence translation

<400> 275
Cys Ala Lys Tyr Trp Asp Arg Leu Ala Tyr Phe Asp Tyr Trp
 1               5                   10

<210> 276
<211> 6
<212> PRT
<213> Artificial Sequence

<220>
<223> partial VDJ clone J4C diversity sequence
      translation

<400> 276
Cys Ala Lys Trp Gly Gly
1               5


<210> 277
<211> 7
<212> PRT
<213> Artificial Sequence

<220>
<223> partial VDJ clone J4C diversity sequence
      translation

<400> 277
Arg Arg Tyr Phe Asp Tyr Trp
1               5


<210> 278
<211> 14
<212> PRT
<213> Artificial Sequence

<220>
<223> VDJ clone J4D diversity sequence translation

<400> 278
Cys Ala Lys Thr Val Pro Val Ala Ala Tyr Phe Asp Tyr Trp
1               5                   10


<210> 279
<211> 14
<212> PRT
<213> Artificial Sequence

<220>
<223> VDJ clone J4E diversity sequence translation

<400> 279
Cys Ala Lys Gln Arg Arg Val Pro Ala Tyr Phe Asp Tyr Trp
1               5                   10


<210> 280
<211> 14
<212> PRT
<213> Artificial Sequence

<220>
<223> VDJ clone L3A diversity sequence translation

<400> 280

Cys Ala Lys Val Leu Arg Leu Gly Thr Tyr Phe Asp Tyr Trp
1               5                   10

<210> 281
<211> 13
<212> PRT
<213> Artificial Sequence

<220>
<223> VDJ clone L3C diversity sequence translation

<400> 281
Cys Ala Lys Asp Ser Gly Ser Tyr Ser Pro Gly Tyr Trp
1               5                   10

<210> 282
<211> 14
<212> PRT
<213> Artificial Sequence

<220>
<223> VDJ clone L3D diversity sequence translation

<400> 282
Cys Ala Lys Glu Gly Arg Met Tyr Thr Tyr Phe Asp Tyr Trp
1               5                   10

<210> 283
<211> 7
<212> PRT
<213> Artificial Sequence

<220>
<223> partial VDJ clone L3D diversity sequence
      translation

<400> 283
Cys Ala Lys Glu Gly Arg Met
1               5

<210> 284
<211> 6
<212> PRT
<213> Artificial Sequence

<220>
<223> partial VDJ clone L3D diversity sequence
      translation

<400> 284
Thr Tyr Phe Asp Tyr Trp
1               5

<210> 285
<211> 14
<212> PRT
<213> Artificial Sequence

<220>
<223> VDJ clone L3E diversity sequence translation

<220>
<221> MOD_RES
<222> (4)...(4)
<223> Xaa = Val, Ala, Glu or Gly

<220>
<221> MOD_RES
<222> (5)...(5)
<223> Xaa = Asp or Glu

<220>
<221> MOD_RES
<222> (8)...(8)
<223> Xaa = Phe, Ser, Tyr or Cys

<400> 285
Cys Ala Lys Xaa Xaa Met Gly Xaa Gly Tyr Phe Asp Tyr Trp
 1               5                   10